# EUROPEAN PATENT SPECIFICATION

(11) **EP 4 398 821 B1**
(45) Date of publication and mention of the grant of the patent: **24.12.2025**
(21) Application number: 22786166.3
(22) Date of filing: 09.09.2022
(51) Int. Cl.: A61B 17/221

(54) **CLOT RETRIEVAL SYSTEM**
GERINNSELBESEITIGUNGSSYSTEM
SYSTÈME DE RÉCUPÉRATION DE CAILLOT

(30) Priority: 10.09.2021 US 202117472169
(43) Date of publication of application: 17.07.2024
(73) Proprietor: Vesalio, Inc., Plano, TX 75024 (US)
(72) Inventor: ULM, III, Arthur John, Nashville, Tennessee 37205 (US); PRADO, Gustavo, San Diego, California 92106 (US)
(74) Representative: Dehns
(86) International application number: PCT/US2022/042983
(87) International publication number: WO 2023/039119

(56) References cited:
- US-A1- 2017 265 877
- US-A1- 2019 239 907
- US-A1- 2019 365 396

## Description

### BACKGROUND

### TECHNICAL FIELD

The present invention relates to a deployable system for removing a blood clot or other object from a lumen of an animal as well as to methods of manufacturing catheter-delivered medical devices from a tube of a memory metal.

### BACKGROUND OF THE INVENTION

Acute ischemic strokes develop when a blood clot (thrombus) blocks an artery supplying blood to the brain. Needless to say, when a blood clot creates such a blockage, time in removing the clot is critical.

The removal of intracranial obstructions is limited by several factors, such as the distance of the intracranial obstruction from the femoral access site, the tortuosity (twists and turns in the artery as it enters the base of the skull) of the cervical and proximal intracranial vasculature, the small size of the vessels and the extremely thin walls of intracranial vessels, which lack a significant muscular layer. These limitations require a device to be small and flexible enough to navigate through tortuous vessels within a guide catheter and microcatheter, expand after delivery at the site of occlusion and be retrievable into the microcatheter and yet be strong enough to dislodge strongly adherent thrombus from the vessel wall. In addition, the device should distally entrap or encase the thrombus to prevent embolization to other vessels and to completely remove the occlusion. The device should be retrievable without the need for proximal occlusion of the vessel, which carries risk of further ischemia and risk of vessel injury. The device should be simple to use and be capable of multi-use within the same patient treatment. The device should not be abrasive and should not have sharp corners exposed to the endothelial layer of the vessel wall.

Currently available intravascular thrombus and foreign body removal devices lack several of these features. Currently available devices include the MERCI^{™} RETRIEVER clot retriever device marketed by Concentric Medical, Inc. (Mountainview, CA), the PENUMBRA^{™} system marketed by Penumbra Inc. (Alameda, CA) to retrieve clots, and the newer stent retrieval devices TREVO^{™} (Stryker, Kalamazoo, MI) and SOLITAIRE^{™} (eV3 Endovascular Inc., Plymouth, MA, which is a subsidiary of Covidien). All the devices are ineffectual at removing organized hard thrombus that embolize to the brain from the heart and from atherosclerotic proximal vessels. These "hard" thrombi constitute the majority of strokes which are refractory to medical treatment and are therefore referred for removal by mechanical means through an endovascular approach. The MERCI retrieval system is comprised of coiled spring-like metal and associated suture material. The method of use is deployment distal to the thrombus and by withdrawing the device through the thrombus, the thrombus becomes entangled in the coil and mesh and then is retrieved. The MERCI system requires occlusion of the proximal vessel with a balloon catheter and simultaneous aspiration of blood while the thrombus is being removed. Most of the time, the device fails to dislodge the thrombus from the wall of the vessel and often, even when successfully dislodging the thrombus, the thrombus embolizes into another or the same vessel due to the open ended nature of the device.

The next attempt at a thrombus removal system was the PENUMBRA. The PENUMBRA is a suction catheter with a separator that macerates the thrombus which is then removed by suction. The device is ineffective at removing hard, organized thrombus which has embolized from the heart, cholesterol plaque from proximal feeding arteries and other foreign bodies.

The SOLITAIRE and TREVO systems are self-expanding non-detachable stents. The devices are delivered across the thrombus which is then supposed to become entwined in the mesh of the stent and which is then removed in a manner similar to the MERCI system. Again, these devices are ineffectual at treating hard thrombus. In fact, the thrombus is often compressed against the vessel wall by the stent which temporarily opens the vessel by outwardly pressing the clot against the vessel wall. Upon retrieval of the devices, the clot remains or is broken up into several pieces which embolize to vessels further along the vessel.

Thus, there is a need for new, easy-to-use, easy-to-manufacture, safe surgical devices for removing obstructions, such as blood clots, from internal lumens of humans and other animals in a timely manner.

In addition, it may be desirable to make memory-metal based mechanical thrombectomy devices, also referred to in the art as stent retrievers, from a single tube of the memory-metal (e.g., nitinol), and in the process, laser cut and shape set the middle portion to form the capture portion (e.g., the basket) and leave the proximal and distal ends at least partially intact. To provide design flexibility to the designer of the basket (so that he/she may include complicated structure in the middle portion), it is desirable that the single tube have a relatively large diameter. However, it is also desirable to allow the devices to fit into a small catheter (called a microcatheter), which creates issues if the proximal and distal ends remain on the device. Thus, there is a need for processes of making devices that have the advantages of being cut from a larger diameter tube but are also able to fit inside a small catheter.
US 2019/365396 A1 discloses a catheter delivered endovascular medical device with a pull wire attached to a distal body.
US 2017/265877 A1 discloses a catheter delivered endovascular medical device with a pull wire attached to a distal body.
US 2019/239907 A1 discloses a clot retrieval device with an inner body having a collapsed state and an expanded state.

### BRIEF SUMMARY

The present invention relates to a system removing objects from an interior lumen of an animal as claimed in claim 1. Further embodiments of the invention are recited in the dependent claims. The system may be deployed in the lumen from a distal end of a catheter and, in some examples, includes a pull wire having a proximal end and a distal end; a distal body attached to the pull wire, the distal body comprising an interior, an exterior, a proximal end, a distal end, a plurality of proximal memory metal strips located at the proximal end, a proximal hub/junction located in the distal body interior, and a distal hub/junction located distal relative to the proximal hub/junction. The distal body has a relaxed state wherein the distal body has a first height and width and a collapsed state wherein the distal body has a second height and width, the second height less than said first height, the second width less than the first width. The system further includes a catheter having an interior, a proximal end leading to the interior and a distal end leading to the interior, the catheter comprised of a biocompatible material and configured to envelope the distal body when the distal body is in the collapsed state. Each of the proximal memory metal strips has a proximal end and a distal end and preferably, in the relaxed state, each of the proximal ends of the proximal memory metal strips is located proximal relative to the proximal hub/junction. Preferably, in the relaxed state, the proximal ends of the proximal memory metal strips are configured to move towards each other and towards the pull wire when an operator moves the proximal hub/junction distally and closer to the stationary distal hub/junction (i.e., when the operator decreases the distance between the hubs/junctions). Preferably, in the relaxed state, the proximal ends of the proximal memory metal strips are configured to move away from each other and away from the pull wire by moving the proximal hub/junction proximally away from the stationary distal hub/junction (i.e., when the operator increases the distance between the hubs/junctions).

Optionally, the system further includes a plurality of memory metal connector strips, the plurality of memory metal connector strips each having a proximal end attached to a proximal memory metal strip and a distal end attached to the proximal hub/junction. Optionally, the connector strips are integral with the proximal hub/junction (i.e., optionally, the connector strips and the proximal hub/junction are formed from the same piece of memory metal). Optionally, the proximal hub/junction is a tube having an aperture and the pull wire passes through the aperture. Optionally, in the relaxed state, the proximal hub/junction is slideable along the pull wire (i.e., at least a segment of the pull wire). Optionally, in the relaxed state, the proximal memory metal strips are distributed substantially evenly about a perimeter of the distal body. Optionally, the distal hub/junction is a tube having an aperture. Optionally, the distal hub/junction is attached to the pull wire such that the distal hub/junction is not slideable along the pull wire. Optionally, the distal body further comprises a lead wire extending distally from the distal hub/junction. Optionally, the distal body comprises a basket comprised of a plurality of memory metal strips distal relative to the proximal memory metal strips. Optionally, the distal hub/junction, the proximal hub/junction, and the distal basket are comprised of a nitinol having the same material composition. Optionally, the distal body further comprises an x-ray marker. Optionally, the proximal memory metal strips form a claw, the claw having a closeable proximal end formed by the proximal ends of the proximal memory metal strips. Optionally, between 2 and 4 proximal memory metal strips form the claw. Optionally, the distal body, in the relaxed state, has a tapered shape in which the distal body height and width decrease from the proximal end to the distal end. Optionally, the distal body, in the relaxed state, has a bullet shape. Optionally, the proximal hub/junction and the distal hub/junction are generally cylindrical in shape and each has an outer diameter and an inner diameter that forms the apertures of the proximal and distal hub/junctions, the outer diameters of the proximal and distal hub/junctions are substantially the same size, and the inner diameters of the proximal and distal hubs/junctions are substantially the same size. Optionally, the outer diameters of the proximal and distal hubs/junctions are from about 0.011 inches to about 0.054 inches, and the inner diameters of the proximal and distal hubs are from about 0.008 inches to about 0.051 inches (1 inch = 25.4 mm). Optionally, the pull wire is generally cylindrical and the diameter of the pull wire is between about 0.008 inches and about 0.051 inches. Optionally, the proximal memory metal strips have a length of between about 10 and about 60 millimeters. Optionally, the first height and first width of the distal body are between about 2 millimeters (mm) and about 6 millimeters. Optionally, the proximal memory metal strips are configured to a separate a clot from a blood vessel wall.

In some examples, a system for removing objects from an interior lumen of an animal that includes:
a pull wire having a proximal end and a distal end;
a distal body attached to the pull wire, the distal body comprising an interior, a proximal end, a distal end, a distal body length extending from the proximal end to the distal end, a proximal hub/junction (preferably in the form of a tube) forming the proximal end of the distal body, a basket comprised of a plurality of cells formed by a plurality of basket strips, a plurality of proximal strips, and, optionally a distal hub/junction (preferably in the form of a tube) forming a distal end of the basket, the basket comprising a basket interior, each proximal strip having a proximal end attached to the proximal hub/junction, and a distal end attached to a cell, the distal body having a relaxed state wherein the distal body has a first height and a first width, and a collapsed state wherein the distal body has a second height and a second width, the second height less than the first height, the second width less than the first width; and
a catheter having an interior, a proximal end leading to the interior and a distal end leading to the interior, the catheter comprised of a biocompatible material and configured to envelope the distal body when the distal body is in the collapsed state,
wherein, in the relaxed state, the basket comprises a first pair of distal crowns not attached to another cell of the basket and pointing generally in the distal direction, the first pair of distal crowns located approximately the same distance from the proximal hub/junction and approximately 180 degrees relative to each other (e.g., between about 150 degrees and about 180 degrees relative to each other), and further wherein the basket further comprises a second pair of distal crowns not attached to another cell of the basket and pointing generally in the distal direction, the second pair of distal crowns located distally relative to, and approximately 90 degrees relative to, the first pair of distal crowns (e.g., each distal crown of the second pair of distal crowns is located approximately 60 degrees to 90 degrees relative to a distal crown of the first pair of distal crowns), the distal crowns in the second pair of distal crowns located approximately the same distance from the proximal hub/junction and further wherein each of the distal crowns in the first and second pair of distal crowns comprises an x-ray marker, the x-ray maker more visible under x-ray as compared to the basket strips when the distal body is located in a cranial blood vessel inside the body of a human and the x-ray is taken from outside the human's body. When it is said that the first pair of distal crowns are located approximately the same distance from the proximal hub/junction, it will be understood that if one of the first pair of distal crowns is located X distance from the proximal hub/junction, the other of the first pair of distal crowns is located X distance plus or minus (+/-) 3 mm from the proximal hub/junction, more preferably X distance plus or minus (+/-) 0.5 mm from the proximal hub/junction. Similarly, when it is said that the second pair of distal crowns are located approximately the same distance from the proximal hub/junction, it will be understood that if one of the second pair of distal crowns is located Y distance from the proximal hub/junction, the other of the first pair of distal crowns is located Y distance plus or minus (+/-) 3 mm from the proximal hub/junction, more preferably Y distance plus or minus (+/-) 0.5 mm from the proximal hub/junction. Optionally, instead of a distal hub/junction, the basket includes an open distal end.

sOptionally, the x-ray markers are comprised of a material different than the material forming the basket strips. Optionally, in the relaxed state, the basket interior is substantially hollow. Optionally, in the relaxed state, the distal body does not have another x-ray marker that is located approximately the same distance from the proximal hub/junction as the first pair of x-ray markers and the distal body does not have another x-ray marker that is located approximately the same distance from the proximal hub/junction as the second pair of x-ray markers. In other words, the first and second pair of x-ray markers are the only markers their respective distances from the proximal hub/junction. Optionally, each distal crown in the first and second pair of distal crowns forms part of an enlarged cell and further wherein the surface area of each enlarged cell in the relaxed state is greater than the surface area of each of the other individual cells of the basket and further wherein the enlarged cells are configured to allow a thrombus to pass therethrough and into the basket interior. Optionally, in the relaxed state, the distal body does not have another free distal-pointing crown that is located approximately the same distance from the proximal hub/junction as the first pair of distal crowns and the distal body does not have another free distal-pointing crown that is located approximately the same distance from the proximal hub/junction as the second pair of distal crowns. Optionally, the basket strips are comprised of a memory metal. Optionally, each of the distal crowns in the first pair and second pair of distal crowns curve radially inward toward the basket interior in the relaxed state, wherein the distal crowns of the first pair of distal crowns are configured to contact each other when an exterior, external compressive force (such as a thrombus) is exerted on a distal crown of the first pair of distal crowns when the distal body is in the relaxed state, and further wherein the distal crowns of the second pair of distal crowns are configured to contact each other when an exterior, external compressive force (such as a thrombus) is exerted on a distal crown of the second pair of distal crowns when the distal body is in the relaxed state. Optionally, the proximal hub/junction is located approximately in the center of the first height and first width in the relaxed state. For example, preferably the proximal hub/junction is located within 0.5 mm of the center of first width and the first height. Optionally, the catheter is comprised of a polymeric material (i.e., one or more polymeric materials such as silicone, PVC, latex rubber or braided nylon). Optionally, the pull wire is comprised of a biocompatible metallic material (e.g., a biocompatible metal or a biocompatible metal alloy). Optionally, the proximal end of a first proximal strip is located at least about 65 degrees (e.g., between about 65 and about 180 degrees) relative to the distal end of the first proximal strip, wherein the proximal end of a second proximal strip is located at least about 65 degrees (e.g., between about 65 and about 180 degrees) relative to the distal end of the second proximal strip, and further wherein the first and second proximal strips intersect adjacent and distal to the proximal hub/junction (e.g., within about 0 and about 4 mm of the proximal hub/junction). Optionally, each distal crown forms part of a cell that further comprises a proximal crown pointing generally in the proximal direction and connected to a memory metal strip (e.g., a proximal strip comprised of a memory metal or a basket strip comprised of a memory metal). In other words, the proximal crowns are not free. Optionally, the basket, the proximal hub/junction and the proximal strips are comprised of a memory metal, wherein the proximal hub/junction comprises a proximal end and a distal end, and further wherein the proximal strips are integral with the distal end of the proximal hub/junction. Optionally, the length of the distal body from the proximal hub/junction to the distal hub/junction (not including any lead wire) is from about 20 mm to about 65 mm.

In some examples, the system includes:
a pull wire having a proximal end and a distal end;
a distal body attached to the pull wire, the distal body comprising an interior, a proximal end, a distal end, a distal body length extending from the proximal end to the distal end, a proximal hub/junction (preferably in the form of a tube) forming the proximal end of the distal body, a basket comprised of a plurality of cells formed by a plurality of basket strips, a plurality of proximal strips, and optionally a distal hub/junction (preferably in the form of a tube) forming a distal end of the basket, the basket comprising a basket interior, each proximal strip having a proximal end attached to the proximal hub/junction, and a distal end attached to a cell, the distal body having a relaxed state wherein the distal body has a first height and a first width, and a collapsed state wherein the distal body has a second height and a second width, the second height less than the first height, the second width less than the first width; and
a catheter having an interior, a proximal end leading to the interior and a distal end leading to the interior, the catheter comprised of a biocompatible material and configured to envelope the distal body when the distal body is in the collapsed state,
wherein, in the relaxed state, the basket comprises a first pair of distal crowns not attached to another cell of the basket and pointing generally in the distal direction, the first pair of distal crowns located approximately the same distance from the proximal hub/junction and approximately 180 degrees relative to each other (e.g., between about 150 degrees and about 180 degrees relative to each other), and further wherein the basket further comprises a second pair of distal crowns not attached to another cell of the basket and pointing generally in the distal direction, the second pair of distal crowns located distally relative to, and approximately 90 degrees relative to, the first pair of distal crowns (e.g., each distal crown of the second pair of distal crowns is located approximately 60 degrees to 90 degrees relative to a distal crown of the first pair of distal crowns), the distal crowns in the second pair of distal crowns located approximately the same distance from the proximal hub/junction, wherein each distal crown of the first and second pair of distal crowns form a cell, each cell further comprising a proximal crown pointing generally in the proximal direction and connected to a memory metal strip, wherein each of the distal crowns in the first pair and second pair of distal crowns curve radially inward toward the basket interior in the relaxed state, wherein the distal crowns of the first pair of distal crowns are configured to contact each other when an exterior, external compressive force (e.g., a thrombus) is exerted on a distal crown of the first pair of distal crowns when the distal body is in the relaxed state, and further wherein the distal crowns of the second pair of distal crowns are configured to contact each other when an exterior, external compressive force (e.g., a thrombus) is exerted on a distal crown of the second pair of distal crowns when the distal body is in the relaxed state. When it is said that a proximal crown pointing generally in the proximal direction and is connected to a memory metal strip, it is meant that the proximal crown is either connected to a basket strip or a proximal strip comprised of a memory metal (e.g., nitinol). When it is said that the first pair of distal crowns are located approximately the same distance from the proximal hub/junction, it will be understood that if one of the first pair of distal crowns is located X distance from the proximal hub/junction, the other of the first pair of distal crowns is located X distance plus or minus (+/-) 0.5 mm from the proximal hub/junction. Similarly, when it is said that the second pair of distal crowns are located approximately the same distance from the proximal hub/junction, it will be understood that if one of the second pair of distal crowns is located Y distance from the proximal hub/junction, the other of the first pair of distal crowns is located Y distance plus or minus (+/-) 0.5 mm from the proximal hub/junction. Optionally, instead of a distal hub/junction, the basket includes an open distal end.

Optionally, the proximal hub/junction is located approximately in the center of the first height and first width in the relaxed state. For example, preferably the proximal hub/junction is located within 0.5 mm of the center of first width and the first height. Optionally, the catheter is comprised of a polymeric material (i.e., one or more polymeric materials such as silicone, PVC, latex rubber or braided nylon). Optionally, the pull wire is comprised of a biocompatible metallic material (e.g., a biocompatible metal or a biocompatible metal alloy). Optionally, in the relaxed state, the basket interior is substantially hollow. Optionally, the proximal end of a first proximal strip is located at least about 65 degrees (e.g., between about 65 and about 180 degrees) relative to the distal end of the first proximal strip, wherein the proximal end of a second proximal strip is located at least about 65 degrees (e.g., between about 65 and about 180 degrees) relative to the distal end of the second proximal strip, and further wherein the first and second proximal strips intersect adjacent and distal to the proximal hub/junction (e.g., within about 0 mm and about 4 mm of the proximal hub/junction). Optionally, each distal crown in the first and second pair of distal crowns forms part of an enlarged cell and further wherein the surface area of each enlarged cell in the relaxed state is at least twice as large as the surface area of each other individual cell of the basket and further wherein the enlarged cells are configured to allow a thrombus to pass therethrough and into the basket interior. Optionally, the pull wire is attached to the proximal hub/junction. Optionally, the basket, the proximal hub/junction and the proximal strips are comprised of a memory metal, wherein the proximal hub/junction comprises a proximal end and a distal end, and further wherein the proximal strips are integral with the distal end of the proximal hub/junction. Optionally, the distal body further comprises a lead wire extending distally from the distal hub/junction, the lead wire having a length of from about 3 mm to about 10 mm. Optionally, the distal hub/junction, the proximal hub/junction, and the basket are comprised of a nitinol having the same material composition and further wherein the proximal and the distal hubs/junctions are tubular and generally cylindrical in shape and each has an outer diameter and an inner diameter, the inner diameter forming apertures of the proximal and distal hubs/junctions and further wherein the outer diameters of the proximal and distal hubs/junctions are substantially the same size and further wherein the inner diameters of the proximal and distal hubs/junctions are substantially the same size. Optionally, the length of the distal body from the proximal hub/junction to the distal hub/junction (not including any lead wire) is from about 20 mm to about 65 mm.

In some examples, a catheter-delivered endovascular device may comprise:
a) a pull wire having a proximal end, a distal end and a pull wire longitudinal axis extending from the proximal end to the distal end;
b) a deployable dual basket system attached to the pull wire and comprising a system circumference separating a system interior from a system exterior, a system proximal end, a system distal end, a system height having a system height center, a system width perpendicular to the system height and having a system width center, a system longitudinal axis from the system proximal end to the system distal end and extending through the system height center and system width center, the deployable dual basket system comprising:
   i) a proximal basket attached to the pull wire, the proximal basket comprising a proximal basket circumference separating a proximal basket interior from a proximal basket exterior, a proximal end forming the system proximal end, a distal end, a proximal basket height generally parallel to the system height, a proximal basket width generally parallel to the system width and perpendicular to the proximal basket height, a proximal basket longitudinal axis extending from the proximal basket proximal end to the proximal basket distal end and generally parallel to the system longitudinal axis and generally perpendicular to the proximal basket height and proximal basket width, a proximal junction located at the proximal end of the proximal basket, a plurality of proximal cells distal to the proximal junction and defined by a plurality of proximal basket memory metal strips, each proximal cell comprising a proximal crown located at the proximal end of the proximal cell and pointing generally in the proximal direction and a distal crown located at the distal end of the proximal cell and pointing generally in the distal direction, a plurality of proximal tether memory metal strips located between the proximal junction and the proximal cells and connecting the proximal cells to the proximal junction, each proximal tether memory metal strip having a proximal end attached to the proximal junction, a distal end attached to a proximal crown of a proximal cell, the proximal basket having a relaxed state wherein the proximal basket has a first height and a first width and a collapsed state wherein the proximal basket has a second height and a second width, the second height less than the first height and the second width less than the first width; and
   ii) a distal basket distal to the proximal basket and comprising a distal basket circumference separating a distal basket interior from a distal basket exterior, a proximal end, a distal end forming the system distal end, a distal basket height generally parallel to the system height, a distal basket width generally parallel to the system width and generally perpendicular to the distal basket height, a distal basket longitudinal axis extending from the distal basket proximal end to the distal basket distal end and generally parallel to the system longitudinal axis, a distal junction located at the distal end of the distal basket, a plurality of distal cells proximal to the distal junction and defined by a plurality of distal basket memory metal strips, each distal cell comprising a proximal crown located at the proximal end of the distal cell and pointing generally in the proximal direction and a distal crown located at the distal end of the distal cell and pointing generally in the distal direction, the distal basket having a relaxed state wherein the distal basket has a first height and a first width and a collapsed state wherein the distal basket has a second height and a second width, the second height less than the first height; and
   iii) a plurality of basket connector tether memory metal strips located between the proximal basket and the distal basket and connecting the proximal basket to the distal basket and located between the proximal basket and the distal basket, each basket connector tether memory metal strip having a proximal end attached to a distal crown of a cell located at the distal end of the proximal basket and a distal end attached to a proximal crown of a cell located at the proximal end of the distal basket; and
c) a catheter having an interior, a proximal end leading to the interior and a distal end leading to the interior, the catheter comprised of a biocompatible material and configured to envelope the deployable dual basket system when the proximal basket and distal basket are in the collapsed state,
wherein, in the relaxed state and the collapsed state, the basket connector tether memory metal strips rotate a degree of rotation about the system circumference relative to the proximal basket longitudinal axis, the distal basket longitudinal axis and the system longitudinal axis.

Optionally, in the relaxed state and the collapsed state, a distal crown of the proximal basket attached to the proximal end of a basket connector tether memory metal strip is offset about the system circumference relative to the proximal crown of the distal basket attached to the distal end of the same basket connector tether memory metal strip. Optionally, each basket connector tether memory metal strip rotates a greater degree of rotation in the collapsed state as compared to the degree of rotation of the same basket tether connector memory metal strip in the relaxed state. Optionally, at least some of the distal basket memory metal strips are located at the distal end of the distal basket, wherein each of the distal basket memory metal strips located at the distal end of the distal basket have a distal end, wherein each of the distal ends of the distal basket memory metal strips located at the distal end of the distal basket converge at the distal junction and further wherein the distal basket, in the relaxed state, comprises a tapered region in which the distal basket height and width decrease as the distal basket memory metal strips located at the distal end of the distal basket approach the distal junction. Optionally, the proximal basket, in the relaxed state, comprises a tapered region in which the proximal basket height and width decrease as the proximal tether memory metal strips approach the proximal junction. Optionally, in the relaxed state, except for the tapered regions and the basket connector tether memory metal strips, the deployable dual basket system has a generally tubular shape. Optionally, in the relaxed state, the radial force of the deployable dual basket system from the proximal ends of the basket connector tether memory metal strips to the distal ends of the basket connector tether memory metal strips is less than the radial force of the proximal basket, as measured from the proximal crowns of the cells of the proximal basket attached to the plurality of proximal memory metal strips to the distal crowns of the cells of the proximal basket attached to the plurality of basket connector tether memory metal strips.

Optionally, the system has two basket connector tether memory metal strips. Optionally, in the relaxed state, the basket connector tether memory metal strips each rotate at least about fifteen degrees in the same direction relative to the proximal basket longitudinal axis and the distal basket longitudinal axis. Optionally, in the collapsed state, the distal end of a first basket connector tether memory metal strip is located between about 90 degrees and about 270 degrees relative to the proximal end of the first basket connector tether memory metal strip, and further wherein in the collapsed state, the distal end of a second basket connector tether memory metal strip is located between about 90 degrees and about 270 degrees relative to the proximal end of the second connector tether memory metal strip. Optionally, in the relaxed state, the height of the proximal basket is greater than the height of the distal basket and further wherein the width of the proximal basket is greater than the width of the distal basket. Optionally, in the relaxed state, the radial force of the distal basket, as measured from the proximal crowns of the cells of the distal basket attached to the plurality of basket connector tether memory metal strips to the distal-most crown of the distal cells of the distal basket, is less than the radial force of the proximal basket, as measured from the proximal crowns of the cells of the proximal basket attached to the plurality of proximal memory metal strips to the distal crowns of the cells of the proximal basket attached to the plurality of basket connector tether memory metal strips. Optionally, in the relaxed state, the radial force of the proximal basket is substantially uniform from the proximal crowns of the cells of the proximal basket attached to the plurality of proximal memory metal strips to the distal crowns of the cells of the proximal basket attached to the plurality of basket connector tether memory metal strips. Optionally, in the relaxed state, the radial force of the distal basket is substantially uniform from the proximal crowns of the cells of the distal basket attached to the plurality of basket connector tether memory metal strips to the distal-most crown of the distal cells of the distal basket. Optionally, the proximal basket interior and the distal basket interior are generally hollow and the proximal basket cells are spaced about the circumference of the proximal basket and further wherein the distal basket cells are spaced about the circumference of the distal basket. Optionally, the basket connector tether memory metal strips do not traverse the system interior. Optionally, each of the distal crowns of the proximal basket connected to the basket connector tether memory metal strips are approximately the same distance from the proximal junction and further wherein each of the proximal crowns of the distal basket connected to the basket connector tether memory metal strips are approximately same distance from the distal junction. Optionally, each of the proximal crowns of the proximal basket and distal basket are connected to a memory metal strip extending proximally from the proximal crowns and each of the distal crowns of the proximal basket and distal basket are connected to a memory metal strip extending distally from the distal crowns. Optionally, the basket connector tether memory metal strips and the proximal tether memory metal strips form flex points of the deployable dual basket system. Optionally, in the collapsed state, the distal end of a first proximal tether memory metal strip is located between about 90 degrees and about 270 degrees relative to the proximal end of the first proximal tether memory metal strip, and further wherein in the collapsed state, the distal end of a second proximal tether memory metal strip is located between about 90 degrees and about 270 degrees relative to the proximal end of the second proximal tether memory metal strip. Optionally, the first and second proximal memory metal strips intersect adjacent and distal to the proximal junction. Optionally, the basket connector tether memory metal strips form the sole attachment of the proximal basket to the distal basket.

In some examples, a catheter-delivered endovascular device may comprise:
a) a pull wire having a proximal end, a distal end and a pull wire longitudinal axis extending from the proximal end to the distal end;
b) a deployable dual basket system attached to the pull wire and comprising a system circumference separating a system interior from a system exterior, a system proximal end, a system distal end, a system height having a system height center, a system width perpendicular to the system height and having a system width center, a system longitudinal axis from the system proximal end to the system distal end and extending through the system height center and system width center, the deployable dual basket system comprising:
   i) a proximal basket attached to the pull wire, the proximal basket comprising a proximal basket circumference separating a proximal basket interior from a proximal basket exterior, a proximal end forming the system proximal end, a distal end, a proximal basket height generally parallel to the system height, a proximal basket width generally parallel to the system width and perpendicular to the proximal basket height, a proximal basket longitudinal axis extending from the proximal basket proximal end to the distal end and generally parallel to the system longitudinal axis and generally perpendicular to the proximal basket height and proximal basket width, a proximal junction located at the proximal end of the proximal basket, a plurality of proximal cells distal to the proximal junction and defined by a plurality of proximal basket memory metal strips, each proximal cell comprising a proximal crown located at the proximal end of the proximal cell and pointing generally in the proximal direction and a distal crown located at the distal end of the proximal cell and pointing generally in the distal direction, a plurality of proximal tether memory metal strips located between the proximal junction and the proximal cells and connecting the proximal cells to the proximal junction, each proximal tether memory metal strip having a proximal end attached to the proximal junction, a distal end attached to a proximal crown of a proximal cell, the proximal basket having a relaxed state wherein the proximal basket has a first height and a collapsed state wherein the proximal basket has a second height, the second height less than the first height and the second width less than the first width; and
   ii) a distal basket distal to the proximal basket and comprising a distal basket circumference separating a distal basket interior from a distal basket exterior, a proximal end, a distal end forming the system distal end, a distal basket height generally parallel to the system height, a distal basket width generally parallel to the system width and generally perpendicular to the distal basket height, a distal basket longitudinal axis extending from the distal basket proximal end to the distal end and generally parallel to the system longitudinal axis, a distal junction located at the distal end of the distal basket, a plurality of distal cells proximal to the distal junction and defined by a plurality of distal basket memory metal strips, each distal cell comprising a proximal crown located at the proximal end of the distal cell and pointing generally in the proximal direction and a distal crown located at the distal end of the distal cell and pointing generally in the distal direction, the distal basket having a relaxed state wherein the distal basket has a first height and a first width and a collapsed state wherein the distal basket has a second height and a second width, the second height less than the first height; and
   iii) a plurality of basket connector tether memory metal strips located between the proximal basket and the distal basket and connecting the proximal basket to the distal basket and located between the proximal basket and the distal basket, each basket connector tether memory metal strip having a proximal end attached to a distal crown of a cell located at the distal end of the proximal basket and a distal end attached to a proximal crown of a cell located at the proximal end of the distal basket; and
c) a catheter having an interior, a proximal end leading to the interior and a distal end leading to the interior, the catheter comprised of a biocompatible material and configured to envelope the deployable dual basket system when the proximal basket and distal basket are in the collapsed state,

Optionally, in the relaxed state, each basket connector tether memory metal strip rotates a degree of rotation about the system circumference relative to the proximal basket longitudinal axis, the distal basket longitudinal axis and the system longitudinal axis. Optionally, in the relaxed state, a distal crown of the proximal basket attached to the proximal end of a basket connector tether memory metal strip is offset about the system circumference relative to the proximal crown of the distal basket attached to the distal end of the same basket connector tether memory metal strip.

In some examples, a system for removing objects from an interior lumen of an animal may comprise: a pull wire having a proximal end and a distal end; a distal body attached to the pull wire, the distal body comprising a distal body perimeter separating a distal body interior from a distal body exterior, a proximal end having a proximal end center, a distal end having distal end center, a distal body length extending from the proximal end to the distal end, a longitudinal axis extending through the proximal end center and the distal end center and parallel to the distal body length, a proximal junction forming the proximal end of the distal body, a basket comprising a proximal portion comprised of a plurality of proximal cells spaced about the distal body perimeter and formed by a plurality of basket memory metal strips and a distal portion located adjacent to a distal end of the basket and connected to the proximal portion at at least one connection point, the proximal portion comprising a proximal portion interior, the distal portion comprised of a plurality of distal braided mesh openings formed by a plurality of woven linear strands, the distal portion having a perimeter, each woven linear strand rotating about the distal portion perimeter relative to the distal body longitudinal axis a plurality of times in a helical fashion, the distal basket comprising a basket interior, the distal body having a relaxed state wherein the distal body has a first height and a first width, and a collapsed state wherein the distal body has a second height and a second width, the second height less than the first height, the second width less than the first width; and a catheter having an interior, a proximal end leading to the interior and a distal end leading to the interior, the catheter comprised of a biocompatible material and configured to envelope the distal body when the distal body is in the collapsed state.

Optionally, in the relaxed state, the median surface area of the proximal cells is larger than the median surface area of the distal braided mesh openings. Optionally, in the relaxed state, the median radial force of the distal portion is substantially less than the median radial force of the proximal portion. Optionally, the radial force of the proximal portion through its connection to the distal portion at the at least one connection point is configured to cause the distal portion to move to the relaxed state when the proximal portion moves from the collapsed state to the relaxed state. Optionally, the proximal portion and the distal portion each have a length generally parallel to the distal body length, the proximal portion and distal portion lengths configured to elongate upon moving from the relaxed state to the collapsed state. Optionally, upon moving from the relaxed state to the collapsed state, the length of the distal portion is configured to elongate a greater percentage as compared to the elongation of the proximal portion. Optionally, the woven linear strands rotate about the distal portion perimeter relative to the distal body longitudinal axis a fewer number of times per unit of distance in the collapsed state as compared to the relaxed state.

Optionally, in the relaxed state, the distal portion comprises at least a segment distal to the proximal portion. Optionally, the distal portion is located in the proximal portion interior. Optionally, the distal basket further comprises a distal junction comprising a proximal end, the proximal end of the distal junction forming the distal end of the basket, wherein the basket strips and the distal woven strands are attached to the distal junction and the at least one connection point is the distal junction. Optionally, the distal junction is a tube. Optionally, the proximal portion, but not the distal portion, is configured to alter the shape of a curved intracranial artery. Optionally, in the relaxed state, the distal portion is more flexible than the proximal portion. Optionally, distal portion in the relaxed state comprises a tapered region in which the distal body height and width decrease as the woven linear strands approach the distal end of the distal basket. Optionally, in the relaxed state, the basket interior is substantially hollow. Optionally, the proximal portion comprises a distal end comprising between two and four basket memory metal strip distal ends and further wherein each woven linear strand comprises a proximal end attached to a basket memory metal strip distal end. Optionally, the distal portion comprises at least two woven linear strands attached to each basket memory metal strip distal end. Optionally, in the relaxed state, the proximal portion comprises an interior surface facing the distal body interior and the distal portion comprises an outer surface facing and connected to the proximal portion interior surface, and further wherein at least a segment of the distal portion is interior to the proximal portion in the relaxed state. Optionally, each woven linear strand comprises a free proximal end and further wherein all free proximal ends of the woven linear strands are located in the proximal portion interior in the relaxed state. Optionally, the distal portion is configured to elongate proximally and distally relative to the proximal portion and the at least one connection point upon moving from the relaxed state to the collapsed state. Optionally, the distal portion is attached to the proximal portion by at least two connection points, and further wherein said at least two connection points are located a different distance from the proximal junction in the relaxed state, and further wherein said at least two connection points are located a different distance from the proximal junction in the collapsed state. Optionally, in the relaxed state, the distal portion impedes blood flow to a greater extent than the proximal portion when the proximal portion and the distal portion are placed in a blood vessel. Optionally the distal portion is configured to reduce blood flow by at least 25% when the distal portion is placed in a blood vessel. Optionally, the distal body further comprises a plurality of proximal strips, each proximal strip having a distal end attached to a proximal cell and a proximal end, the proximal ends of the proximal strips converging at the proximal junction. Optionally, in the relaxed state, the proximal portion comprises a first pair of distal crowns not attached to another cell of the basket and pointing generally in the distal direction, the distal crowns in the first pair of distal crowns located approximately the same distance from the proximal junction and between 150 degrees and 180 degrees relative to each other, and further wherein the basket further comprises a second pair of distal crowns not attached to another cell of the basket and pointing generally in the distal direction, the second pair of distal crowns located distally relative to the first pair of distal crowns, each of the distal crowns in the second pair of distal crowns located between 60 degrees and 90 degrees relative to a distal crown in the first pair of distal crowns, the distal crowns in the second pair of distal crowns located approximately the same distance from the proximal junction, each of the distal crowns forming a portion of a proximal cell,
wherein each distal crown in the first and second pair of distal crowns forms part of a different enlarged proximal cell, each enlarged proximal cell having a center,
wherein the centers of the enlarged proximal cells of the first pair of distal crowns are approximately 180 degrees relative to each other (i.e., 150 degrees to 180 degrees relative to each other) and approximately 90 degrees relative to the centers of the enlarged cells of the second pair of distal crowns (i.e., between 60 degrees and 90 degrees relative to the centers of the enlarged cells of the second pair of distal crowns),
wherein the surface area of the enlarged proximal cells in the relaxed state is greater than the surface area of the other cells of the basket,
wherein the enlarged proximal cells are configured to allow a thrombus to pass therethrough and into the basket interior.

Optionally, the distal portion is radiopaque.

In some examples, a system for removing objects from an interior lumen of an animal may comprise: a pull wire having a proximal end and a distal end; a distal body comprising a distal body proximal end comprising a distal body proximal junction attached to the pull wire, a distal body distal end comprising a distal body distal junction, a distal body length extending from the distal body proximal end to the distal body distal end, a distal body longitudinal axis extending from the distal body proximal junction to the distal body distal junction, and a distal body height and width perpendicular to the distal body length. The distal body may include a distal body outer body (also referred to herein as the proximal portion of the distal body) extending from the distal body proximal end to the distal body distal end, the distal body outer body comprising the distal body proximal junction and the distal body distal junction, the distal body outer body comprising a distal body outer body perimeter separating a distal body outer body interior from a distal body outer body exterior, the distal body outer body comprising a basket comprised of a plurality of cells spaced about the distal body outer body perimeter and formed by a plurality of basket memory metal strips, wherein at least some of the basket memory metal strips are located at a distal end of the basket, wherein each of the basket strips located at the distal end of the basket have a distal end, and wherein each of the distal ends of the basket strips located at the distal end of the basket converge at, and are attached to, the distal junction. The distal body may also include a distal body inner body (also referred to herein as the distal portion of the distal body) comprised of a plurality of braided mesh openings formed by a plurality of woven linear strands, the distal body inner body having a distal body inner body perimeter, each woven linear strand rotating about the distal body inner body perimeter relative to the distal body longitudinal axis a plurality of times in a helical fashion, the distal body inner body comprising a distal body inner body proximal end and a distal body inner body distal end. Optionally, in the relaxed state, the proximal ends of at least some of the woven linear strands are adjacent to the interior surface of at least some of the basket memory metal strips.

Optionally, the distal body has a relaxed state wherein the distal body has a first height and a first width, and a collapsed state wherein the distal body has a second height and a second width, the second height less than the first height, the second width less than the first width. Optionally, the system further comprises a catheter having an interior, a proximal end leading to the interior and a distal end leading to the interior, the catheter comprised of a biocompatible material and configured to envelope the distal body when the distal body is in the collapsed state. Optionally, at least some (preferably all) of the woven linear strand comprises a free proximal end and a distal end attached to the distal junction. Optionally, in the relaxed state, the median surface area of the cells is larger than the median surface area of the braided mesh openings. Optionally, the distal body inner body and the distal body outer body each have a length generally parallel to the distal body length, the distal body inner body and distal body outer body lengths configured to elongate upon moving from the relaxed state to the collapsed state. Optionally, upon moving from the relaxed state to the collapsed state, the length of the distal body inner body is configured to elongate a greater percentage than the length of the distal body outer body. Optionally, upon moving from the relaxed state to the collapsed state, the distal body inner body is configured to elongate proximally within the distal body outer body interior toward the distal body proximal junction. Optionally, in the relaxed state, the distal body inner body proximal end is located a first distance distal from the distal body proximal junction. Optionally, in the collapsed state, the distal body inner body proximal end is located a second distance distal from the proximal junction, the second distance less than the first distance. Optionally, in the collapsed state and in the relaxed state, the distal body inner body is located in the distal body outer body interior. Optionally, the woven linear strands rotate about the distal body inner body perimeter relative to the distal body longitudinal axis a fewer number of times per unit of length in the collapsed state as compared to the relaxed state. Optionally, the basket memory metal strips are located on the distal body outer body perimeter and comprise an interior surface facing the distal body outer body interior and an exterior surface opposite the interior surface, and further wherein in the relaxed state, at least a portion of the woven linear strands are adjacent to and preferably contact the interior surface of at least a portion of the basket memory metal strips. Optionally, the proximal ends of the woven linear strands are free floating within the distal body outer body interior.

Optionally, the distal junction is the sole connection point of the distal body inner body to the distal body outer body. Optionally, the distal junction is a tube. Optionally, in the relaxed state, the distal body outer body, but not the distal body inner body, is configured to alter the shape of a curved intracranial artery. Optionally, in the relaxed state, the distal body inner body is more flexible than the distal body outer body and wherein, in the relaxed state, the median radial force of the distal body inner body is substantially less than the median radial force of the distal body outer body. Optionally, wherein the distal body inner body comprises a distal body inner body height and a distal body inner body width, wherein the distal body inner body in the relaxed state comprises a distal body inner body distal tapered region in which the distal body inner body height and the distal body inner body width decrease as the strand distal ends approach the distal junction, wherein the distal body outer body comprises a distal body outer body height and a distal body outer body width, and further wherein the distal body outer body comprises a tapered region in which the distal body inner body height and the distal body inner body width decrease as the distal ends of the basket memory metal strips located at the distal end of the basket approach the distal junction. Optionally, in the relaxed state, the distal body inner body impedes blood flow to a greater extent than the distal body outer body when the distal body outer body and the distal body inner body are placed in a blood vessel. Optionally, the distal body inner body is configured to reduce blood flow by at least 25% when the distal body inner body is placed in a blood vessel. Optionally, in the relaxed state, the distal body outer body comprises a first pair of distal crowns not attached to another cell of the basket and pointing generally in the distal direction, the distal crowns in the first pair of distal crowns located approximately the same distance from the proximal junction and between 150 degrees and 180 degrees relative to each other. Optionally, the basket further comprises a second pair of distal crowns not attached to another cell of the basket and pointing generally in the distal direction, the second pair of distal crowns located distally relative to the first pair of distal crowns, each of the distal crowns in the second pair of distal crowns located between 60 degrees and 90 degrees relative to a distal crown in the first pair of distal crowns. Optionally, distal crowns in the second pair of distal crowns are located approximately the same distance from the proximal junction, each of the distal crowns forming a portion of a cell. Optionally, each distal crown in the first and second pair of distal crowns forms part of a different enlarged cell. Optionally, each enlarged cell has a center, wherein the centers of the enlarged cells of the first pair of distal crowns are between 150 degrees and 180 degrees relative to each other and between 60 degrees and 90 degrees relative to the centers of the enlarged cells of the second pair of distal crowns. Optionally, the surface area of the enlarged cells in the relaxed state is greater than the surface area of the other cells of the basket. Optionally, the enlarged cells are configured to allow a thrombus to pass therethrough and into the basket interior. Optionally, in the relaxed state, the distal body inner body is located distally relative to the first and second pair of distal crowns. Optionally, the distal body inner body is radiopaque. Optionally, in the relaxed state, the distal body inner body length is no more than about 33% of the distal body outer body length.

In some examples, t a system for removing objects from an interior lumen of an animal may comprise: a pull wire having a proximal end and a distal end; a distal body comprising a distal body proximal end comprising a distal body proximal junction attached to the pull wire, a distal body distal end comprising a distal body distal junction, a distal body length extending from the distal body proximal end to the distal body distal end, a distal body longitudinal axis extending from the distal body proximal junction to the distal body distal junction, and a distal body height and width perpendicular to the distal body length. The distal body may include a distal body outer body extending from the distal body proximal end to the distal body distal end, the distal body outer body comprising the distal body proximal junction and the distal body distal junction, the distal body outer body comprising a distal body outer body perimeter separating a distal body outer body interior from a distal body outer body exterior, the distal body outer body comprising a basket comprised of a plurality of cells spaced about the distal body outer body perimeter and formed by a plurality of basket memory metal strips. Optionally, at least some of the basket memory metal strips are located at a distal end of the basket, wherein each of the basket strips located at the distal end of the basket have a distal end, and wherein each of the distal ends of the basket strips located at the distal end of the basket converge at, and are attached to, the distal junction. Optionally, the system further includes a distal body inner body comprised of a plurality of braided mesh openings formed by a plurality of woven linear strands, the distal body inner body having a distal body inner body perimeter, each woven linear strand rotating about the distal body inner body perimeter relative to the distal body longitudinal axis a plurality of times in a helical fashion, the distal body inner body comprising a distal body inner body proximal end and a distal body inner body distal end. Optionally, the distal body has a relaxed state wherein the distal body has a first height and a first width, and a collapsed state wherein the distal body has a second height and a second width, the second height less than the first height, the second width less than the first width. Optionally, the system further comprises a catheter having an interior, a proximal end leading to the interior and a distal end leading to the interior, the catheter comprised of a biocompatible material and configured to envelope the distal body when the distal body is in the collapsed state. Optionally, the woven linear strands comprise a proximal end and a distal end, and at least some (preferably all) of the distal ends of the woven linear strands are attached to the distal junction. Optionally, in the relaxed state, the median surface area of the cells is larger than the median surface area of the braided mesh openings. Optionally, the distal body inner body and the distal body outer body each have a length generally parallel to the distal body length, the distal body inner body and distal body outer body lengths configured to elongate upon moving from the relaxed state to the collapsed state. Optionally, upon moving from the relaxed state to the collapsed state, the length of the distal body inner body is configured to elongate a greater percentage than the length of the distal body outer body. Optionally, upon moving from the relaxed state to the collapsed state, the distal body inner body is configured to elongate proximally within the distal body outer body interior toward the distal body proximal junction. Optionally, in the relaxed state, the distal body inner body proximal end is located a first distance distal from the distal body proximal junction. Optionally, in the collapsed state, the distal body inner body proximal end is located a second distance distal from the proximal junction, the second distance less than the first distance. Optionally, in the collapsed state and in the relaxed state, the distal body inner body is located in the distal body outer body interior. Optionally, the woven linear strands rotate about the distal body inner body perimeter relative to the distal body longitudinal axis a fewer number of times per unit of length in the collapsed state as compared to the relaxed state. Optionally, the proximal ends of at least some (preferably all) of the woven linear strands converge at and are attached to a distal body inner body proximal junction. Optionally, the distal body inner body proximal junction forms the proximal end of the distal body inner body and is free floating within the distal body outer body interior.

Optionally, the basket memory metal strips are located on the distal body outer body perimeter and comprise an interior surface facing the distal body outer body interior and an exterior surface opposite the interior surface, and further wherein in the relaxed state, at least a portion of the woven linear strands contact the interior surface of at least a portion of the basket memory metal strips. Optionally, the distal body inner body proximal junction is located approximately in the center of the distal body height and the distal body width in the relaxed state. Optionally, the distal body inner body in the relaxed state comprises a distal body inner body proximal tapered region in which the distal body inner body height and the distal body inner body width decrease as the proximal ends of the woven linear strands approach the distal body inner body proximal junction. Optionally, the distal junction is the sole connection point of the distal body inner body to the distal body outer body. Optionally, the distal body outer body further comprises a plurality of proximal strips, each proximal strip having a distal end attached to a proximal crown of a cell and a proximal end, the proximal ends of the proximal strips converging at the distal body proximal junction. Optionally, the proximal ends of each of the woven linear strands converge at and are attached to the distal body inner body proximal junction and further wherein the distal ends of each of the woven linear strands converge at and are attached to the distal body distal junction. Optionally, in the relaxed state, the distal body inner body is more flexible than the distal body outer body and wherein, in the relaxed state, the median radial force of the distal body inner body is substantially less than the median radial force of the distal body outer body. Optionally, the distal body inner body comprises a distal body inner body height and a distal body inner body width, wherein the distal body inner body in the relaxed state comprises a distal body inner body distal tapered region in which the distal body inner body height and the distal body inner body width decrease as the strand distal ends approach the distal junction, wherein the distal body outer body comprises a distal body outer body height and a distal body outer body width, and further wherein the distal body outer body comprises a tapered region in which the distal body inner body height and the distal body inner body width decrease as the distal ends of the basket memory metal strips located at the distal end of the basket approach the distal junction. Optionally, in the relaxed state, the distal body inner body impedes blood flow to a greater extent than the distal body outer body when the distal body outer body and the distal body inner body are placed in a blood vessel. Optionally, the distal body inner body is configured to reduce blood flow by at least 25% when the distal body inner body is placed in a blood vessel. Optionally, in the relaxed state, the distal body outer body comprises a first pair of distal crowns not attached to another cell of the basket and pointing generally in the distal direction, the distal crowns in the first pair of distal crowns located approximately the same distance from the proximal junction and between 150 degrees and 180 degrees relative to each other. Optionally, the basket further comprises a second pair of distal crowns not attached to another cell of the basket and pointing generally in the distal direction. Optionally, the second pair of distal crowns are located distally relative to the first pair of distal crowns. Optionally, each of the distal crowns in the second pair of distal crowns is located between 60 degrees and 90 degrees relative to a distal crown in the first pair of distal crowns. Optionally, the distal crowns in the second pair of distal crowns located approximately the same distance from the proximal junction, each of the distal crowns forming a portion of a cell. Optionally, each distal crown in the first and second pair of distal crowns forms part of a different enlarged cell, each enlarged cell having a center. Optionally, the centers of the enlarged cells of the first pair of distal crowns are between 150 degrees and 180 degrees relative to each other and between 60 degrees and 90 degrees relative to the centers of the enlarged cells of the second pair of distal crowns. Optionally, the surface area of the enlarged cells in the relaxed state is greater than the surface area of the other cells of the basket. Optionally, the enlarged cells are configured to allow a thrombus to pass therethrough and into the basket interior. Optionally, in the relaxed state, the distal body inner body is located distally relative to the first and second pair of distal crowns. Optionally, the distal body inner body is radiopaque. Optionally, in the relaxed state, the distal body inner body length is no more than about 33% of the distal body outer body length.

In some examples, a system for removing objects from an interior lumen of an animal may comprise: a pull wire having a proximal end and a distal end; a distal body comprising a distal body proximal end comprising a distal body proximal junction (which may be attached to the pull wire), a distal body distal end comprising a distal body distal junction, a distal body length extending from the distal body proximal end to the distal body distal end, a distal body longitudinal axis extending from the distal body proximal junction to the distal body distal junction, and a distal body height and width perpendicular to the distal body length. The distal body may comprise a distal body outer body extending from the distal body proximal end to the distal body distal end, the distal body outer body comprising the distal body proximal junction and the distal body distal junction. The distal body outer body may comprise a distal body outer body perimeter separating a distal body outer body interior from a distal body outer body exterior. The distal body outer body may comprise a basket comprised of a plurality of cells spaced about the distal body outer body perimeter and formed by a plurality of basket memory metal strips. At least some of the basket memory metal strips may be located at a distal end of the basket. Each of the basket strips located at the distal end of the basket may have a distal end, and each of the distal ends of the basket strips located at the distal end of the basket may converge at, and be attached to, the distal junction. The distal body may also include a distal body inner body comprised of a plurality of braided mesh openings formed by a plurality of woven linear strands. The distal body inner body may have a distal body inner body perimeter. Each woven linear strand may rotate about the distal body inner body perimeter relative to the distal body longitudinal axis a plurality of times in a helical fashion. The distal body inner body may comprise a distal body inner body proximal end and a distal body inner body distal end. Optionally, the distal body has a relaxed state wherein the distal body has a first height and a first width, and a collapsed state wherein the distal body has a second height and a second width, the second height less than the first height, the second width less than the first width. Optionally, the system further comprises a catheter having an interior, a proximal end leading to the interior and a distal end leading to the interior, the catheter comprised of a biocompatible material and configured to envelope the distal body when the distal body is in the collapsed state. Optionally, the woven linear strands comprise a proximal end and a distal end, and at least some of the distal ends of the woven linear strands are attached to the distal junction. Optionally, in the relaxed state, the median surface area of the cells is larger than the median surface area of the braided mesh openings. Optionally, the distal body inner body and the distal body outer body each have a length generally parallel to the distal body length, and optionally, the distal body inner body and distal body outer body lengths are configured to elongate upon moving from the relaxed state to the collapsed state. Optionally, upon moving from the relaxed state to the collapsed state, the length of the distal body inner body is configured to elongate a greater percentage than the length of the distal body outer body. Optionally, upon moving from the relaxed state to the collapsed state, the distal body inner body is configured to elongate proximally within the distal body outer body interior toward the distal body proximal junction. Optionally, in the relaxed state, the distal body inner body proximal end is located a first distance distal from the distal body proximal junction. Optionally, in the collapsed state, the distal body inner body proximal end is located a second distance distal from the distal body proximal junction, the second distance less than the first distance. Optionally, in the collapsed state and in the relaxed state, the distal body inner body is located in the distal body outer body interior. Optionally, the woven linear strands rotate about the distal body inner body perimeter relative to the distal body longitudinal axis a fewer number of times per unit of length in the collapsed state as compared to the relaxed state. Optionally, the proximal ends of at least some of the woven linear strands converge at and are attached to a distal body inner body proximal junction. Optionally, the distal body inner body proximal junction forms the proximal end of the distal body inner body.

Optionally, the system further comprises a tether connecting the distal body proximal junction to the distal body inner body proximal junction. Optionally, the tether is a segment of the pull wire. Optionally, the tether is comprised of a conductive material. Optionally, the tether is comprised of a synthetic polymer. Optionally, the tether comprises a proximal end attached to the distal body proximal junction and a distal end attached to the distal body inner body proximal junction. (The attachment can be soldering, welding, crimping, etc.). Optionally, the tether is located approximately in the center of the distal body height and the distal body width of the distal body when the distal body is in the relaxed state and the tether is generally parallel to the distal body longitudinal axis when the distal body is in the relaxed state. Optionally, the basket memory metal strips are located on the distal body outer body perimeter and comprise an interior surface facing the distal body outer body interior and an exterior surface opposite the interior surface, and further wherein in the relaxed state, at least some of the woven linear strands contact the interior surface of at least some of the basket memory metal strips. Optionally, the distal body inner body proximal junction is located approximately in the center of the distal body height and the distal body width in the relaxed state. Optionally, the distal body inner body comprises a distal body inner body height and a distal body inner body width and wherein the distal body inner body in the relaxed state comprises a distal body inner body proximal tapered region in which the distal body inner body height and the distal body inner body width decrease as the proximal ends of the woven linear strands approach the distal body inner body proximal junction. Optionally, the distal junction is the sole connection point of the distal body inner body to the distal body outer body. Optionally, the distal body outer body further comprises a plurality of proximal strips, each proximal strip having a distal end attached to a proximal crown of a cell and a proximal end, the proximal ends of the proximal strips converging at the distal body proximal junction. Optionally, the proximal ends of each of the woven linear strands converge at and are attached to the distal body inner body proximal junction and further wherein the distal ends of each of the woven linear strands converge at and are attached to the distal body distal junction. Optionally, in the relaxed state, the distal body inner body is more flexible than the distal body outer body and wherein, in the relaxed state, the median radial force of the distal body inner body is substantially less than the median radial force of the distal body outer body. Optionally, the distal body inner body comprises a distal body inner body height and a distal body inner body width, wherein the distal body inner body in the relaxed state comprises a distal body inner body distal tapered region in which the distal body inner body height and the distal body inner body width decrease as the strand distal ends approach the distal junction, wherein the distal body outer body comprises a distal body outer body height and a distal body outer body width, and further wherein the distal body outer body comprises a tapered region in which the distal body inner body height and the distal body inner body width decrease as the distal ends of the basket memory metal strips located at the distal end of the basket approach the distal junction. Optionally, in the relaxed state, the distal body inner body impedes blood flow to a greater extent than the distal body outer body when the distal body outer body and the distal body inner body are placed in a blood vessel. Optionally, the distal body inner body is configured to reduce blood flow by at least 25% when the distal body inner body is placed in a blood vessel. Optionally, in the relaxed state, the distal body outer body comprises a first pair of distal crowns not attached to another cell of the basket and pointing generally in the distal direction, the distal crowns in the first pair of distal crowns located approximately the same distance from the proximal junction and located between 150 degrees and 180 degrees relative to each other, and further wherein the basket further comprises a second pair of distal crowns not attached to another cell of the basket and pointing generally in the distal direction, the second pair of distal crowns located distally relative to the first pair of distal crowns, each of the distal crowns in the second pair of distal crowns located between 60 degrees and 90 degrees relative to a distal crown in the first pair of distal crowns, the distal crowns in the second pair of distal crowns located approximately the same distance from the distal body proximal junction, each of the distal crowns forming a portion of a cell, wherein each distal crown in the first and second pair of distal crowns forms part of a different enlarged cell, each enlarged cell having a center, wherein the centers of the enlarged cells of the first pair of distal crowns are between 150 degrees and 180 degrees relative to each other and between 60 degrees and 90 degrees relative to the centers of the enlarged cells of the second pair of distal crowns, wherein the surface area of the enlarged cells in the relaxed state is greater than the surface area of the other cells of the basket, and wherein the enlarged cells are configured to allow a thrombus to pass therethrough and into the basket interior.

Optionally, in the relaxed state, the distal body inner body is located distally relative to the first and second pair of distal crowns. Optionally, the distal body inner body is radiopaque. Optionally, in the relaxed state, the distal body inner body length is no more than about 33% of the distal body outer body length.

In some examples, a system for removing objects from an interior lumen of an animal may comprise: a pull wire having a proximal end and a distal end; a distal body attached to the pull wire and comprising a distal body proximal end comprising a distal body proximal junction, a distal body distal end comprising a distal body distal junction, a distal body length extending from the distal body proximal end to the distal body distal end, a distal body longitudinal axis extending from the distal body proximal junction to the distal body distal junction, and a distal body height and width perpendicular to the distal body length. The distal body may comprise a distal body outer body extending from the distal body proximal end to the distal body distal end, the distal body outer body comprising the distal body proximal junction and the distal body distal junction, the distal body outer body comprising a distal body outer body perimeter separating a distal body outer body interior from a distal body outer body exterior, the distal body outer body comprising a basket comprised of a plurality of cells spaced about the distal body outer body perimeter and formed by a plurality of basket memory metal strips. Optionally, at least some of the basket memory metal strips are located at a distal end of the basket. Optionally, each of the basket memory metal strips located at the distal end of the basket have a distal end. Optionally, each of the distal ends of the basket memory metal strips located at the distal end of the basket converge at, and are attached to, the distal body distal junction. The distal body may also include a distal body inner body comprised of a plurality of braided mesh openings formed by a plurality of woven linear strands, the distal body inner body having a distal body inner body perimeter, each woven linear strand rotating about the distal body inner body perimeter relative to the distal body longitudinal axis a plurality of times in a helical fashion, the distal body inner body comprising a distal body inner body proximal end and a distal body inner body distal end.

Optionally, the distal body has a relaxed state wherein the distal body has a first height and a first width, and a collapsed state wherein the distal body has a second height and a second width, the second height less than the first height, the second width less than the first width. Optionally, the system further comprises a catheter having an interior, a proximal end leading to the interior and a distal end leading to the interior, the catheter comprised of a biocompatible material and configured to envelope the distal body when the distal body is in the collapsed state. Optionally, the woven linear strands comprise a proximal end and a distal end, and at least some of the distal ends of the woven linear strands are attached to the distal junction. Optionally, in the relaxed state, the median surface area of the cells is larger than the median surface area of the braided mesh openings. Optionally, the distal body inner body and the distal body outer body each have a length generally parallel to the distal body length, the distal body inner body and distal body outer body lengths configured to elongate upon moving from the relaxed state to the collapsed state. Optionally, upon moving from the relaxed state to the collapsed state, the length of the distal body inner body is configured to elongate a greater percentage than the length of the distal body outer body. Optionally, upon moving from the relaxed state to the collapsed state, the distal body inner body is configured to elongate proximally within the distal body outer body interior toward the distal body proximal junction. Optionally, in the relaxed state, the distal body inner body proximal end is located a first distance distal from the distal body proximal junction. Optionally, in the collapsed state, the distal body inner body proximal end is located a second distance distal from the distal body proximal junction, the second distance less than the first distance. Optionally, in the collapsed state and in the relaxed state, the distal body inner body is located in the distal body outer body interior. Optionally, the woven linear strands rotate about the distal body inner body perimeter relative to the distal body longitudinal axis a fewer number of times per unit of length in the collapsed state as compared to the relaxed state. Optionally, the distal body inner body comprises an active agent when the distal body inner body is in the catheter interior.

Optionally, the active agent is selected from the group consisting of a reolytic agent, a neuroprotective agent and combinations thereof. Optionally, the active agent is located in the distal body inner body interior. Optionally, the active agent is too large to pass through the braided mesh openings when the distal body inner body is located in the catheter interior. Optionally, the woven linear strands are coated with the active agent. Optionally, the basket memory metal strips are not coated with the active agent. Optionally, the proximal ends of at least some of the woven linear strands converge at and are attached to a distal body inner body proximal junction, the distal body inner proximal junction located distal relative to the distal body proximal junction. Optionally, the distal body inner body proximal junction forms the proximal end of the distal body inner body. Optionally, the system further comprises a tether connecting the distal body proximal junction to the distal body inner body proximal junction. Optionally, the tether is a segment of the pull wire. Optionally, the tether is comprised of a conductive material. Optionally, the tether is comprised of a synthetic polymer. Optionally, the tether comprises a proximal end attached to the distal body proximal junction and a distal end attached to the distal body inner body proximal junction. Optionally, the tether is located approximately in the center of the distal body height and the distal body width of the distal body when the distal body is in the relaxed state and the tether is generally parallel to the distal body longitudinal axis when the distal body is in the relaxed state. Optionally, the distal body outer body further comprises a plurality of proximal strips, each proximal strip having a distal end attached to a proximal crown of a cell and a proximal end, the proximal ends of the proximal strips converging at the distal body proximal junction. Optionally, the proximal ends of each of the woven linear strands converge at and are attached to the distal body inner body proximal junction and further wherein the distal ends of each of the woven linear strands converge at and are attached to the distal body distal junction. Optionally, the basket memory metal strips are located on the distal body outer body perimeter and comprise an interior surface facing the distal body outer body interior and an exterior surface opposite the interior surface, and further wherein in the relaxed state, at least some of the woven linear strands contact the interior surface of at least some of the basket memory metal strips. Optionally, the distal junction is the sole connection point of the distal body inner body to the distal body outer body. Optionally, in the relaxed state, the distal body inner body is more flexible than the distal body outer body and wherein, in the relaxed state, the median radial force of the distal body inner body is substantially less than the median radial force of the distal body outer body. Optionally, the distal body inner body comprises a distal body inner body height and a distal body inner body width, wherein the distal body inner body in the relaxed state comprises a distal body inner body distal tapered region in which the distal body inner body height and the distal body inner body width decrease as the strand distal ends approach the distal junction, wherein the distal body outer body comprises a distal body outer body height and a distal body outer body width, and further wherein the distal body outer body comprises a tapered region in which the distal body inner body height and the distal body inner body width decrease as the distal ends of the basket memory metal strips located at the distal end of the basket approach the distal junction. Optionally, in the relaxed state, the distal body inner body impedes blood flow to a greater extent than the distal body outer body when the distal body outer body and the distal body inner body are placed in a blood vessel. Optionally, the distal body inner body is configured to reduce blood flow by at least 25% when the distal body inner body is placed in a blood vessel. Optionally, in the relaxed state, the distal body outer body comprises a first pair of distal crowns not attached to another cell of the basket and pointing generally in the distal direction, the distal crowns in the first pair of distal crowns located approximately the same distance from the proximal junction and located between 150 degrees and 180 degrees relative to each other, and further wherein the basket further comprises a second pair of distal crowns not attached to another cell of the basket and pointing generally in the distal direction, the second pair of distal crowns located distally relative to the first pair of distal crowns, each of the distal crowns in the second pair of distal crowns located between 60 degrees and 90 degrees relative to a distal crown in the first pair of distal crowns, the distal crowns in the second pair of distal crowns located approximately the same distance from the proximal junction, each of the distal crowns forming a portion of a cell, wherein each distal crown in the first and second pair of distal crowns forms part of a different enlarged cell, each enlarged cell having a center, wherein the centers of the enlarged cells of the first pair of distal crowns are between 150 degrees and 180 degrees relative to each other and between 60 degrees and 90 degrees relative to the centers of the enlarged cells of the second pair of distal crowns, wherein the surface area of the enlarged cells in the relaxed state is greater than the surface area of the other cells of the basket, wherein the enlarged cells are configured to allow a thrombus to pass therethrough and into the basket interior.

Optionally, in the relaxed state, the distal body inner body is located distally relative to the first and second pair of distal crowns. Optionally, the distal body inner body is radiopaque. Optionally, in the relaxed state, the distal body inner body length is no more than about 33% of the distal body outer body length.

In some examples, a system for removing objects from an interior lumen of an animal may comprise: a pull wire having a proximal end and a distal end; a distal body attached to the pull wire and comprising a distal body proximal end comprising a distal body proximal junction, a distal body distal end comprising a distal body distal junction, a distal body length extending from the distal body proximal end to the distal body distal end, a distal body longitudinal axis extending from the distal body proximal junction to the distal body distal junction, and a distal body height and width perpendicular to the distal body length. The distal body may comprise a distal body outer body extending from the distal body proximal end to the distal body distal end, the distal body outer body comprising the distal body proximal junction and the distal body distal junction, the distal body outer body comprising a distal body outer body perimeter separating a distal body outer body interior from a distal body outer body exterior, the distal body outer body comprising a basket comprised of a plurality of cells spaced about the distal body outer body perimeter and formed by a plurality of basket memory metal strips. Optionally, at least some of the basket memory metal strips are located at a distal end of the basket. Optionally, each of the basket memory metal strips located at the distal end of the basket have a distal end. Optionally, each of the distal ends of the basket memory strips located at the distal end of the basket converge at, and are attached to, the distal body distal junction. Optionally, the distal body may also include a distal body inner body comprised of a plurality of braided mesh openings formed by a plurality of woven linear strands, the distal body inner body having a distal body inner body perimeter, each woven linear strand rotating about the distal body inner body perimeter relative to the distal body longitudinal axis a plurality of times in a helical fashion, the distal body inner body comprising a distal body inner body proximal end and a distal body inner body distal end.

Optionally, the distal body has a relaxed state wherein the distal body has a first height and a first width, and a collapsed state wherein the distal body has a second height and a second width, the second height less than the first height, the second width less than the first width. Optionally, the system further comprises a catheter having an interior, a proximal end leading to the interior and a distal end leading to the interior, the catheter comprised of a biocompatible material and configured to envelope the distal body when the distal body is in the collapsed state. Optionally, the woven linear strands comprise a proximal end and a distal end, and at least some of the distal ends of the woven linear strands are attached to the distal junction. Optionally, in the relaxed state, the median surface area of the cells is larger than the median surface area of the braided mesh openings. Optionally, the distal body inner body and the distal body outer body each have a length generally parallel to the distal body length, the distal body inner body and distal body outer body lengths configured to elongate upon moving from the relaxed state to the collapsed state. Optionally, upon moving from the relaxed state to the collapsed state, the length of the distal body inner body is configured to elongate a greater percentage than the length of the distal body outer body. Optionally, upon moving from the relaxed state to the collapsed state, the distal body inner body is configured to elongate proximally within the distal body outer body interior toward the distal body proximal junction. Optionally, in the relaxed state, the distal body inner body proximal end is located a first distance distal from the distal body proximal junction. Optionally, in the collapsed state, the distal body inner body proximal end is located a second distance distal from the distal body proximal junction, the second distance less than the first distance. Optionally, in the collapsed state and in the relaxed state, the distal body inner body is located in the distal body outer body interior. Optionally, the woven linear strands rotate about the distal body inner body perimeter relative to the distal body longitudinal axis a fewer number of times per unit of length in the collapsed state as compared to the relaxed state. Optionally, the pull wire is in the form of an active agent delivery catheter having an open proximal end and an open distal end, the active agent delivery catheter configured to deliver an active agent to the distal body.

Optionally, the active agent delivery catheter distal end is located distal relative to the distal body proximal junction. Optionally, the active agent delivery catheter comprises a wall, wherein the distal body outer body further comprises a plurality of proximal strips, each proximal strip having a distal end attached to a proximal crown of a cell and a proximal end and a proximal end attached to the wall of the active agent delivery catheter.

In some examples, a system for removing objects from an interior lumen of an animal may include a pull wire having a proximal end and a distal end. Optionally, the system may also include a distal body attached to the pull wire and comprising a distal body proximal end comprising a distal body proximal junction, a distal body distal end comprising a distal body distal junction, a distal body length extending from the distal body proximal end to the distal body distal end, a distal body longitudinal axis extending from the distal body proximal junction to the distal body distal junction, and a distal body height and width perpendicular to the distal body length. Optionally, the distal body further comprises a distal body outer body that may extend from the distal body proximal end to the distal body distal end, the distal body outer body may comprise the distal body proximal junction and the distal body distal junction, the distal body outer body may comprise a distal body outer body perimeter separating a distal body outer body interior from a distal body outer body exterior, the distal body outer body may comprise a basket comprised of a plurality of cells spaced about the distal body outer body perimeter and formed by a plurality of basket memory metal strips. Optionally, at least some of the basket memory metal strips are located at a distal end of the basket, wherein each of the basket memory metal strips located at the distal end of the basket have a distal end, and wherein each of the distal ends of the basket memory metal strips located at the distal end of the basket converge at, and are attached to, the distal body distal junction. Optionally, the distal body further comprises a distal body inner body that may be comprised of a plurality of braided mesh openings formed by a plurality of woven linear strands, the distal body inner body may have a distal body inner body perimeter, each woven linear strand rotating about the distal body inner body perimeter relative to the distal body longitudinal axis a plurality of times in a helical fashion, the distal body inner body may comprise a distal body inner body proximal end and a distal body inner body distal end.

Optionally, the distal body has a relaxed state wherein the distal body has a first height and a first width, and a collapsed state wherein the distal body has a second height and a second width, the second height less than the first height, the second width less than the first width. Optionally, the system further comprises a catheter having an interior, a proximal end leading to the interior and a distal end leading to the interior, the catheter comprised of a biocompatible material and configured to envelope the distal body when the distal body is in the collapsed state. Optionally, the woven linear strands comprise a proximal end and a distal end, and at least some of the distal ends of the woven linear strands are attached to the distal body distal junction. Optionally, in the relaxed state, the median surface area of the cells is larger than the median surface area of the braided mesh openings. Optionally, the distal body inner body and the distal body outer body each have a length generally parallel to the distal body length, the distal body inner body and distal body outer body lengths configured to elongate upon moving from the relaxed state to the collapsed state. Optionally, upon moving from the relaxed state to the collapsed state, the length of the distal body inner body is configured to elongate a greater percentage than the length of the distal body outer body. Optionally, upon moving from the relaxed state to the collapsed state, the distal body inner body is configured to elongate proximally within the distal body outer body interior toward the distal body proximal junction. Optionally, in the relaxed state, the distal body inner body proximal end is located a first distance distal from the distal body proximal junction. Optionally, in the collapsed state, the distal body inner body proximal end is located a second distance distal from the distal body proximal junction, the second distance less than the first distance. Optionally, in the collapsed state and in the relaxed state, the distal body inner body is located in the distal body outer body interior. Optionally, the woven linear strands rotate about the distal body inner body perimeter relative to the distal body longitudinal axis a fewer number of times per unit of length in the collapsed state as compared to the relaxed state. Optionally, the proximal ends of at least some of the woven linear strands converge at and are attached to a distal body inner body proximal junction. Optionally, the distal body inner body proximal junction forms the proximal end of the distal body inner body. Optionally, the system further comprises a tether connecting the distal body proximal junction to the distal body inner body proximal junction, the tether comprising a segment in the form of a helical coil, the helical coil having a coil length generally parallel to the distal body length, the helical coil having an expanded state in which the helical coil has a first length and a relaxed state in which the helical coil has a second length, the first length greater than the second length.

Optionally, the helical coil is adjacent to the distal body inner body proximal junction. Optionally, the helical coil is configured to move to the expanded state when tension is exerted on the tether. Optionally, the tether is a segment of the pull wire. Optionally, the tether is comprised of a conductive material. Optionally, the tether is comprised of a synthetic polymer. Optionally, the tether comprises a proximal end attached to the distal body proximal junction and a distal end attached to the distal body inner body proximal junction. Optionally, the tether is located approximately in the center of the distal body height and the distal body width when the distal body is in the relaxed state and the tether is generally parallel to the distal body longitudinal axis when the distal body is in the relaxed state. Optionally, the basket memory metal strips are located on the distal body outer body perimeter and comprise an interior surface facing the distal body outer body interior and an exterior surface opposite the interior surface, and further wherein in the relaxed state, at least some of the woven linear strands contact the interior surface of at least some of the basket memory metal strips. Optionally, the distal body inner body proximal junction is located approximately in the center of the distal body height and the distal body width in the relaxed state. Optionally, the distal body inner body comprises a distal body inner body height and a distal body inner body width and wherein the distal body inner body in the relaxed state comprises a distal body inner body proximal tapered region in which the distal body inner body height and the distal body inner body width decrease as the proximal ends of the woven linear strands approach the distal body inner body proximal junction. Optionally, in the relaxed state, the basket does not have any free crowns that point generally in the proximal direction. Optionally, the distal body outer body further comprises a plurality of proximal strips, each proximal strip having a distal end attached to a proximal crown of a cell and a proximal end, the proximal ends of the proximal strips converging at the distal body proximal junction. Optionally, the proximal ends of each of the woven linear strands converge at and are attached to the distal body inner body proximal junction and further wherein the distal ends of each of the woven linear strands converge at and are attached to the distal body distal junction. Optionally, in the relaxed state, the distal body inner body is more flexible than the distal body outer body and wherein, in the relaxed state, the median radial force of the distal body inner body is substantially less than the median radial force of the distal body outer body. Optionally, the distal body inner body comprises a distal body inner body height and a distal body inner body width, wherein the distal body inner body in the relaxed state comprises a distal body inner body distal tapered region in which the distal body inner body height and the distal body inner body width decrease as the woven linear strand distal ends approach the distal body distal junction, wherein the distal body outer body comprises a distal body outer body height and a distal body outer body width, and further wherein the distal body outer body comprises a tapered region in which the distal body outer body height and the distal body outer body width decrease as the distal ends of the basket memory metal strips located at the distal end of the basket approach the distal body distal junction. Optionally, in the relaxed state, the distal body inner body impedes blood flow to a greater extent than the distal body outer body when the distal body outer body and the distal body inner body are placed in a blood vessel. Optionally, wherein, prior to removal of an obstruction, the distal body inner body is configured to automatically reduce blood flow when the distal body inner body is placed in a blood vessel.

Optionally, in the relaxed state, the distal body outer body comprises a first pair of distal crowns not attached to another cell of the basket and pointing generally in the distal direction, the distal crowns in the first pair of distal crowns located approximately the same distance from the distal body proximal junction and located between 150 degrees and 180 degrees relative to each other, and further wherein the basket further comprises a second pair of distal crowns not attached to another cell of the basket and pointing generally in the distal direction, the second pair of distal crowns located distally relative to the first pair of distal crowns, each of the distal crowns in the second pair of distal crowns located between 60 degrees and 90 degrees relative to a distal crown in the first pair of distal crowns, the distal crowns in the second pair of distal crowns located approximately the same distance from the distal body proximal junction, each of the distal crowns forming a portion of a cell, wherein each distal crown in the first and second pair of distal crowns forms part of a different enlarged cell, each enlarged cell having a center, wherein the centers of the enlarged cells of the first pair of distal crowns are between 150 degrees and 180 degrees relative to each other and between 60 degrees and 90 degrees relative to the centers of the enlarged cells of the second pair of distal crowns, wherein the enlarged cells are configured to allow a thrombus to pass therethrough and into the basket interior. Optionally, in the relaxed state, the distal body inner body proximal junction is located distally relative to the first and second pair of distal crowns. Optionally, the distal body inner body is radiopaque. Optionally, in the relaxed state, the distal body inner body length is no more than about 33% of the distal body outer body length. Optionally, the system further comprises a lead wire extending distally from the distal body distal junction. Optionally, the distal body inner body proximal end is substantially closed.

The present disclosure provides a system for removing objects from an interior lumen of an animal. The system includes a pull wire that may have a proximal end and a distal end; a distal body that is be attached to the pull wire and includes a distal body proximal end comprising a distal body proximal junction, a distal body distal end comprising a distal body distal junction, a distal body length extending from the distal body proximal end to the distal body distal end, a distal body longitudinal axis extending from the distal body proximal junction to the distal body distal junction, and a distal body height and width perpendicular to the distal body length. The distal body includes a distal body outer body that extends from the distal body proximal end to the distal body distal end, the distal body outer body comprises the distal body proximal junction and the distal body distal junction, the distal body outer body comprises a distal body outer body perimeter separating a distal body outer body interior from a distal body outer body exterior, the distal body outer body comprises a basket comprised of a plurality of cells spaced about the distal body outer body perimeter and formed by a plurality of basket memory metal strips. At least some of the basket memory metal strips are located at a distal end of the basket, each of the basket memory metal strips located at the distal end of the basket have a distal end, and each of the distal ends of the basket memory metal strips located at the distal end of the basket converge at, and are attached to, the distal body distal junction. The distal body includes a distal body inner body comprised of a plurality of braided mesh openings formed by a plurality of woven linear strands, the distal body inner body has a distal body inner body perimeter, each woven linear strand rotating about the distal body inner body perimeter relative to the distal body longitudinal axis a plurality of times in a helical fashion, the distal body inner body comprises a distal body inner body proximal end and a distal body inner body distal end. The distal body has a relaxed state wherein the distal body has a first height and a first width, and a collapsed state wherein the distal body has a second height and a second width, the second height less than the first height, the second width less than the first width. The system further comprises a catheter having an interior, a proximal end leading to the interior and a distal end leading to the interior, the catheter comprised of a biocompatible material and configured to envelope the distal body when the distal body is in the collapsed state. The woven linear strands comprise a proximal end and a distal end, and at least some of the distal ends of the woven linear strands are attached to the distal body distal junction. In the relaxed state, the median surface area of the cells is larger than the median surface area of the braided mesh openings, wherein, the distal body inner body and the distal body outer body each have a length generally parallel to the distal body length, the distal body inner body and distal body outer body lengths configured to elongate upon moving from the relaxed state to the collapsed state. In the collapsed state and in the relaxed state, the distal body inner body is located in the distal body outer body interior. The woven linear strands rotate about the distal body inner body perimeter relative to the distal body longitudinal axis a fewer number of times per unit of length in the collapsed state as compared to the relaxed state. The proximal ends of at least some of the woven linear strands converge at and are attached to a distal body inner body proximal junction. The distal body inner body proximal junction forms the proximal end of the distal body inner body. The system further comprises a proximal tether connecting the distal body proximal junction to the distal body inner body proximal junction. The system further comprises a distal tether connecting the distal body inner body distal junction to the distal body distal junction.

The distal tether comprises a distal helical coil, the distal helical coil having a coil length generally parallel to the distal body length, the distal helical coil having an expanded state in which the distal helical coil has a first length and a relaxed state in which the distal helical coil has a second length, the first length greater than the second length. The distal helical coil is configured to move to the expanded state when tension is exerted on the basket. Optionally, the proximal tether comprises a proximal helical coil, the proximal helical coil having a coil length generally parallel to the distal body length, the proximal helical coil having an expanded state in which the proximal helical coil has a first length and a relaxed state in which the proximal helical coil has a second length, the first length greater than the second length. Optionally, the proximal helical coil is adjacent to the distal body inner body proximal junction. Optionally, the proximal helical coil is configured to move to the expanded state when tension is exerted on the basket. Optionally, the proximal tether is a segment of the pull wire. Optionally, the proximal tether comprises a proximal end attached to the distal body proximal junction and a distal end attached to the distal body inner body proximal junction. Optionally, the proximal and distal tethers are located approximately in the center of the distal body height and the distal body width when the distal body is in the relaxed state and the proximal and distal tethers are generally parallel to the distal body longitudinal axis when the distal body is in the relaxed state. Optionally, the basket memory metal strips are located on the distal body outer body perimeter and comprise an interior surface facing the distal body outer body interior and an exterior surface opposite the interior surface, and further wherein in the relaxed state, at least some of the woven linear strands contact the interior surface of at least some of the basket memory metal strips. Optionally, the distal body inner body proximal junction is located approximately in the center of the distal body height and the distal body width in the relaxed state. Optionally, the distal body inner body comprises a distal body inner body height and a distal body inner body width and wherein the distal body inner body in the relaxed state comprises a distal body inner body proximal tapered region in which the distal body inner body height and the distal body inner body width decrease as the proximal ends of the woven linear strands approach the distal body inner body proximal junction. Optionally, in the relaxed state, the basket does not have any free crowns that point generally in the proximal direction. Optionally, the distal body outer body further comprises a plurality of proximal strips, each proximal strip having a distal end attached to a proximal crown of a cell and a proximal end, the proximal ends of the proximal strips converging at the distal body proximal junction. Optionally, the proximal ends of each of the woven linear strands converge at and are attached to the distal body inner body proximal junction and further wherein the distal ends of each of the woven linear strands converge at and are attached to the distal body distal junction. Optionally, in the relaxed state, the distal body inner body is more flexible than the distal body outer body and wherein, in the relaxed state, the median radial force of the distal body inner body is substantially less than the median radial force of the distal body outer body. Optionally, the distal body inner body comprises a distal body inner body height and a distal body inner body width, wherein the distal body inner body in the relaxed state comprises a distal body inner body distal tapered region in which the distal body inner body height and the distal body inner body width decrease as the woven linear strand distal ends approach the distal body distal junction, optionally the distal body outer body comprises a distal body outer body height and a distal body outer body width, and optionally the distal body outer body comprises a tapered region in which the distal body outer body height and the distal body outer body width decrease as the distal ends of the basket memory metal strips located at the distal end of the basket approach the distal body distal junction. Optionally, in the relaxed state, the distal body inner body impedes blood flow to a greater extent than the distal body outer body when the distal body outer body and the distal body inner body are placed in a blood vessel. Optionally, prior to removal of an obstruction, the distal body inner body is configured to automatically reduce blood flow when the distal body inner body is placed in a blood vessel. Optionally, in the relaxed state, the distal body outer body comprises a first pair of distal crowns not attached to another cell of the basket and pointing generally in the distal direction, the distal crowns in the first pair of distal crowns located approximately the same distance from the distal body proximal junction and located between 150 degrees and 180 degrees relative to each other, and further wherein the basket further comprises a second pair of distal crowns not attached to another cell of the basket and pointing generally in the distal direction, the second pair of distal crowns are optionally located distally relative to the first pair of distal crowns, optionally each of the distal crowns in the second pair of distal crowns located between 60 degrees and 90 degrees relative to a distal crown in the first pair of distal crowns, the distal crowns in the second pair of distal crowns located approximately the same distance from the distal body proximal junction, optionally each of the distal crowns forming a portion of a cell, optionally, each distal crown in the first and second pair of distal crowns forms part of a different enlarged cell, optionally each enlarged cell having a center, optionally the centers of the enlarged cells of the first pair of distal crowns are between 150 degrees and 180 degrees relative to each other and between 60 degrees and 90 degrees relative to the centers of the enlarged cells of the second pair of distal crowns, optionally, the enlarged cells are configured to allow a thrombus to pass therethrough and into the basket interior. Optionally, in the relaxed state, the distal body inner body proximal junction is located distally relative to the first and second pair of distal crowns. Optionally, that the distal body inner body is radiopaque. Optionally in the relaxed state, the distal body inner body length is no more than about 33% of the distal body outer body length. Optionally, the system further comprises a lead wire extending distally from the distal body distal junction. Optionally, the distal body inner body proximal end is substantially closed. Optionally, in the relaxed state, the distal body outer body does not have any free crowns that point generally in the proximal direction. Optionally, upon moving from the relaxed state to the collapsed state, the length of the distal body inner body is configured to elongate a greater percentage than the length of the distal body outer body, upon moving from the relaxed state to the collapsed state, the distal body inner body is configured to elongate proximally within the distal body outer body interior toward the distal body proximal junction, in the relaxed state, optionally the distal body inner body proximal end is located a first distance distal from the distal body proximal junction, in the collapsed state, optionally the distal body inner body proximal end is located a second distance distal from the distal body proximal junction, optionally the second distance less than the first distance.

### BRIEF DESCRIPTION OF THE DRAWINGS

FIG. 1A illustrates a side, elevation view of a memory metal tube prior to being cut by a laser.
FIG. 1B illustrates a side, elevation view of the memory metal tube of FIG. 1A being cut by a laser.
FIG. 2A illustrates a side, elevation view of the memory metal tube of FIG. 1B after being cut by a laser; in FIG. 2A, the tube is shown as though it were flat for purposes of illustrating the cut pattern only.
FIG. 2B illustrates a side, perspective view of the memory metal tube of FIG. 1B after being cut by a laser.
FIG. 2C illustrates another side, perspective view of the memory metal tube of FIG. 1B after being cut by a laser; in FIG. 2C, the tube is rotated as compared to FIG. 2B.
FIGs. 3A-3H illustrate a method of manufacturing a distal body using the laser cut memory metal tube of FIGs. 1 and 2; in FIGs. 3A-3H, the basket portion of the distal body is not shown for simplicity of illustration.
FIGs. 4A-4D illustrate the welding steps of the method of manufacturing shown in FIG. 3; in FIGs. 4A-4D, the basket portion of the distal body is not shown for simplicity of illustration.
FIGs. 5 and 6 illustrate different locations that connector strips may be welded to the proximal memory metal strips.
FIG. 7 illustrates a side, elevation view of a catheter and the distal body of FIG. 6.
FIG. 8 illustrates a side, elevation view of a deployable system being used to capture a blood clot; in FIG. 8, the basket portion of the distal body is not shown for simplicity of illustration.
FIG. 9 illustrates a side, elevation view of a claw being closed by a claw actuator tube; in FIG. 9, the basket portion of the distal body is not shown for simplicity of illustration.
FIG. 10 illustrates a side, elevation view of a deployable system being used to capture a blood clot; in FIG. 10, the basket portion of the distal body is not shown for simplicity of illustration.
FIG. 11 illustrates a first, perspective view of a distal body; the distal body is in what is referred to herein as "Orientation 1".
FIG. 12A illustrates a second, perspective view of the distal body of FIG. 11; the distal body is in what is referred to herein as "Orientation 2".
FIG. 12B illustrates a proximal, elevation view of the proximal strips of the distal body of FIG. 11.
FIG. 13 illustrates a close-up, perspective view of two unattached distal-pointing crowns of the distal body of FIG. 11.
FIG. 14A illustrates a native memory metal tube used to manufacture the distal body of FIG. 11; the native tube has been rolled out flat and the lines in the tube indicate where the tube has been cut by a laser.
FIG. 14B illustrates a first, perspective view of the distal body manufactured from the native tube of FIG. 14A; the distal body is in Orientation 1.
FIG. 14C illustrates a second, perspective view of the distal body manufactured from the native tube of FIG. 14A; the distal body is in Orientation 2.
FIGs. 15A-G illustrate stepwise use of the distal body of FIG. 11 in retrieving a soft clot; the distal body is in Orientation 1.
FIGs. 16A-H illustrate stepwise use of the distal body of FIG. 11 in retrieving a hard clot; the distal body is in Orientation 1.
FIGs. 17A-G illustrate stepwise use of the distal body of FIG. 11 in retrieving a soft clot; the distal body is in Orientation 2.
FIGs. 18A-G illustrate stepwise use of the distal body of FIG. 11 in retrieving a hard clot; the distal body is in Orientation 2.
FIGs. 19A-N illustrate stepwise use of the distal body of FIG. 11 in retrieving a deformable, cohesive adherent clot; the distal body is in Orientation 2.
FIG. 20A illustrates a view of a native memory metal tube used to manufacture a distal body; the native tube has been rolled out flat, the lines in the tube indicate where the tube has been cut by a laser, and the distal body of FIGs. 20A-20C is slightly shorter than the distal body of FIGs. 11-19 and is meant for use in tortuous blood vessels.
FIG. 20B illustrates a first, perspective view of the distal body manufactured from the native tube of FIG. 20A; the distal body is in Orientation 1.
FIG. 20C illustrates a second, perspective view of the distal body manufactured from the native tube of FIG. 20A; the distal body is in Orientation 2.
FIG. 21 shows a perspective view of a clot retrieval system that includes the distal body of FIGs. 20B-C being delivered in a blood vessel using a delivery catheter.
FIG. 22 shows a perspective view of the distal body of FIG. 21, after deployment of the distal body and retraction of the delivery catheter, in a blood vessel.
FIG. 23 shows a perspective view of the distal body of FIG. 21; as compared to FIG. 22, the distal body has been moved proximally and tension has been exerted on the pull wire.
FIG. 24 shows a perspective view of a suction catheter that is being delivered over the pull wire of the system of FIG. 21.
FIG. 25 shows a perspective view of the distal end of the suction catheter of FIG. 24 being pushed into a clot; a syringe is sucking the clot to the suction catheter because the user has pulled back on the lever of the syringe.
FIG. 26 shows a perspective view of the distal end of the suction catheter of FIG. 24 being pushed into a clot; in FIG. 26, the user has locked the syringe lever at the desired volume.
FIG. 27 shows a perspective view of the system of FIG. 24; in FIG. 27, the suction catheter has partially sucked the distal body and clot into the suction catheter.
FIG. 28 shows a perspective view of the system of FIG. 24; in FIG. 28, the suction catheter has completely sucked the distal body and clot into the suction catheter.
FIG. 29 shows a perspective view of the system of FIG. 24; the system, and captured clot, is being removed proximally from the vessel.
FIG. 30 illustrates a right side perspective view of a mandrel used to prepare unattached distal-pointing crowns that curve radially toward the basket interior.
FIG. 31 illustrates a right side elevation view of the mandrel of FIG. 30.
FIG. 32 illustrates a distal body; in the distal body of FIG. 32, the proximal strips converge and are soldered or welded at the proximal hub/junction and the basket strips located at the distal end of the basket converge and are soldered or welded at the distal hub/junction.
FIG.33A illustrates a side, elevation view of a memory metal tube.
FIG. 33B illustrates a side, elevation view of the memory metal tube of FIG. 33A being cut by a laser.
FIG. 34 illustrates a side, elevation view of the memory metal tube of FIG. 33B after being cut by a laser; in FIG. 34, the tube is shown as though it were flat for purposes of illustrating the cut pattern only.
FIG. 35 illustrates a side, elevation view of the circled area labelled 35 in FIG. 34 (namely, the distal portion of the cut memory metal tube of FIG. 34 - the distal portion includes the distal ends of the distal memory metal strips, the distal end tabs and the distal longitudinal tabs); in FIG. 35, the tube is shown as though it were flat for purposes of illustrating the cut pattern only.
FIG. 36 illustrates a side, elevation view of the circled area labelled 36 in FIG. 34 (namely, the proximal portion of the cut memory metal tube of FIG. 34 - the proximal portion includes the proximal ends of the proximal memory metal strips, the proximal end tabs and the proximal distal longitudinal tabs); in FIG. 36, the tube is shown as though it were flat for purposes of illustrating the cut pattern only.
FIG. 37 illustrates a side, elevation view of the circled area labelled 37 in FIG. 36 (namely, a close-up of the proximal portion of the cut memory metal tube of FIG. 36); in FIG. 37, the tube is shown as though it were flat for purposes of illustrating the cut pattern only.
FIG. 38 illustrates a side, elevation view of the close-up of the proximal portion of the cut memory metal tube of FIG. 37 after electropolishing; in FIG. 38, the tube is shown as though it were flat for purposes of illustrating the cut pattern only.
FIG. 39 illustrates a side, elevation view of the close-up of the proximal portion of the cut memory metal tube of FIG. 37 after electropolishing and tearing along the peforations; in FIG. 39, the tube is shown as though it were flat for purposes of illustrating the cut pattern only.
FIG. 40 illustrates a side, elevation view of the close-up of the proximal portion of the cut memory metal tube of FIG. 36.
FIG. 41 illustrates a side, elevation view of the proximal portion of the cut memory metal tube of FIG. 40 after electropolishing and after tearing along the perforations to remove the proximal end tab and the proximal longitudinal tabs from the proximal segments of the proximal memory metal strips.
FIG. 42 illustrates another side elevation view of the proximal portion of the cut memory metal tube of FIG. 40 after electropolishing and after tearing along the perforations to remove the proximal end tab and the proximal longitudinal tabs from the proximal segments of the proximal memory metal strips; as compared to FIG. 41, the proximal end of the cut memory metal tube has been rotated 90 degrees in FIG. 42.
FIG. 43A illustrates a side elevation view of a pull wire.
FIG. 43B illustrates a side elevation view of a coil system that includes a core and a coil wrapped around the core.
FIG. 43C illustrates a side elevation of the pull wire of FIG. 43A being soldered to the coil system of FIG. 43B.
FIG. 43D illustrates a close-up, side elevation view of the area denoted by the dashed rectangle in FIG. 43C (namely, the distal end of the pull wire and the coil system of FIG. 43C).
FIG. 43E, FIG. 43F and FIG. 43G illustrate stepwise, side elevation views of the proximal ends of the proximal memory metal strips of FIG. 42 being soldered to the coil system of FIG. 43D; as shown in FIG. 43F and FIG. 43G, the proximal memory strips are placed between the core and the coil.
FIG. 44 illustrates a side, elevation view of the coil system of FIG. 43G being placed through a distal end of a catheter.
FIG. 45 illustrates a side, elevation view of a tube (referred to herein as a third tube) being used to re-join distal ends of distal memory metal strips; the distal ends of the distal memory metal strips were initially joined by a distal end tab and distal longitudinal tabs.
FIG. 46 illustrates a side elevation view of the proximal portion of the cut memory metal tube and is similar to FIG. 36; the line is merely drawn in to show how each proximal memory metal strips tapers adjacent to the proximal end of the respective proximal memory metal strips (and the line is not present in the device).
FIG. 47 illustrate side views of a middle portion cut from the memory metal tube of FIG. 33B and expanded using the mandrel of FIG. 31; in FIG. 47, the middle portion is in the form of a basket with offset enlarged areas/drop zones adjacent to crowns pointing generally in the distal direction; FIG. 47 also includes proximal memory metal strips having a free proximal end and a distal end connected to a proximal cell of the basket and distal memory metal strips having a free distal end and a proximal end connected to a distal cell of the basket.
FIG. 48 illustrates a medical device that includes the catheter of FIG. 44, the pull wire of FIG. 44, the coil system, which is attached to the proximal memory metal strips as shown in FIG. 44, the basket of FIG. 47 and the re-joined distal ends of the distal memory metal strips of FIG. 45.
FIG. 49 illustrates a side, elevation view of proximal memory metal strips and longitudinal perforations at the proximal end of a cut memory metal tube; in FIG. 49, only longitudinal perforations are present, and as with FIG. 46, the line is merely drawn in to show how each proximal memory metal strips tapers adjacent to the proximal end of the respective proximal memory metal strips (and the line is not present in the device).
FIG. 50 illustrates a side elevation view of a deployable dual basket system.
FIG. 51 illustrates another side elevation view of the deployable dual basket system of FIG. 50; as compared to FIG. 50, the deployable dual basket system has been rotated 90 degrees.
FIG. 52 illustrates a side, elevation view of a memory metal tube being cut by a laser to form a deployable dual basket system; in FIG. 52, the tube is shown as though it were flat for purposes of illustrating the cut pattern only.
FIG. 53A illustrates a side elevation view of the proximal end of the memory metal tube of FIG. 49; in FIG. 53A, the tube is shown as though it were flat for purposes of illustrating the cut pattern only.
FIG. 53B illustrates a side elevation view of the distal end of the memory metal tube of FIG. 52; in FIG. 53B, the tube is shown as though it were flat for purposes of illustrating the cut pattern only.
FIG. 53C illustrates a side elevation view of the proximal tether memory metal strips prepared from the tube of FIGs. 53A after removing the proximal longitudinal tabs and the proximal perimeter tabs.
FIG. 53D illustrates a side elevation view of the distal basket memory metal strips prepared from the tube of FIGs. 53A after removing the distal longitudinal tabs and the distal perimeter tabs.
FIG. 54 illustrates use of a third tube to re-join the distal basket memory metal strips of FIG. 53D.
FIG. 55 illustrates use of a coil to re-join the proximal tether memory metal strips of FIG. 53C.
FIGs. 56A-56H illustrate deployment and use of a catheter-delivered endovascular device that includes the deployable dual basket system of FIGs. 50 and 51 to treat a human having a subarrachnoid hemorrhage induced vasospasm in a constricted blood vessel having a proximal region having a constricted height and a constricted width and a distal region having a constricted height and a constricted width.
FIG. 57 illustrates a side elevation view of a deployable basket system of an embodiment of the present invention that includes a basket with a proximal portion comprising proximal cells and a distal portion comprising braided mesh openings; in FIG. 57 the basket is in the relaxed state.
FIG. 58 illustrates another side elevation view of a deployable basket system of another embodiment of the present invention in the relaxed state; as compared to FIG. 57, the distal portion is located further distally in FIG. 58.
FIG. 59 illustrates a side elevation view of the deployable basket system of FIG. 58; in FIG. 59 the basket is in the partially collapsed state.
FIG. 60 illustrates use of the deployable basket system of FIG. 57 in a blood vessel.
FIG. 61 illustrates a side elevation view of a deployable basket system of another embodiment of the present invention; in FIG. 61, the basket is in the relaxed state and a segment of the distal portion is located in the proximal portion interior.
FIG. 62 illustrates a side elevation view of the deployable basket system of FIG. 61; in FIG. 62, the basket is in the partially collapsed state.
FIG. 63 illustrates a side elevation view of a deployable basket system of another embodiment of the present invention; in FIG. 63, the basket is in the relaxed state.
FIG. 64 illustrates a side elevation view of the deployable basket system of FIG. 63; in FIG. 64, the basket is in the partially collapsed state.
FIG. 65 illustrates a side elevation view of a deployable basket system of another embodiment of the present invention; in FIG. 65, the deployable basket system is at an initial step of deployment from the catheter.
FIG. 66 illustrates a side elevation view of the deployable basket system of FIG. 65 at a second step of deployment from the catheter.
FIG. 67 illustrates a side elevation view of the deployable basket system of FIG. 65 at a third step of deployment from the catheter.
FIG. 68 illustrates a side elevation view of the deployable basket system of FIG. 65 almost fully deployed from the catheter.
FIG. 69 illustrates a side elevation view of a deployable basket system of another embodiment of the present invention.
FIG. 70 illustrates a side elevation view of a deployable basket system of another embodiment of the present invention.
FIG. 71A illustrates a side elevation view of a deployable basket system of another embodiment of the present invention with a positive charge propagated along the pull wire to the inner body.
FIG. 71B illustrates a side elevation view of the deployable basket system of FIG. 71A with a negative charge propagated along the pull wire to the inner body.
FIG. 72 illustrates a close-up cross-sectional view of the area denoted by the rectangular box labelled 105 in FIG. 71B.
FIG. 73 illustrates a side elevation view of a deployable basket system of another embodiment of the present invention in which an active agent is coating the woven linear strands of the distal body inner body.
FIG. 73A illustrates a side elevation view of a deployable basket system of another embodiment of the present invention in which an active agent is coating the woven linear strands of the distal body inner body.
FIG. 74 illustrates a side elevation view of the deployable basket system of FIG. 73 in use in a blood vessel delivering the active agent to dissolve distal emboli.
FIG. 75 illustrates a side elevation view of a deployable basket system of another embodiment of the present invention in which an active agent is located in the interior of the distal body inner body.
FIG. 76 illustrates a side elevation view of the deployable basket system of FIG. 75 with the distal body in the collapsed state.
FIG. 77 illustrates a side elevation view of the deployable basket system of another embodiment of the present invention with a negative and positive charge being used to deliver the active agent into the blood vessel.
FIG. 78 illustrates a side elevation view of a deployable basket system of another embodiment of the present invention with an active agent delivery catheter.
FIG. 79 illustrates a cross-sectional view of proximal strips attached to an active agent delivery catheter.
FIG. 80 illustrates a cross-sectional view of proximal strips attached to an active agent delivery catheter.
FIG. 81A illustrates a side elevation view of a deployable basket system of another embodiment of the present invention.
FIG. 81B illustrates a closeup view of the area denoted by the rectangular box labelled 81B in FIG. 81A

### DETAILED DESCRIPTION

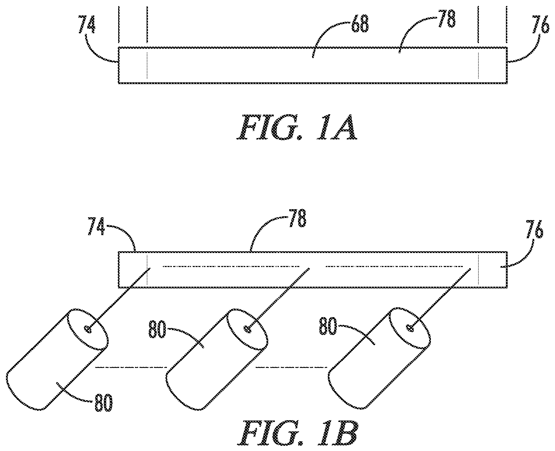

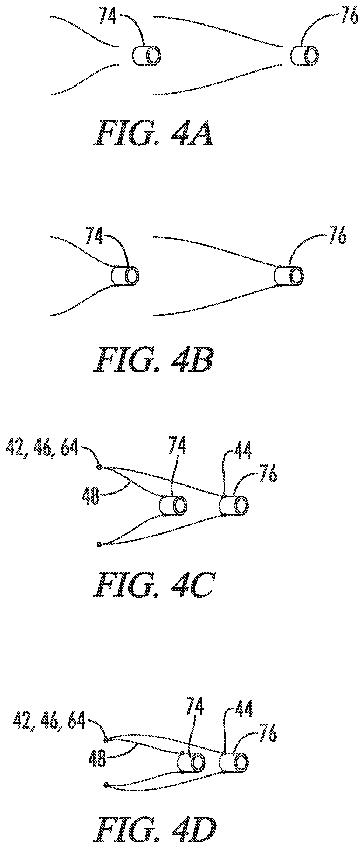

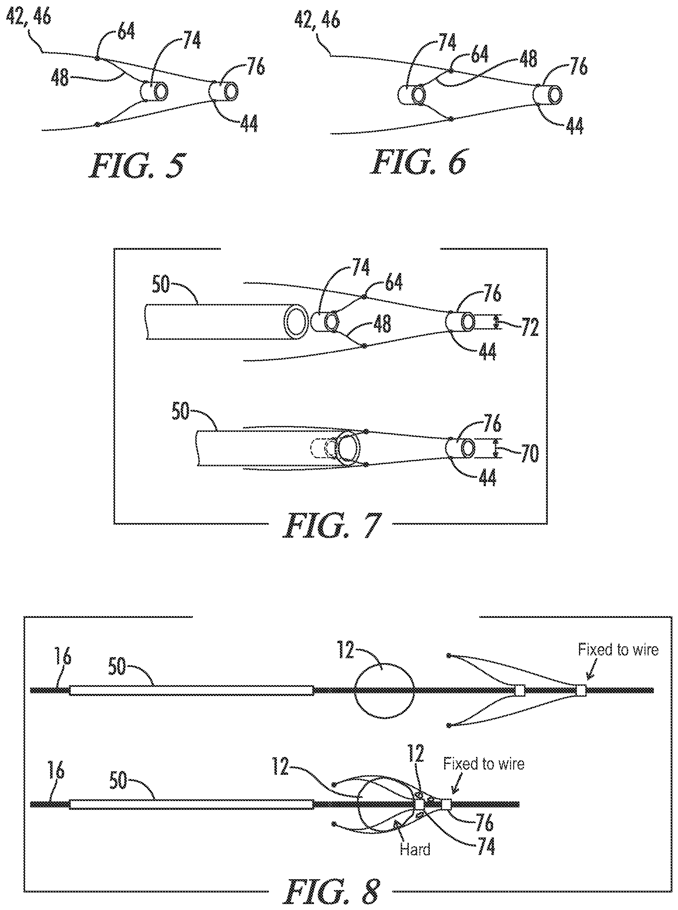

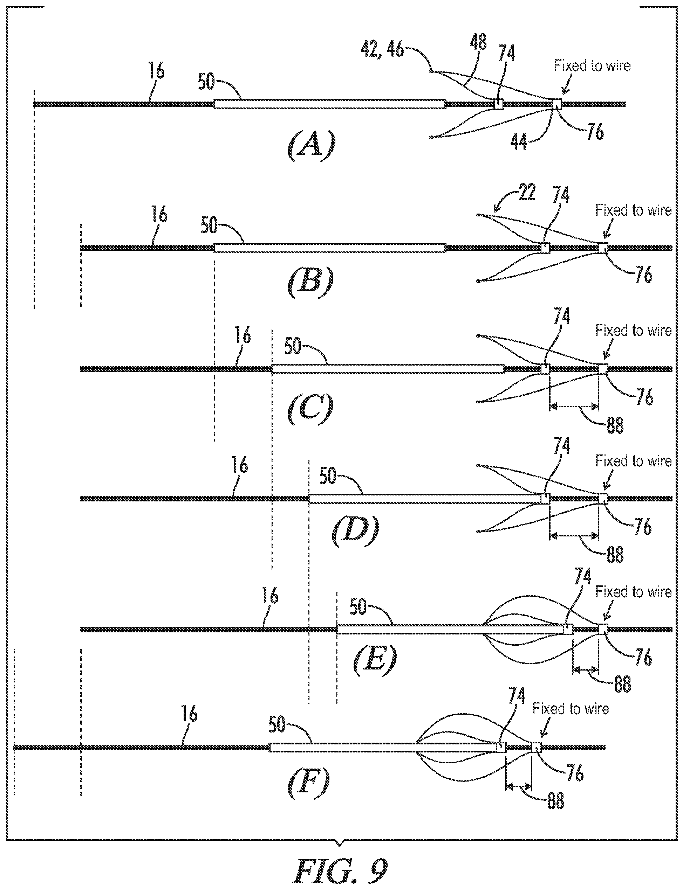

With reference to FIGs. 1-10, there is shown an example of a deployable system shown for reference purposes only, generally designated by the numeral 10, for removing an obstruction such as a blood clot 12 or other object from a blood vessel 14 or other interior lumen of an animal. In addition to a blood clot 12, the obstruction may be, for example, extruded coils during aneurysm treatment, intravascular embolic material such as onyx or other obstructions requiring mechanical intravascular removal from small distal vessels. In the drawings, not all reference numbers are included in each drawing for the sake of clarity.

Referring further to FIGs. 1-10, the deployable system 10 includes a pull wire 16 that has a proximal end (not shown) and a distal end 20. Optionally, the diameter of the pull wire is between about 0.008 inches and about 0.051 inches. Preferably, the pull wire 16 is comprised of a biocompatible metallic material.

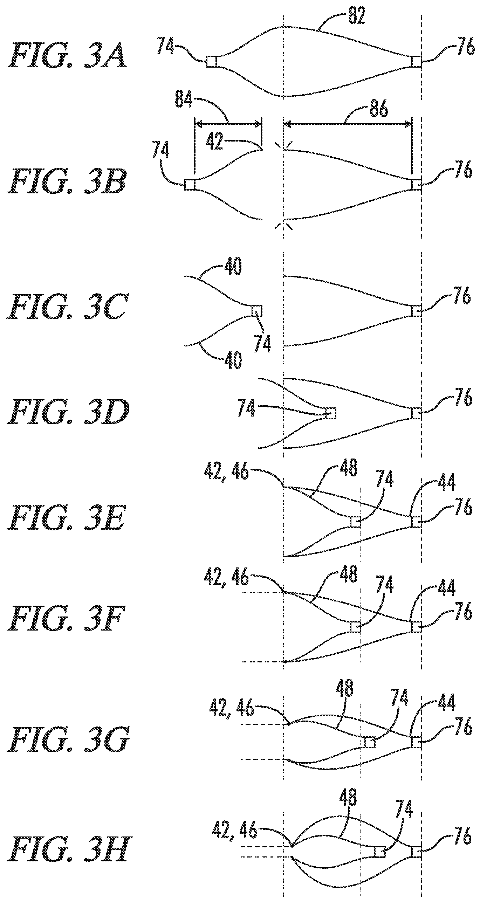

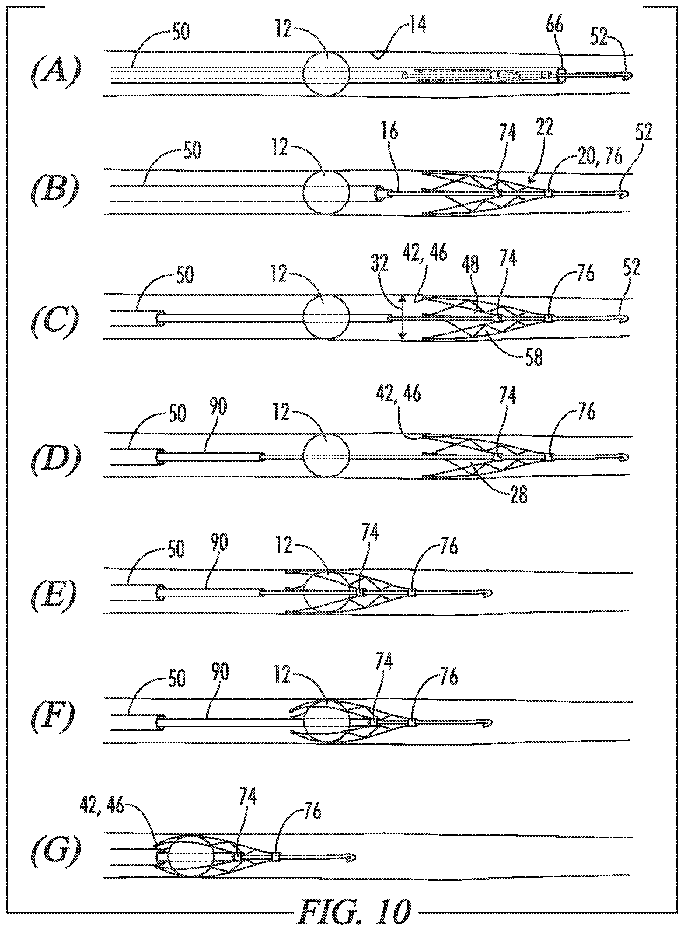

The system 10 further includes a distal body 22, which is attached to the pull wire 16. The distal body 22 has a proximal end 24, a distal end 26, an interior 28, and an exterior 30. The distal body 22 has a collapsed state, wherein the distal body 22 has a first height and width and is configured to fit into a catheter 50 (see FIG. 10A), and a relaxed state wherein the distal body 22 has a different height 32 and width and is configured to expand to about the height and width of a human blood vessel **14** when the distal body **22** is deployed from the catheter **50** (see FIGS. 10B-G). The distal body **22** further includes a proximal hub/junction **74** and a distal hub/junction **76** that is located distal relative to the proximal hub/junction **74.** In some examples, the distal body **22** includes a plurality of strips **40** comprised of a memory metal (e.g., a memory metal alloy such as nitinol) that form the proximal end **24** of the distal body **22.** Optionally, the proximal memory metal strips **40** each have a distal end **44** and a proximal end **42** that forms an openable and closeable claw **46.** Optionally, the proximal memory metal strips **40** are attached to the proximal hub/junction **74** through connector memory metal strips **48.** In such examples, the proximal hub/junction **74** may be slideable along at least a segment of the pull wire **16,** in contrast to the distal hub/junction **76,** which is optionally fixed to the pull wire **16** and not slideable along the pull wire **16.** Moving the proximal hub/junction **74** distally and closer to the distal hub/junction **76** (i.e., shortening the distance **88** between the proximal hub/junction **74** and distal hub/junction **76** by moving the proximal hub/junction **74** distally while keeping the distal hub/junction **76** stationary) exerts tension on the connector memory metal strips **48** and, in turn, the proximal memory metal strips **40.** This tension, in turn, causes the proximal ends **42** of the proximal memory metal strips **40** to move radially toward each other and the pull wire **16.** As the proximal ends **42** of the proximal memory metal strips **40** move radially toward each other and the pull wire **16,** the claw **46** (formed by the proximal memory metal strips **40**) is brought from the open position to at least a partially closed position, which in turn, separates the obstruction **12** from the wall of the human lumen **14** and captures the obstruction **12.** See FIG. 3H, FIG. 8, FIG. 9F, and FIG. 10F and 10G. Conversely, preferably, movement of the proximal hub/junction **74** proximally and away from the distal hub/junction **76** (i.e., increasing the distance **88** between the hubs/junctions **74** and **76**) releases the tension in the proximal memory metal strips **40,** which in turn, causes the proximal ends **42** of the proximal memory metal strips **40** to move away from each other and the pull wire **16,** opening the claw **46.** The claw **46** and proximal hub/junction **74** form several functions. First, as described, closing of the claw **46** captures the obstruction **12.** Second, closing the claw **46** retracts the claw **46** from the wall of the lumen **14** so that the claw **46** does not scrape against (and damage) the lumen wall while capturing the obstruction **12.** Third, closing the claw **46** reduces the height and width of the distal body **22,** which allows the distal body **22** to be re-sheathed in the catheter **50,** which may be desired, for example, if the operator seeks to re-deploy the distal body **22** in another location in the body (which may be the case if the operator originally deploys the distal body **22** in the wrong location in the lumen **14**). For purposes of the present invention, "closing the claw" embraces both partially closing the claw **46** (where the proximal ends **42** of the proximal memory metal strips **40** do not contact the pull wire **16**) and fully closing the claw **46** (where the proximal ends **42** contact the pull wire **16**).

The claw **46** may be comprised of any number of proximal memory metal strips **40.** Preferably, however, between 2 and 4 proximal memory metal strips **40** comprise the claw **46** (it being understood that the connector strips **48,** if present, merely serve to tether the claw **46** to the proximal hub/junction **74**). Preferably, the proximal memory metal strips **40** have a length of between about 10 and about 60 millimeters. The proximal memory metal strips **40** can be thought of as arms of the claw **46.**

In some examples, the connector strips **48** are integral with the proximal hub/junction **74** (i.e., formed from the same piece of memory metal). In other examples, the proximal hub/junction **74** may be welded or soldered to the connector strips **48.** Optionally, in the relaxed state, the proximal memory metal strips **42** are distributed substantially evenly about a perimeter of the distal body **22.**

Optionally, the distal body **22** includes a lead wire **52** extending distally from the distal body **22.** Optionally, the lead wire **52** extends distally from the distal hub/junction **76.** If present, the lead wire **52** may be used to facilitate movement of the system **10** in the lumen **14.**

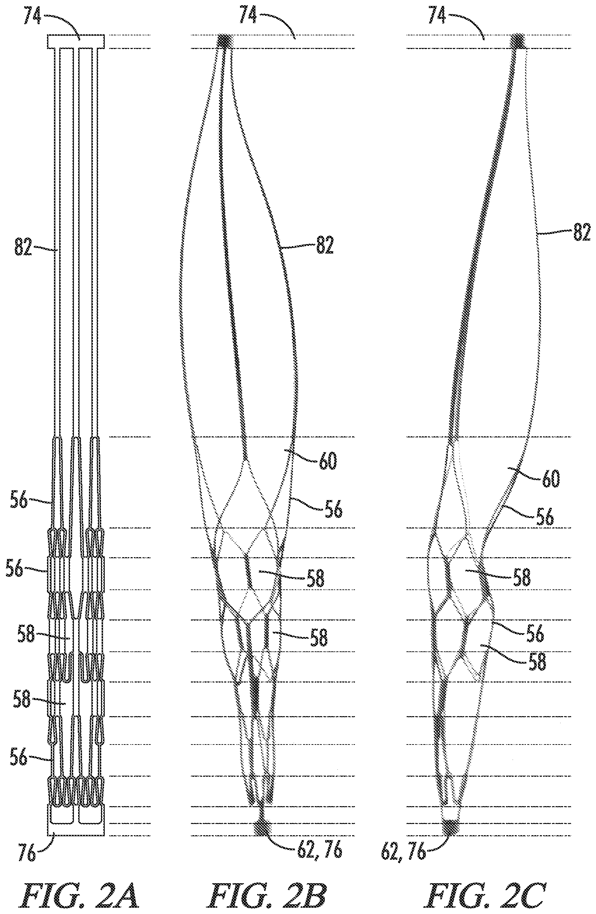

Optionally, the distal body **22** includes a basket **54** distal to the proximal memory metal strips **40,** the basket **54** comprised of a plurality of memory metal strips **56** distal relative to the proximal memory metal strips **40.** The distal memory metal strips **56** may, for example, form a basket **54** with a plurality of mesh openings **58.** Optionally, the size of the mesh openings **58** in the basket **54** when the distal body **22** is in its relaxed state is less (preferably significantly less) than the diameter of an average-sized ischemic blood clot **12** so that the blood clot **12** does not escape from the distal basket **54** after being captured by the distal body **22.** Optionally, the basket **54** has an open proximal end **60** and a substantially closed distal end **62,** which is formed by distal tube **76.** Optionally, the distal and proximal hubs/junctions **74** and **76** and the distal basket **54** are comprised of a nitinol having the same material composition. Optionally, the size of the mesh openings **58** decreases from the proximal end **60** of the basket **54** to the distal end **62.** The distal basket **54** is best seen in FIG. 2 and can be comprised of a different number of cell patterns. The distal basket **54** is not shown in FIGs. 3-10 for ease of illustrating the other components in the system **10.**

Optionally, the proximal hub/junction **74** and the distal hub/junction **76** are cylindrical tubes comprising substantially circular apertures that span the length of the hubs/junctions **74** and **76** and the hubs/junctions **74** and **76** have approximately the same inner diameter **72** and the same outer diameter **70.** Preferably, the inner diameter **72** is at least slightly larger than the diameter of the pull wire **16** so that the pull wire **16** can slide through the proximal hub/junction **74.** In some examples, the outer diameters **70** of the proximal and distal hubs/junctions **74** and **76** may be from about 0.011 inches to about 0.054 inches and the inner diameters **72** of the proximal and distal hubs/junctions **74** and **76** may be from about 0.008 inches to about 0.051 inches.

Optionally, the distal body **22** further comprises an x-ray marker **64** that is more visible under x-ray as compared to the proximal memory metal strips **40** when the distal body **22** is located in a cranial blood vessel inside the body of a human and the x-ray is taken from outside the human's body. If the connector strips **48** are welded or soldered to the proximal memory metal strips **40,** the x-ray markers **64** may be, for example, located at the welding or soldering site. In some cases, the increased thickness at the welding or soldering site may in of itself comprise the x-ray marker **64.** Preferably, the x-ray marker **64** is comprised of a radiopaque material. Some examples of radiopaque materials can include, but are not limited to, gold, platinum, palladium, tantalum, tungsten alloy, polymer material loaded with radiopaque filler, and the like. Preferably, the proximal memory metal strips **40** are comprised of nitinol and the x-ray marker **64** is comprised of a material having a density greater than the nitinol.

A catheter **50** with an open proximal end (not shown) and an open distal end **66** initially envelopes the system **10.** As used herein, the term "catheter" generally refers to any suitable tube through which the system **10** can be deployed. Preferably, the catheter **50** is sterile and comprised of a biocompatible material (i.e., a material that does not irritate the human body during the course of a 45 minute operation that involves using the system **10** to remove a clot **12** from an intracranial blood vessel **14**). The catheter **50** can be any suitable shape, including but not limited to generally cylindrical. Preferably, the catheter **50** is a microcatheter. For purposes of the present invention, when it is said that the catheter **50** envelopes the system **10,** it will be understood that the catheter **50** envelopes at least one component of the system **10** (preferably, the distal body **22,** the lead wire **52,** and the pull wire **16**). In some examples, the catheter **50** is about 2.5 French in diameter. Optionally, the catheter **50** is delivered to the region of the lumen **14** that has the obstruction **12** as follows: a guide wire is delivered to the obstruction region past the obstruction **12;** the catheter **50** is delivered over the guide wire; the guide wire is removed; and the system **10** is delivered with its pull wire **16** and lead wire **52** through the catheter **50.** Optionally, the pull wire **16** is used to push the system **10** through the catheter **50** as well as to retrieve the distal body **22** after capturing the obstruction **14** as described below. The system **10** may utilize a plurality of catheters **50,** such as, for example, a wider catheter that travels to the brain and a very flexible, smaller diameter microcatheter that is delivered from the first catheter and travels through the small arteries of the brain. Preferably, the catheter **50** is comprised of a biocompatible, polymeric material (i.e., one or more polymeric materials such as silicone, PVC, latex rubber or braided nylon).

Optionally, in the relaxed, opened-claw state, the distal body **22** or optionally just the distal basket **54** has a tapered shape (e.g., substantially conical or bullet in shape) so that the distal body **22** or just the distal basket **54** tapers from the distal body **22** or the distal basket's **54** proximal end to the distal end.

The proximal end of the system **10** is shown at the left end of FIGs. 1 and 3-10 and the distal end of the system **10** is shown at the right end of FIGs. 1 and 3-10 because a principal use of the system **10** is to remove a blood clot **12** from a human intracranial artery **14,** in which case the system **10** generally will enter the artery **14** at its proximal end by the surgeon entering the patient's body near the groin and pushing the catheter **50** towards the brain. The diameter of human arteries **14** generally decrease from their proximal end to their distal end. However, when used in other types of lumens, the distal body **22** may be located proximally relative to the catheter **50** as the term proximally and distally are used in that lumen.

The surgeon may deploy the distal body **22** by, for example, moving the catheter **50** proximally so as to unsheathe the distal body **22** or by pushing the distal body **22** out of the catheter **50.**

Use of the system **10** will now be described to remove a blood clot **12** from an intracranial artery **14** of a human ischemic stroke patient, however, it will be appreciated that the system **10** may be used to remove other objects from other interior lumens.

A catheter **50,** which contains the collapsed distal body **22** is positioned in the lumen **14** distal to the clot **12.** See FIG. 10A.

The distal body **22** is deployed from the catheter **50** and the height and width of the distal body **22** expand to about the height and width of the blood vessel **14.** See FIG. 10B.

The catheter **50** is pulled proximally and a claw-actuator tube **90** is deployed into the blood vessel **14.** See FIG. 10C.

The distal body **22** is moved proximally so that the clot **12** is located in the interior **28** of the distal body **22.** See FIGs. 10D and 10E.

The claw-actuator tube **90** is moved distally, which pushes the proximal hub/junction **74** distally so that the distance **88** between the proximal hub/junction **74** and the distal hub/junction **76** (which is fixed to the pull wire **16** and kept stationary) decreases. Distal movement of the proximal hub/junction **74** exerts tension on the connector and proximal memory metal strips **40** and **48,** which in turn, closes the claw **46.** See FIG. 10F. (The claw actuator tube **90** should float on the pull wire **16** - i.e., have an aperture extending the tube's length that has a diameter larger than the diameter of the pull wire **16** - and the aperture of the claw actuator tube **90** should be smaller than the diameter of the proximal hub/junction **74** so that the claw actuator tube **90** pushes the proximal hub/junction **74**).

The system **10** is withdrawn proximally and removed from the body. See FIG. 10G.

To test the efficacy of the system **10,** a distal body **22** with a distal basket **54,** proximal and distal hubs/junctions **74** and **76,** and a claw **46** comprised of three proximal memory metal strips **42** was tested in a flow model that included a tube and a moist cotton ball located in the tube. The cotton ball was used to simulate a blood clot. The system **10** was deployed distal to the cotton ball. The claw **46** was closed by moving the proximal hub/junction **74** distally to capture the cotton ball. The system **10** and cotton ball were withdrawn proximally in the tube.

In some examples, the distal body **22** is prepared by a process that includes one or more of the following steps, as illustrated in FIGs. 1-4
a) providing a single tube **68** comprised of a memory metal such as nitinol, the single tube **68** having an exterior, a substantially hollow interior, a wall separating the exterior from the substantially hollow interior, an open proximal end **74,** an open distal end **76,** a middle portion **78** between the open proximal end **74** and the open distal end **76** (see FIG. 1A);
b) cutting the wall of the middle portion **78** with a laser **80** (see FIG. 1B);
c) removing the pieces of the middle portion **78** cut by the laser **80** to form a proximal tube **74,** a distal tube **76** and a middle portion **78** comprising a plurality of memory metal strips **82** attached to the proximal tube **74;**
d) altering the shape of the middle portion **78** using a mandrel and allowing the middle portion **78** to expand relative to the distal tube **76** and proximal tube **74** to form the distal basket **54;**
e) quenching the middle portion **78** at room temperature;
f) removing the mandrel from the middle portion **78** (see FIGs. 2 and 3A);
g) mechanically or chemically electropolishing the middle portion **78** to remove oxides;
h) cutting the memory metal strips **82** to form a first segment **84** comprising the proximal tube **74** and a proximal segment of the memory metal strips **82** and a second segment **86** comprising the distal tube **76** and a distal segment of the memory metal strips **82** (see FIG. 3B); and
i) joining the proximal segments to the distal segments such that the distal segments form the proximal end **24** of the distal body **22,** such that the proximal tube **74** is located inside the interior **28** of the distal body **22,** and such the proximal tube **74** is located distal relative to the distal body proximal end **24** (see FIGs. 3C-3E).

In some examples, the method further includes placing the pull wire **16** through the proximal tube **74** so that the proximal tube **74** is slideable along at least a segment of the pull wire **16.**

In some examples, the method further includes attaching the pull wire **16** to the distal tube **76** so that the distal tube **76** is not slideable along the pull wire **16** but instead the distal tube **76** moves with the pull wire **16.**

In some examples, after step i, the proximal end **24** of the distal body **22** forms a claw **46** comprised of between 2 to 4 proximal memory metal strips **40,** the claw proximal memory metal strips **40** configured to move towards each other and the pull wire **16** by moving the proximal tube **74** distally and toward the distal tube **76** (i.e., decreasing the distance **88** between the tubes **74** and **76**) and the claw memory metal strips **40** configured to move away from each other and away from the pull wire (i.e., increasing the distance **88** between the tubes **74** and **76**) by moving the proximal tube **76** proximally and away from the distal tube **76** (as described previously).

In some examples, the middle portion **78** is expanded by heating the mandrel and the middle portion **78** by, for example, placing the mandrel and the middle portion **78** in a fluidized sand bath at about 500°C for about 3 to about 7 minutes. As the middle portion **78** is heated, the heating causes the crystalline structure of the memory metal tube **68** to realign. Preferably, the mandrel is tapered (e.g., substantially conical or bullet in shape) so that the distal basket **54** formed from the middle portion **78** tapers from the proximal end **60** to the distal end **62.** Preferably, the proximal and distal ends of the tube **74** and **76** are not shape set by the mandrel and are not cut by the laser **80** so that the proximal and distal ends **74** and **76** do not change in shape and only slightly expand in size under heating and return to the size of the native tube **68** after the heat is removed. Preferably, the laser cuts are programmed via a computer. To ensure that the laser cuts only one surface of the tube wall at the time (and not the surface directly opposite the desired cutting surface), the laser **80** is preferably focused between the inner and outer diameter of the desired cutting surface and a coolant is passed through the memory metal tube **68** so that the laser **80** cools before reaching the surface directly opposite the desired cutting surface.

The portions of the wall not cut by the laser **80** create the distal basket **53,** proximal and distal tubes **74** and **76,** and memory metal strips **40, 48** and **56,** as described.

Preferably, the memory metal selected for the native tube **68** has a heat of transformation below average human body temperature (37°C) so that the distal body **22** has sufficient spring and flexibility after deployment from the catheter **50** in the human blood vessel **14.**

In some examples, the native tube **68** (and hence the distal and proximal tubes **74** and **76**) have an outer diameter of less than about 4 French, e.g., a diameter of about 1 to about 4 French. In some examples, the diameter of the pull wire **16** is between about 0.008 inches and about 0.051, as noted above, and in such examples, the diameter of the pull wire **16** may be approximately equal to the inner diameter **72** of the native nitinol tube **68.**

Without being bound by any particular theory, it is believed that manufacturing the distal body **22** from a single memory metal tube **68** provides ease of manufacturing and safety from mechanical failure and provides tensile strength necessary for the system **10** to remove hard thrombus **12** and other obstructions.

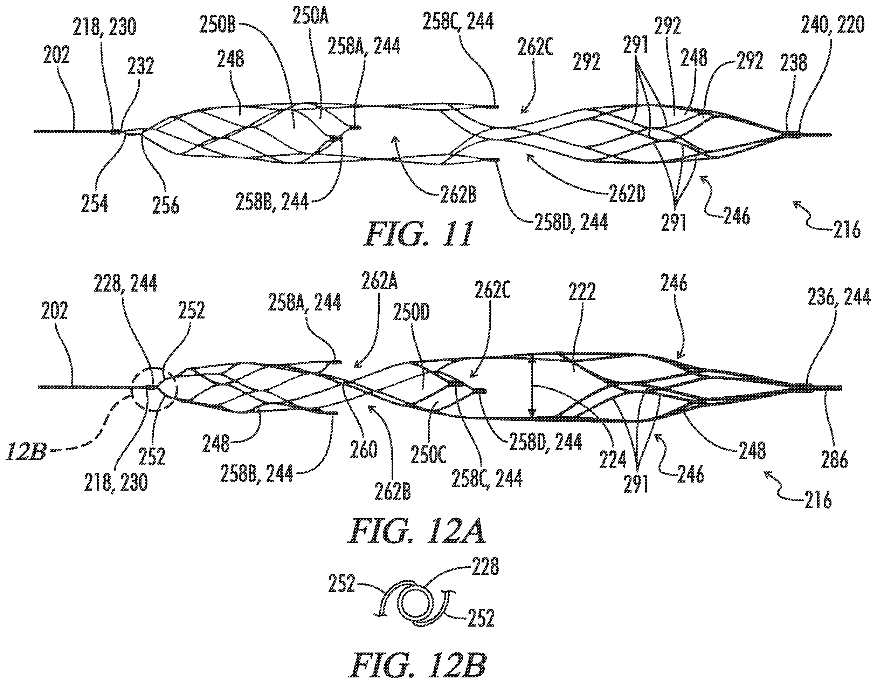

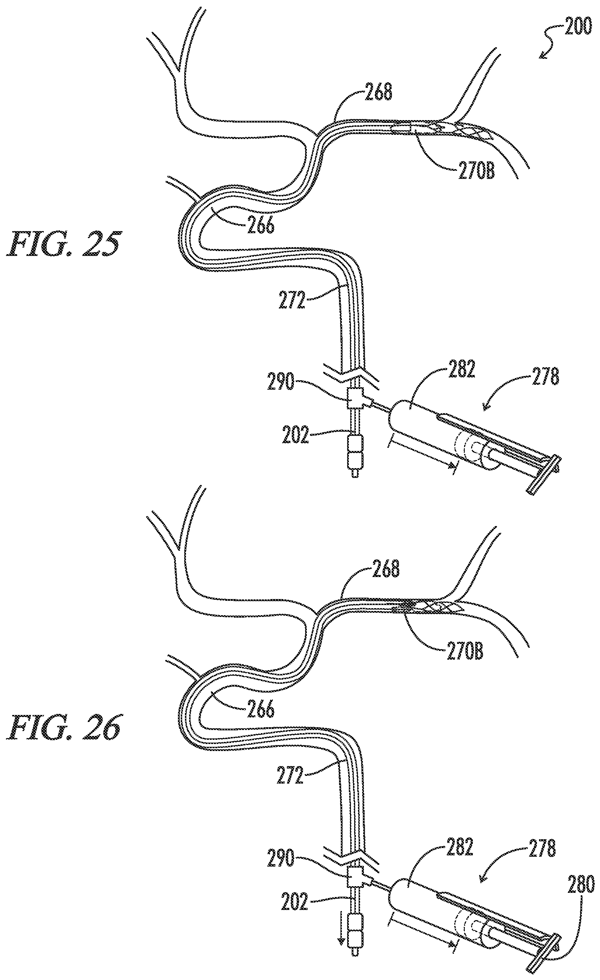

### Examples shown in Figures 11-29

Figures 11-29 illustrate an example**200** shown for reference purposes only that includes one or more of the following additional features, as described below: twisting proximal strips/tethers **252,** unattached/free distal-pointing crowns **258** that optionally curve inward and have x-ray markers **244,** and enlarged openings/drop zones **262** in the basket **246** immediately distal to the unattached, distal-pointing crowns **258** that allow the obstruction or other object **270** to enter the distal basket interior **222.**

More specifically, as shown in FIGs. 11-29, the system **200** may include a pull wire **202** having a proximal end **204** and a distal end **206,** as described above, a distal body **216** attached to the pull wire **202,** the distal body **216** comprising an interior **222,** a proximal end **218,** a distal end **220,** a distal body length **226** extending from the proximal end **218** to the distal end **220,** a distal body height **224,** a proximal hub/junction **228** (preferably in the form of a tube and which has a proximal end **230** and a distal end **232**) forming the proximal end **218** of the distal body **216,** a basket **246** comprised of a plurality of cells/openings **248** formed by a plurality of basket strips **291** that preferably are comprised of a memory metal, optionally a distal hub/junction **236** that forms the distal end **220** of the basket **246** (preferably in the form of a tube that has a proximal end **238** and a distal end **240**), and a plurality of proximal strips **252** (preferably the proximal strips **252** are comprised of a memory metal), each proximal strip **252** having a proximal end **254** attached to the proximal hub/junction/tube **228,** and a distal end **256** attached to a cell **248** (more specifically a proximal-pointing crown of a cell **248** located at the proximal end of the basket **246**), the basket comprising a basket interior **292,** the distal body **216** having a relaxed state wherein the distal body **216** has a first height and width, a collapsed state wherein the distal body **216** has a second height and width, the second height less than the first height, the second width less than the first width; and a delivery catheter **208** for delivering the distal body **216,** as described above, having an interior **210,** a proximal end **212** leading to the interior **210** and a distal end **214** leading to the interior **210,** the delivery catheter **208** comprised of a biocompatible (preferably polymeric) material and configured to envelope the distal body **216** when the distal body **216** is in the collapsed state. Optionally, the basket interior **292** is substantially hollow - i.e., unlike U.S. Patent Publication No. 2013/0345739, the basket interior **292** does not contain an inner elongate body. Optionally, instead of a distal hub/junction **236,** the basket **246** includes an open distal end. Optionally, at least two cells **250** of the basket **246** comprise a proximal crown **260** pointing generally in the proximal direction and a distal crown **258** pointing generally in the distal direction, and the distal crowns **258** of the at least two cells **250** are not attached to another cell **248** of the basket **246.** In other words, the distal crowns **258** of at least two cells **250** are free floating and are not attached to any strip except for the strips forming part of the at least two cells **250;** such distal crowns **258** are referred to below as unattached, distal-pointing crowns **258.** Preferably, the distal tips of the unattached, distal-pointing crowns **258** terminate at an x-ray marker **244.** (Cells labeled with the numerals **250, 250A, 250B, 250C,** and **250D** refer to the at least two cells that include a proximal crown **260** pointing generally in the proximal direction and an unattached, distal-pointing crown **258,** cells labeled with the numerals **262, 262A, 262B, 262C,** and **262D** refer to the enlarged cells/drop zones adjacent to (preferably immediately distal to) an unattached, distal-pointing crown **258,** and cells designated with numeral **248** refer to generally the cells of the basket **246**). (When it is said that the enlarged cells/drop zones **262** are preferably immediately distal to an unattached, distal-pointing crown **258,** it will be understood that at least a portion of an enlarged cell/drop zone **262** is immediately distal to an unattached, distal-pointing crown **258,** and that a portion of the enlarged cell/drop zone **262** may be proximal to an unattached, distal-pointing crown **258,** as shown in FIGs. 11-12 due to the shape of the enlarged cells/drop zones **262**). It will be understood that part number **250** refers generally to one or more of the at least two cells, whereas part numbers **250A, 250B, 250C,** and **250D** refer to a specific one of the at least two cells. Similarly, it will be understood that part number **262** refers generally to one or more of the enlarged cells/drop zones, whereas part numbers **262A, 262B, 262C,** and **262D** refer to a specific one of the enlarged cells/drop zones. Similarly, it will be understood that part number **258** refers generally to one or more of the unattached, distal-pointing crowns, whereas part numbers **258A, 258B, 258C,** and **258D** refer to a specific one of the unattached, distal-pointing crowns.

Optionally, at least two of the unattached, distal-pointing crowns **258** are located approximately 180 degrees (e.g., about 150 to about 180 degrees) relative to each other and approximately the same distance from the proximal hub/junction/tube **228,** as best seen in FIG. 12A. Optionally, the basket **246** comprises a first pair of unattached, distal-pointing crowns **258A** and **258B,** each of the first pair of unattached, distal-pointing crowns **258A** and **258B** is located approximately the same distance from the proximal hub/junction/tube **228** and approximately 180 degrees relative to each other, and the basket **246** further comprises a second pair of unattached, distal-pointing crowns **258C** and **258D** located distally relative to, and approximately 90 degrees (e.g., between about 60 and about 90 degrees) relative to, the first pair of unattached, distal-pointing crowns **258A** and **258B.** Optionally, the second pair of unattached, distal-pointing crowns **258C** and **258D** form cells **250C** and **250D** that are adjacent to, but offset from, the cells **250A** and **250B** formed by the first pair of unattached, distal-pointing crowns **258A** and **258B.** (In other words, optionally, the center of cell **250A** is about 90 degrees relative to the centers of cells **250C** and **250D** and optionally the center of cell **250B** is also about 90 degrees relative to the centers of cells **250C** and **250D**). Optionally, at least one of (and preferably all) the unattached, distal-pointing crowns **258A, 258B, 258C or 258D** comprise an x-ray marker **244** that is more visible under x-ray as compared to the basket strips **291** when the distal body **216** is located in a cranial blood vessel **266** inside the body of a human and the x-ray is taken from outside the human's body. Preferably, the x-ray marker **244** is a radiopaque material. Some examples of radiopaque materials can include, but are not limited to, gold, platinum, palladium, tantalum, tungsten alloy, polymer material loaded with radiopaque filler, and the like. Preferably, the basket strips **291** are comprised of nitinol and the x-ray marker **244** is comprised of a material having a density greater than the nitinol. In some examples, the x-ray markers **244** comprise a heavy metal welded or soldered to the unattached, distal-pointing crowns **258.** Optionally, the unattached, distal-pointing crowns **258** curve subtly towards the interior **222** of the distal basket **246,** which decreases the likelihood that the unattached, distal-pointing crowns **258** will rub against and damage the vessel wall **268.** Optionally, the basket **246** comprises at least two cells proximal to the at least two cells **250** that include the unattached, distal-pointing crowns **258.** Optionally, the unattached, distal-pointing distal crowns **258** are located about at least 5 mm (e.g., about 5 to about 30 mm) from the proximal hub/junction/tube **228.** Optionally, the unattached, distal-pointing crowns **258** are located at least about 5 mm from the distal hub/junction/tube **236.** Optionally, the unattached, distal-pointing crowns **258** of the at least two cells **250** also each form part (namely a portion of the proximal boundary) of an enlarged cell **262** (which is the entry point of hard thrombus **270B** into the basket interior **222**) and further wherein the surface area of the enlarged cells **262** in the relaxed state is greater than the surface area of the other cells of the basket **246** in the relaxed state. Optionally, the unattached, distal-pointing crowns **258** serve several functions: 1) they form flex points of the basket **246,** which makes it easier for the system **200** to navigate the curves of the blood vessels **266** of the brains; 2) through the use of x-ray markers **244** on the unattached, distal-pointing crowns **258,** they allow the operator to locate the enlarged cells **262** of the basket **246** that form the point at which hard thrombuses **270B** enter the basket **246;** and 3) they allow the operator to ratchet or force the object **270** into the basket **246** by moving the unattached, distal-pointing crowns **258** proximally and distally relative to the object **270.** (As explained below, the numeral **270** refers to clots/thrombuses and other objects generally, and **270A** refers to a soft clot, **270B** refers to a hard clot and **270C** refers to a deformable, cohesive, adherent clot). Optionally, the proximal end **254** of a proximal strip **252** is located about 65-180 degrees (preferably approximately 180 degrees) relative to the distal end **256** of the same proximal strip **252,** as best seen in FIG. 12B. In other words, preferably the proximal end **254** of a first proximal strip **252** is attached to the 12 o'clock position on the proximal tube **228** and the distal end **256** of the first proximal strip **252** (which terminates at a proximal cell **248** of the basket **246**) is located at the 6 o'clock position (i.e., 180 degrees from the start position), and the proximal end **254** of a second proximal strip **252** is attached to the 6 o'clock position on the proximal tube **228** and the distal end **254** (which terminates at a cell **248** of the basket **246)** of the second proximal strip **252** is located at the 12 o'clock position (i.e., 180 degrees from the start position). This twisting feature serves two functions: 1) it allows the proximal strips **252** to surround the object **270;** and 2) it allows the manufacturer to insert a mandrel into the basket **246** during the shape-setting procedure. Optionally, the pull wire **202** is attached to the proximal tube **228** (e.g., by gluing, welding, soldering or the like). Preferably, the pull wire **202** does not extend through the distal basket interior **222.** Optionally, the proximal strips **252** are integral with the distal end **232** of the proximal tube **228** and the entire distal body **216** is created from a single tube **264** of a memory metal. Optionally, the proximal crowns **260** of the at least two cells **250** that include the unattached, distal pointing-crowns **258** are each attached to another cell **248** of the basket **246.** In other words, preferably the basket **246** does not have any free-floating proximal-pointing crowns, as free-floating proximal-pointing crowns could damage the vessel **266** when the distal body **216** is pulled proximally. Optionally, the system **200** further comprises a lead wire **286** extending distally from the distal tube **236,** the lead wire **286** having a length of from about 3 mm to about 10 mm. Optionally, the distal hub/junction/tube **236,** the proximal hub/junction/tube **228,** and the basket **246** are comprised of a nitinol having the same material composition. In other words, as with the prior example of FIGs. 1-10, optionally the entire distal body **216** is manufactured from a single tube of nitinol **264.** Optionally, the proximal and distal hubs/junctions/tubes **228** and **236** comprise an x-ray marker **244** that is more visible under x-ray as compared to the basket strips **291** when the distal body **216** is located in a cranial blood vessel **266** inside the body of a human and the x-ray is taken from outside the human's body. Preferably, the x-ray marker **244** is a radiopaque material. Some examples of radiopaque materials can include, but are not limited to, gold, platinum, palladium, tantalum, tungsten alloy, polymer material loaded with radiopaque filler, and the like. Preferably, the basket strips **291** are comprised of nitinol and the x-ray marker **244** is comprised of a material having a density greater than the nitinol. In some examples, the proximal and distal hubs/junctions/tube interiors **234** and **242** may comprise tantalum welded or otherwise attached to the interior **234** and **242** of the proximal and distal hubs/junctions/tubes **228** and **236.** Optionally, the proximal and the distal tubes **228** and **236** are generally cylindrical in shape and each has an outer diameter and an inner diameter, the inner diameter forming apertures of the proximal and distal tubes **228** and **236** and further wherein the outer diameters of the proximal and distal tubes **228** and **23** are substantially the same size and further wherein the inner diameters of the proximal and distal tubes **228** and **236** are substantially the same size. Optionally, the outer diameters of the proximal and distal tubes **228** and **236** are from about 0.011 inches to about 0.054 inches, and further wherein the inner diameters of the proximal and distal tubes **228** and **236** are from about 0.008 inches to about 0.051 inches. Optionally, the pull wire **202** is generally cylindrical and further wherein the diameter of the pull wire **202** is between about 0.008 inches and about 0.051 inches. Optionally, the distal body **216** has a length of between about 10 and about 60 millimeters. Optionally, the first height **224** and first width **226** of the distal body **216** are between about 2 millimeters and about 6 millimeters.

The present disclosure also provides for reference only a method of removing a clot or other object **270** from an interior lumen **266** of an animal, the method comprising the steps of:
a) providing the system **200** of Figures 11-29, wherein at least two cells **250** of the basket **246** comprise a proximal crown **260** pointing generally in the proximal direction and a distal crown **258** pointing generally in the distal direction, and the distal crowns **258** of the at least two cells **250** are not attached to another cell **248** of the basket **246** (i.e., free-floating), and further wherein at least one of the unattached, distal-pointing crowns **258** comprises an x-ray marker **244;**
b) positioning the system **200** in the lumen **266;**
c) deploying the distal body **216** from the distal end **214** of the delivery catheter **208;**
d) allowing the height and width **224** and **226** of the distal body **216** to increase;
e) irradiating the x-ray marker **244** with x-ray radiation and
f) moving the object **270** into the distal basket interior **222.**

Optionally, the object **270** enters the distal basket interior **222** adjacent to (preferably adjacent and immediately distal to) at least one of the unattached, distal-pointing crowns **258** - i.e., in the enlarged cells/drop zones **262.** In some examples, the distal body **216** is deployed so that at least one (e.g., preferably the two proximal **258A** and **258B**) of the unattached, distal-pointing crowns **258** is distal to the object **270.** As explained below, the x-ray markers **244** of the unattached, distal-pointing crowns **258** are used to locate the distal body **216** relative to the clot or other object **270.** It will be appreciated that clots **270** can generally be located in blood vessels **266** by injecting a contrast dye, for example, into the blood vessel **266** proximal and distal to the believed area of obstruction and viewing on an x-ray where the fluid stops moving in the blood vessel **266.** It will also be appreciated that if the object **270** is not a blood clot but is a radio-opaque object, the object **270** may be viewed on an x-ray.

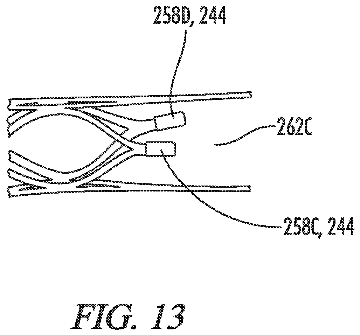

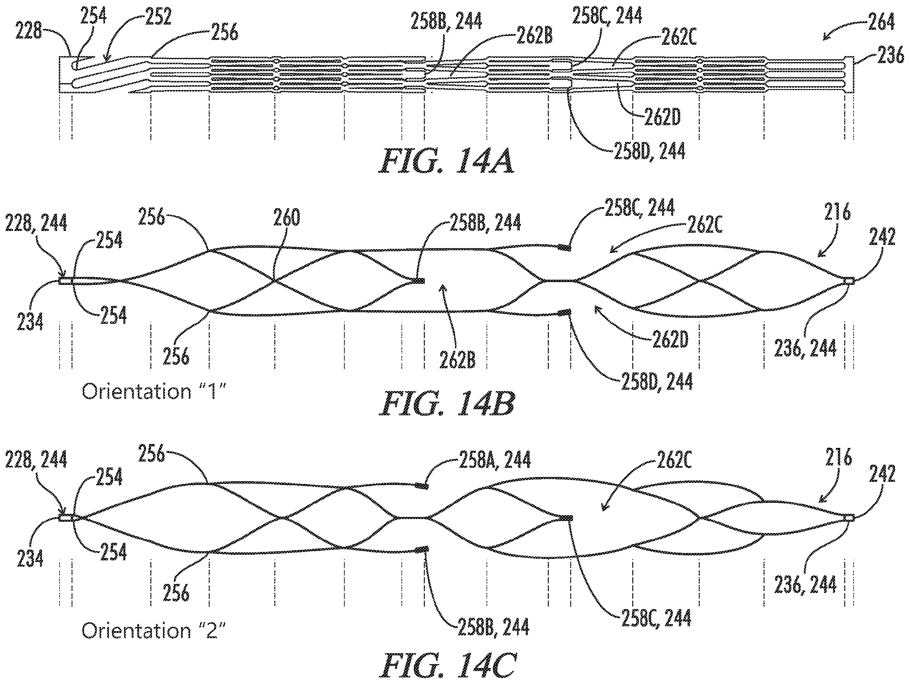

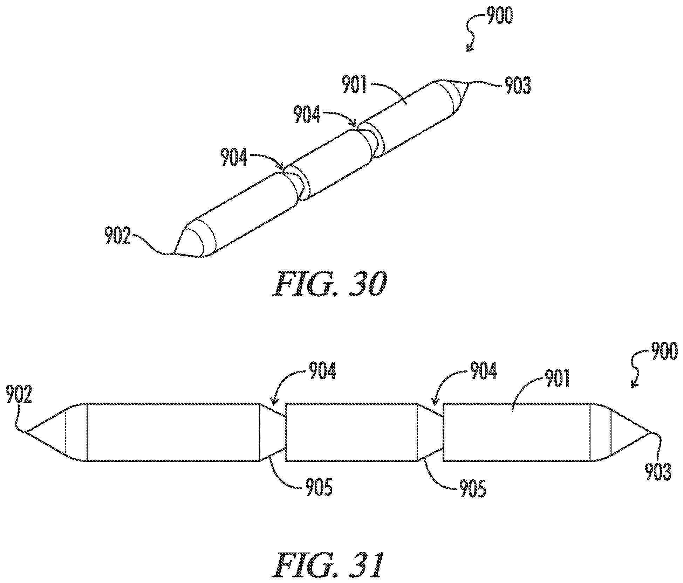

FIGs. 11 and 14B illustrate a first, perspective view of one example of a distal body **216** with twisting proximal strips **252,** unattached distal-pointing crowns **258** that subtly curve inward and have x-ray markers **244,** and enlarged openings/drop zones **262** in the basket **246** that allow the obstruction or other object **270** to enter. In FIGs. 11 and 14B, the distal body **216** is in Orientation 1. (To prepare a basket **246** with unattached distal-pointing crowns **258** that curve inward toward the basket interior **292,** a mandrel 900 such as that illustrated in FIGs. 30 and 31 may be used. The mandrel **900** includes a generally cylindrical body **901** with tapered proximal and distal ends **902** and **903** that slope like the ends of a pencil. The cylindrical body **901** includes two grooves **904** that extend around the circumference of the cylindrical body **901.** The grooves **904** include tapered portions **905** that slope towards the distal end **903,** which are designed to shape the unattached distal-pointing crowns **258.** The grooves **904** are generally in the shape of a truncated cone, as shown in FIGs. 30-31). The two proximal, unattached distal-pointing crowns **258A** and **258B** are located approximately the same distance from the proximal hub/junction/tube **228** and are oriented approximately 180 degrees relative to each other. The two distal, unattached distal-pointing crowns **258C** and **258D** are located approximately the same distance from the proximal hub/junction/tube **228** as each other (and distal to the two proximal, unattached distal-pointing crowns **258A** and **258B)** and are oriented approximately 180 degrees relative to each other and approximately 90 degrees to the proximal, unattached distal-pointing crowns **258A** and **258B.** The two proximal enlarged openings/drop zones **262A** and **262B** distal to the proximal, unattached distal pointing crowns **258A** and **258B** are located approximately the same distance from the proximal hub/junction/tube **228** and the centers of the two proximal enlarged openings/drop zones **262A** and **262B** are oriented approximately 180 degrees relative to each other. (As noted above, preferably, the proximal, unattached distal-pointing crowns **258A** and **258B** form part of the proximal boundary of the proximal, enlarged cells/drop zones **262A** and **262B,** and the distal, unattached distal-pointing crowns **258C** and **258C** form part of the proximal boundary of the distal, enlarged cells/drop zones **262C** and **262D**). The two distal, enlarged openings/drop zones **262C** and **262D** distal to the distal, unattached distal pointing crowns **258C** and **258D** are located approximately the same distance from the proximal hub/junction/tube **228** and the centers of the distal, enlarged openings/drop zones **262C** and **262D** are oriented approximately 180 degrees relative to each other and approximately 90 degrees relative to the proximal enlarged openings/drop zones **262A** and **262B.** FIGs. 12A and 14C illustrate a second view of the distal body **216** of FIG. 11 (Orientation 2). FIG. 13 is a close-up view of two unattached, distal-pointing crowns **262.** The lines in FIG. 14 show how a nitinol tube **264** is cut with a laser to create the distal body **216** shown in FIG. 14B and FIG. 14C. It will be appreciated that FIG. 14B is a simplified view of the distal body **216** and orientation shown in FIG. 11 and FIG. 14C is a simplified view of the distal body **216** and orientation shown in FIG. 12A.

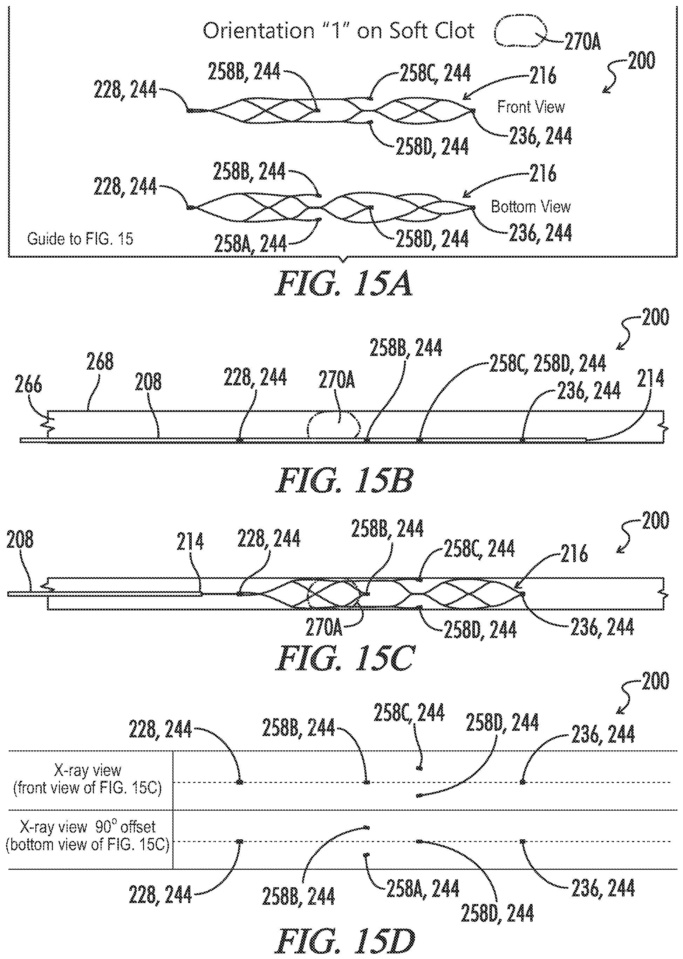

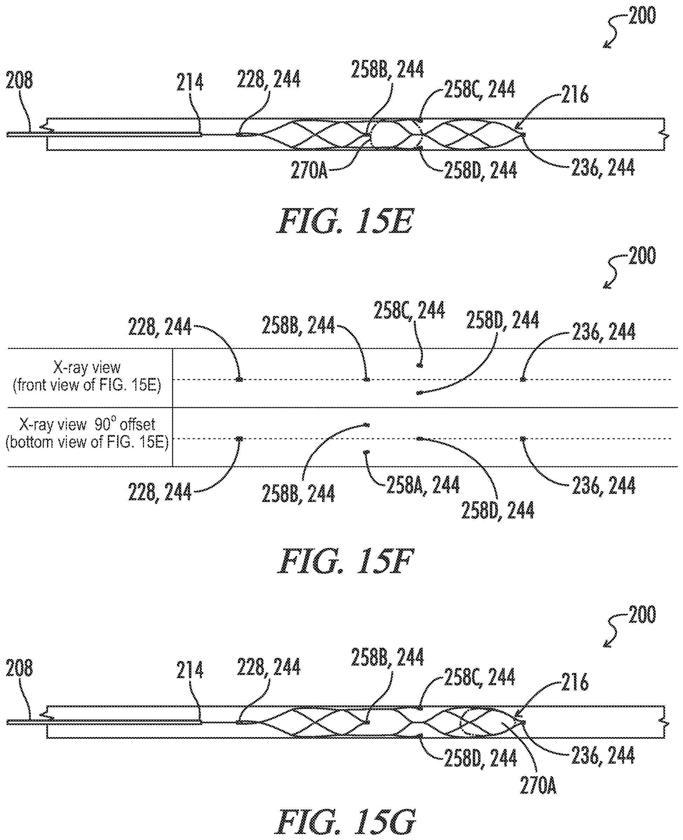

As described below, FIGs. 15-19 describe how the distal body **216** is used to retrieve, soft clots **270A,** hard clots **270B,** and deformable, cohesive adhesive clots **270C** in a human intracranial artery **266.** (In FIGs. 15-19, the center of the artery **266** is denominated by the dashed line). As explained below, the distal body **216** has four rows of x-ray markers namely, 1) a first row of one x-ray marker, which is located inside the proximal tube denominated by the numeral **228, 244;** 2) a second row of two x-ray markers, which are located at the two proximal, unattached distal-pointing crowns (the two markers are oriented 180 degrees relative to each other) denominated by the numerals **258A, 244** and **258B, 244;** 3) a third row of two x-ray markers, which are located at the two distal, unattached distal-pointing crowns (these two markers are oriented 180 degrees relative to each other and 90 degrees relative to the two proximal, unattached distal-pointing crowns) denominated by the numerals **258C, 244** and **258D, 244;** and 4) a fourth row of one x-ray marker, which is located inside the distal tube denominated by the numeral **236, 244.** (It will be appreciated that the first number in the sequence describes the position of the x-ray marker and the second number, **244,** represents the fact that the item is an x-ray marker). As explained below, upon deploying the distal body **216** so that the two proximal, unattached distal-pointing crowns **258A, 244** and **258B, 244** are immediately distal to the clot **270,** the surgeon interventionalist (i.e., operator of the distal body **216**) detects the four rows of x-ray markers using x-ray radiation from a first vantage point and from a second vantage point that is offset from the first vantage point (e.g. 90 degrees). Next, the surgeon moves the distal body **216** proximally relative to the clot **270** and takes additional x-rays from the first and second vantage points. As explained in greater detail below, the surgeon uses the x-ray markers of the proximal and distal, unattached distal-pointing crowns, namely **258A, 244; 258B, 244; 258C, 244;** and **258D, 244** (more specifically, the convergence or lack thereof of the proximal and distal, unattached distal-pointing crowns **258A, 244; 258B, 244; 258C, 244;** and **258D, 244** as shown on the x-ray) to determine whether the clot **270** is located inside the distal body interior **222** or whether the clot **270** is collapsing the distal body **216.**

More specifically, FIGs. 15A-G illustrate stepwise use of the distal body **216** in retrieving a soft clot **270A** in a human intracranial artery **266.** (The distal body **216** in FIGS. 15A-15G is in Orientation 1). First, as always, the surgeon determines the location of the clot **270A** in the vessel **266** using, for example, a contrast dye injected proximal and distal to the clot **270A.** Next, the delivery catheter **208,** which is enveloping the distal body **216,** is positioned in the blood vessel **266** so that the two proximal, unattached distal-pointing crowns **258A** and **258B** are immediately distal to the clot **270A.** See FIG. 15B. The distal body **216** is then deployed from the delivery catheter **208** by moving the catheter **208** proximally. The soft clot **270A,** which is unable to collapse the distal body **216,** then enters the distal body interior **222.** See FIG. 15C. However, at this time, the surgeon is unaware that the clot **270A** has entered into the distal body interior **222.** Thus, without moving the distal body **216,** the surgeon irradiates the four rows of x-ray markers at a first vantage point (i.e., from the front of the distal body **216** in the orientation shown in FIGs. 15A-G; i.e., into the page). As shown in FIG. 15D, the first vantage point shows four rows of x-ray markers. The first row is a single point, which represents the x-ray marker located in the proximal tube **228, 244;** the proximal tube x-ray marker **228, 244** always appears as a single point. The second row is a single point, which represents the x-ray marker located at the front, proximal, unattached distal-pointing crown **258B, 244;** the reason that this second row of markers is a single point is that the rear x-ray marker of the second row **258A, 244** is hidden from view because it is directly behind the front x-ray marker of the second row **258B, 244.** The third row has two points, which represents the two x-ray markers located at the distal, unattached distal-pointing crowns **258C, 244** and **258D, 244;** the reason that this third row of markers has two points is that neither marker in the third row **258C, 244** and **258D, 244** is hidden from view on the x-ray at this angle - rather, one marker **258C, 244** is located above the other marker **258D, 244** - and as shown in FIG. 15C, the distal body **216** is not collapsed at the distal, unattached distal-pointing crowns **258C, 244** and **258D, 244.** The fourth row is a single point, which represents the x-ray marker located in the distal tube **236, 244;** the distal tube x-ray marker **236, 244** always appears as a single point. Without moving the distal body **216,** the surgeon then irradiates the four rows of x-ray markers from a second vantage point 90 degrees offset from the first vantage point (i.e., from the bottom of the distal body **216** in the orientation shown in FIG. 15A). As shown, the first row is, as always, a single point, which represents the x-ray marker located in the proximal tube **228, 244.** The second row has two points, which represents the two x-ray markers located at the proximal, unattached distal-pointing crown **258A, 244** and **258B, 244;** the reason that this second row of markers shows up as two points is that neither marker **258A, 244** and **258B, 244** in the second row is hidden from view on the x-ray at this offset angle - rather, one marker **258B, 244** is located above the other marker **258A, 244** - and the distal body **216** is not collapsed at the proximal, unattached distal-pointing crowns **258A, 244** and **258B, 244.** The third row is a single point, which represents the x-ray marker located at the bottom, distal, unattached distal-pointing crown **258D, 244;** the reason that this third row of markers is a single point is that the top x-ray marker of the third row **258C, 244** is directly behind the bottom x-ray marker of the third row **258D, 244,** and thus, hidden from view. The fourth row is, as always, a single point, which represents the x-ray marker located in the distal tube **236, 244.** The surgeon, thus, concludes that neither the x-ray markers at the second row **258A, 244** and **258B, 244** nor the x-ray markers at the third row **258C, 244** and **258D, 244** (i.e., the x-ray markers at both the proximal and distal unattached distal pointing-crowns) have converged. As shown in FIG. 15E, the surgeon then moves the distal body **216** proximally relative to the soft clot **270A** so that the distal, unattached distal-pointing crowns **258C, 244** and **258D, 244** are immediately distal to the clot **270A** and then the surgeon irradiates the four rows of x-ray markers again from the first vantage point and the second vantage point. As shown in FIG. 15F, the results are the same as FIG. 15D. With the results from FIGs. 15D and 15F, the surgeon concludes that neither x-ray markers at the second row **258A, 244** and **258B, 244** nor the x-ray markers at the third row **258C, 244** and **258D, 244** (i.e., the x-ray markers at both the proximal and distal unattached distal pointing-crowns) converged at either the original position of the distal body **216** (FIGs. 15C and 15D) or the position after moving the distal body **216** proximally (FIGs. 15E and 15F), and, thus, the distal body **216** was expanded in the vessel **266** in both positions. Thus, the surgeon concludes that the clot is a soft clot **270A** that has entered into the distal body interior **222** and the surgeon removes the distal body **216** and the soft clot **270A,** captured by the distal body **216,** by moving the distal body **216** proximally out of the vessel **266,** as shown in FIG. 15G.

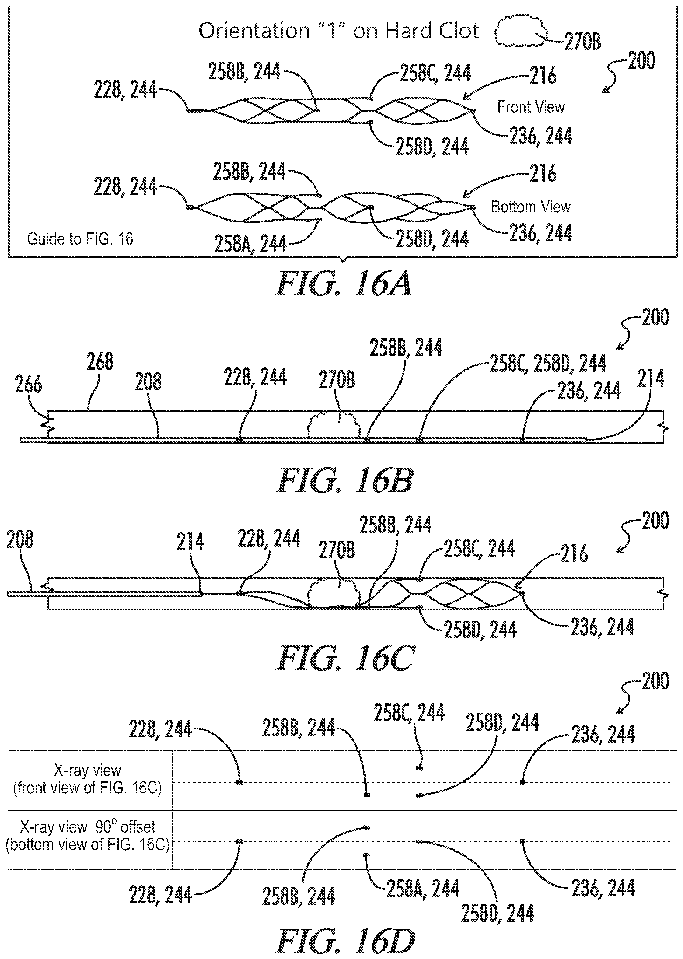

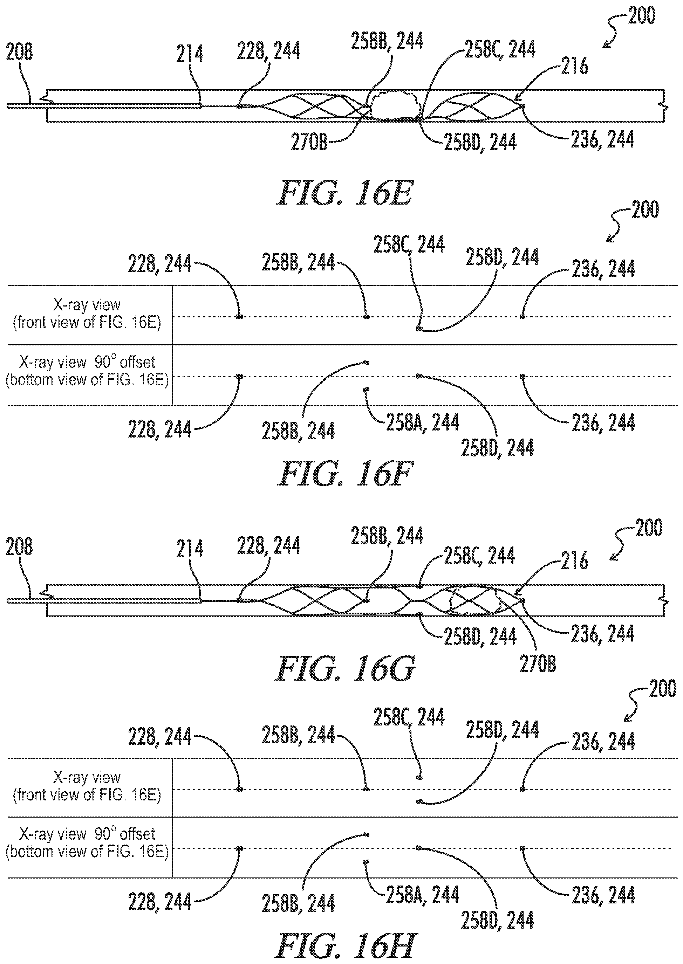

FIGs. 16A-H illustrate stepwise use of the distal body **216** in retrieving a hard clot **270B** in a human intracranial artery **266.** (In FIGs. 16A-H, the distal body **216** is in Orientation 1). First, as always, the surgeon determines the location of the clot **270B** in the vessel **266** using, for example, a contrast dye injected proximal and distal to the clot **270B.** Next, the delivery catheter **208,** which is enveloping the distal body **216,** is positioned in the blood vessel **266** so that the two proximal, unattached distal-pointing crowns **258A** and **258B** are immediately distal to the clot **270B.** See FIG. 16B. The distal body **216** is then deployed from the delivery catheter **208** by moving the catheter **208** proximally. The hard clot **270B,** which is located above the distal body **216,** collapses the distal body **216,** as shown in FIG. 16C. However, at this time, the surgeon is unaware that the clot **270B** has collapsed the distal body **216.** Thus, without moving the distal body **216,** the surgeon irradiates the x-ray markers at a first vantage point (i.e., from the front of the distal body **216;** i.e., into the page). As shown in FIG. 16D, the first vantage point shows four rows of x-ray markers. The first row is, as always, a single point, representing the x-ray marker located in the proximal tube - i.e., **228, 244.** The second row is a single point, which represents the x-ray marker located at the front, proximal, unattached distal-pointing crown **258B, 244;** the reason that this second row of markers is a single point is that the rear x-ray marker of the second row **258A, 244** is hidden from view because it is directly behind the front x-ray marker of the second row **258B, 244.** The third row has two points, which represents the two x-ray markers located at the distal, unattached distal-pointing crowns **258C, 244** and **258D, 244;** the reason that this third row of markers has two points is that neither marker in the third row is hidden from view on the x-ray at this angle - rather, one marker **258C, 244** is located above the other marker **258D, 244** - and as shown in FIG. 16C, the distal body **216** is not collapsed at the distal, unattached distal-pointing crowns **258C, 244** and **258D, 244.** The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body **216**). As shown, the first row is, as always, a single point, which represents the x-ray marker located in the proximal tube **228, 244.** The second row has two points, which represents the two x-ray markers located at the proximal, unattached distal-pointing crowns **258A, 244** and **258B, 244;** the reason that this second row of markers shows up as two points is that neither marker in the second row is hidden from view on the x-ray at this offset angle - rather, one marker **258B, 244** is located above the other marker **258A, 244** - and although the distal body **216** is collapsed at the proximal, unattached distal-pointing crowns as shown in FIG. 16C, the second row of x-ray markers have not converged because the clot **270B** is on top of the second row of x-ray markers. The third row is a single point, which represents the x-ray marker located at the bottom, distal, unattached distal-pointing crown **258D, 244;** the reason that this third row of markers is a single point is that the top x-ray marker of the third row **258C, 244** is directly behind the bottom x-ray marker of the third row **258D, 244,** and thus, hidden from view. The fourth row is, as always, a single point, which represents the x-ray marker located in the distal tube **236, 244.** The surgeon, thus, concludes that neither the second row **258A, 244** and **258B, 244** nor the third row **258C, 244** and **258D, 244** of x-ray markers (i.e., the x-ray markers at both the proximal and distal unattached distal pointing-crowns) has converged. As shown in FIG. 16E, the surgeon then moves the distal body **216** proximally so that the distal, unattached distal-pointing crowns **258C, 244** and **258D, 244** are immediately distal to the clot **270B** and the surgeon then irradiates the x-markers again from the first vantage point. As shown in FIG. 16F, the first row is, as always, a single point, representing the x-ray marker located in the proximal tube **228, 244.** The second row is a single point, which represents the x-ray marker located at the front, proximal, unattached distal-pointing crown **258B, 244;** the reason that this second row of markers is a single point is that the rear x-ray marker of the second row **258A, 244** is hidden from view because it is directly behind the front x-ray marker of the second row **258B, 244.** The third row has only one point because the clot **270B,** which is on top of the third row of x-ray markers **258C, 244** and **258D, 244** (i.e., the markers at the distal, unattached distal-pointing crowns), has pushed the third row of x-ray markers **258C, 244** and **258D, 244** together. The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body). As shown, the first row is, as always, a single point, which represents the x-ray marker located in the proximal tube **228, 244.** The second row has two points, which represents the two x-ray markers located at the proximal, unattached distal-pointing crown **258A, 244** and **258B, 244;** the reason that this second row of markers shows up as two points is that neither marker in the second row is hidden from view on the x-ray at this offset angle and the distal body **216** is not collapsed at the proximal, unattached distal-pointing crowns **258A, 244** and **258B, 244.** The third row is a single point, which represents the x-ray marker located at the bottom, distal, unattached distal-pointing crown **258D, 244;** the reason that this third row of markers is a single point is that the bottom x-ray marker of the third row **258D, 244** is directly in front of the top x-ray marker of the third row **258C, 244,** and thus, the top x-ray marker of the third row **258C, 244** is hidden from view. The fourth row is, as always, a single point, which represents the x-ray marker located in the distal tube **236, 244.** Knowing that the distal, unattached distal-pointing crowns **258C, 244** and **258D, 244** have converged as shown in FIG. 16F, the surgeon moves the distal body **216** proximally and the hard clot **270B** falls into the distal body interior **222** in the enlarged cell/drop zone **262C** immediately distal to the top, distal, unattached distal-pointing crown **258C.** See FIG. 16G. To confirm that the hard clot **270B** has entered the distal body interior **222,** the surgeon takes x-rays from the first and second vantage points. The results are shown in FIG. 16H. As compared to 16F, the front x-ray view of FIG. 16H shows that the distal, unattached distal-pointing crowns **258C, 244** and **258D, 244** are not converged, and, thus, the surgeon concludes that the hard clot **270B** has entered the distal body interior **222.** The surgeon then removes the distal body **216** and the hard clot **270B,** captured by the distal body **216,** by moving the distal body **216** proximally out of the vessel **266.**

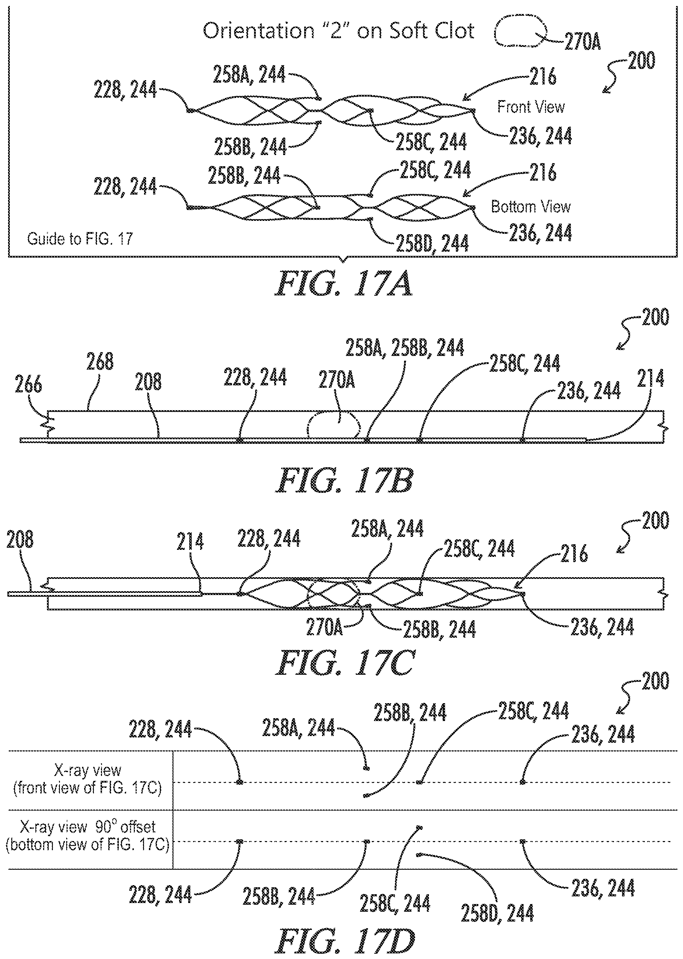

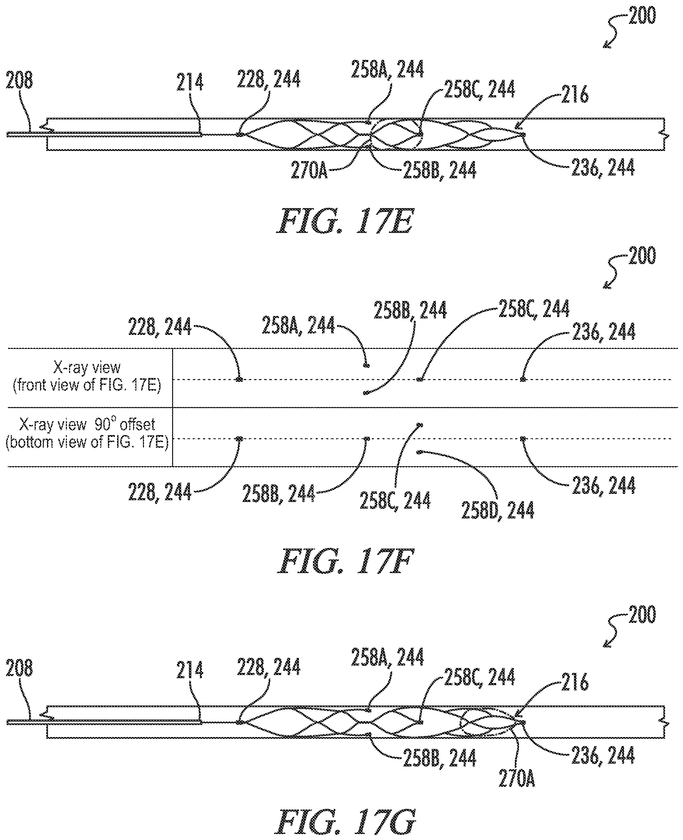

FIGs. 17A-G illustrate stepwise use of the distal body **216** in retrieving a soft clot **270A** in a human intracranial artery **266.** (In FIGs. 17A-G, the distal body **216** is in Orientation 2). First, as always, the surgeon determines the location of the clot **270A** in the vessel **266** using, for example, a contrast dye injected proximal and distal to the clot **270A.** Next, the delivery catheter **208,** which is enveloping the distal body **216,** is positioned in the blood vessel **266** so that the two proximal, unattached distal-pointing crowns **258A** and **258B** are immediately distal to the clot **270A.** See FIG. 17B. The distal body **216** is then deployed from the catheter **208** by moving the catheter **208** proximally. The soft clot **270A,** which is unable to collapse the distal body **216,** then enters the distal body interior **222.** See FIG. 17C. However, at this time, the surgeon is unaware that the clot **270A** has entered into the distal body interior **222.** Thus, without moving the distal body **216,** the surgeon irradiates the x-ray markers at a first vantage point (i.e., from the front of the distal body; into the page). As shown in FIG. 17D, the first vantage point shows four rows of x-ray markers. The first row is, as always, a single point, representing the x-ray marker located in the proximal tube **228, 244.** The second row has two points, which represents the two x-ray markers located at the proximal, unattached distal-pointing crowns **258A, 244** and **258B, 244;** the reason that this second row of markers has two points is that neither marker in the second row is hidden from view on the x-ray at this angle - rather, one marker **258A, 244** is located above the other marker **258B, 244** - and as shown in FIG. 17C, the distal body **216** is not collapsed at the proximal, unattached distal-pointing crowns **258A, 244** and **258B, 244.** The third row has a single point, which represents the x-ray marker located at the front (in Orientation 2), distal, unattached distal-pointing crown **258C, 244;** the reason that this third row of markers is a single point is that the rear (in Orientation 2) x-ray marker **258D, 244** of the third row is hidden from view because it is directly behind the front x-ray marker **258C, 244** of the third row. The fourth row is, as always, a single point, representing the x-ray marker located in the distal tube **236, 244.** Without moving the distal body, the surgeon then irradiates the markers from a second vantage point 90 degrees offset from the first vantage point (i.e., from the bottom of the distal body, as shown in this view). As shown, the first row is, as always, a single point, which represents the x-ray marker located in the proximal tube **228, 244.** The second row is a single point, which represents the x-ray marker located at the bottom (in Orientation 2), proximal, unattached distal-pointing crown **258B, 244;** the reason that this second row of markers is a single point is that the top (in Orientation 2) x-ray marker of the second row **258A, 244** is directly behind the bottom x-ray marker of the second row **258B, 244,** and thus, hidden from view. The third row has two points, which represents the two x-ray markers located at the distal, unattached distal-pointing crowns **258C, 244** and **258D, 244;** the reason that this third row of markers shows up as two points is that neither marker in the third row is hidden from view on the x-ray at this offset angle and the distal body **216** is not collapsed at the distal, unattached distal-pointing crowns **258C, 244** and **258D, 244.** The fourth row is, as always, a single point, which represents the x-ray marker located in the distal tube **236, 244.** The surgeon, thus, concludes that neither the second row **258A, 244** and **258B, 244** nor the third row of x-ray markers **258C, 244** and **258D, 244** (i.e., the x-ray markers at both the proximal and distal unattached distal pointing-crowns) has converged. As shown in FIG. 17E, the surgeon then moves the distal body **216** proximally relative to the clot **270A** so that the distal, unattached distal-pointing crowns **258C, 244** and **258D, 244** are immediately distal to the clot **270A** and then the surgeon irradiates the x-markers again from the first vantage point and the second vantage point. As shown in FIG. 17F, the results are the same as FIG. 17D. With the results from FIGs. 17D and 17F, the surgeon concludes that neither the second row **258A, 244** and **258B, 244** nor the third row of x-ray markers **258C, 244** and **258D, 244** (i.e., the x-ray markers at both the proximal and distal unattached distal pointing-crowns) were converged at either the original position of the distal body **216** (FIG. 17C and 17D) or the position after moving the distal body **216** proximally (FIG. 17E and 17F), and, thus, the distal body **216** was expanded in the vessel **266** in both positions. Thus, the surgeon concludes that the clot **270A** is a soft clot **270A** that has entered into the distal body interior **222** and the surgeon removes the distal body **216** and the soft clot **270A,** captured by the distal body **216,** by moving the distal body **216** proximally out of the vessel **266,** as shown in FIG. 17G.

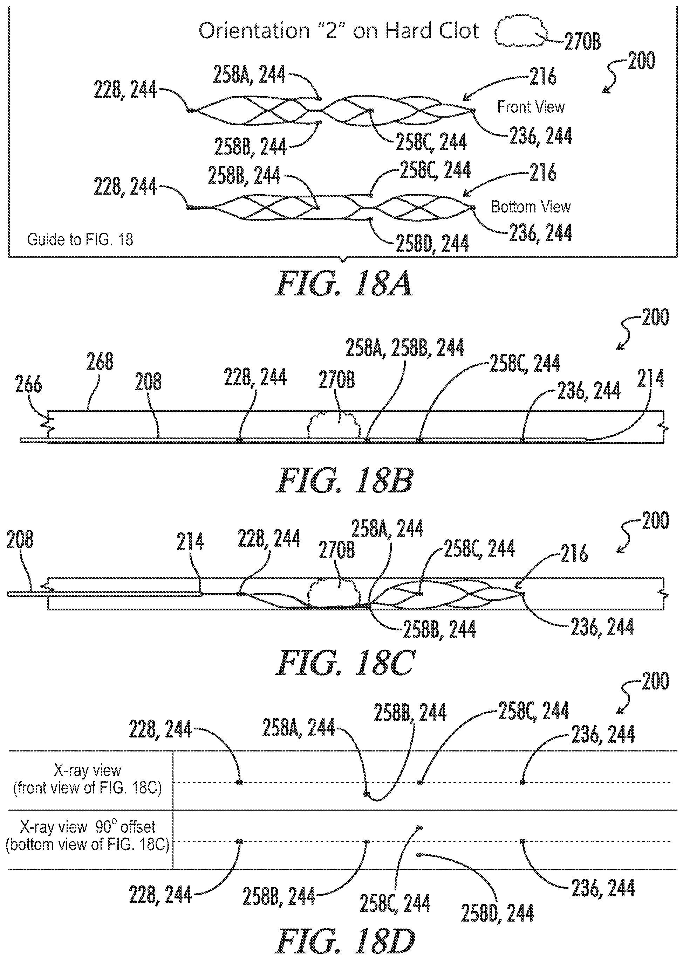

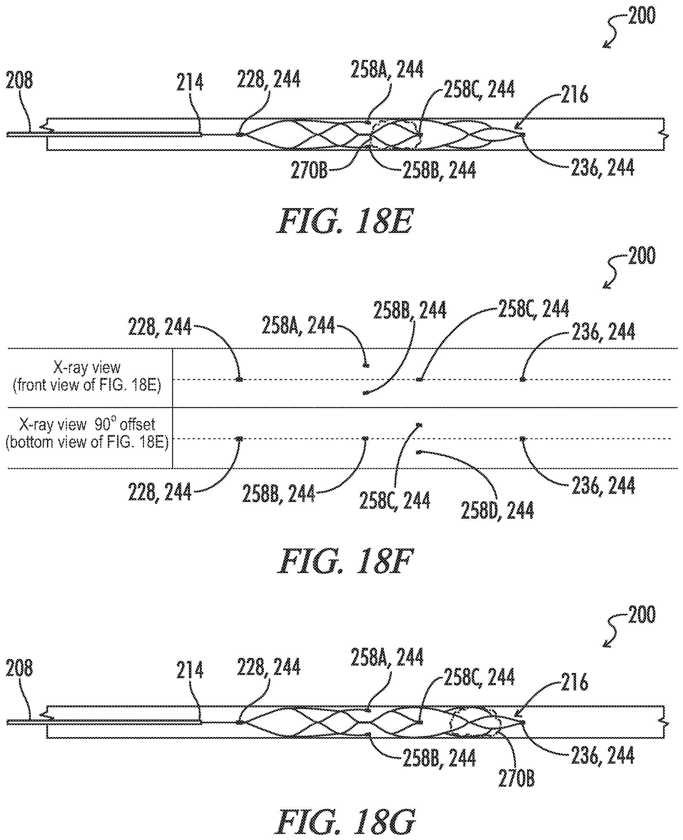

FIGs. 18A-G illustrate stepwise use of the distal body **216** in retrieving a hard clot **270B** in a human intracranial artery **266.** (In FIGS. 18A-G, the distal body **216** is in Orientation 2). (As described below, the primary differences between FIGs 18A-G and FIGs. 16A-G is that the clot **270B** enters the distal body interior **222** in an enlarged cell/drop zone **262A** immediately distal to one of the proximal, unattached distal-pointing crowns **258A** in FIGs. 18A-G, as compared to FIGs. 16A-G where the clot **270B** enters the distal body interior **222** in an enlarged cell/drop zone **262C** immediately distal to one of the distal, unattached distal-pointing crowns **258C**). First, as always, the surgeon determines the location of the clot **270B** in the vessel **266** using, for example, a contrast dye injected proximal and distal to the clot **270B.** Next, the delivery catheter **208,** which is enveloping the distal body **216,** is positioned in the blood vessel **266** so that the two proximal, unattached distal-pointing crowns **258A** and **258B** are immediately distal to the clot **270B.** See FIG. 18B. The distal body **216** is then deployed from the catheter **208** by moving the catheter **208** proximally. The hard clot **270B,** which is located above the distal body **216,** collapses the distal body **216,** as shown in FIG. 18C. However, at this time, the surgeon is unaware that the clot **270B** has collapsed the distal body **216.** Thus, without moving the distal body **216,** the surgeon irradiates the x-ray markers at a first vantage point (i.e., from the front of the distal body in Orientation 2; into the page). As shown in FIG. 18D, the first vantage point shows four rows of x-ray markers. The first row is, as always, a single point, representing the x-ray marker located in the proximal tube **228, 244.** The second row has only one point because the clot **270B,** which is on top of the second row of x-ray markers **258A, 244** and **258B, 244** (i.e., the markers at the proximal, unattached distal-pointing crowns), has pushed them together. The third row has only one point, which represents the x-ray marker located at the front (in Orientation 2), proximal, unattached distal-pointing crown **258C, 244;** the reason that this third row of markers is a single point is that the rear (in this view) x-ray marker of the third row **258D, 244** is hidden from view because it is directly behind the front x-ray marker of the third row **258C, 244.** The fourth row is, as always, a single point, representing the x-ray marker located in the distal tube **236, 244.** Without moving the distal body, the surgeon then irradiates the markers from a second vantage point 90 degrees offset from the first vantage point (i.e., from the bottom of the distal body **216**). As shown, the first row is, as always, a single point, which represents the x-ray marker located in the proximal tube **228, 244.** The second row has a single point because the top (in Orientation 2) x-ray marker of the second row **258A, 244** is located behind the bottom (in Orientation 2) x-ray marker **258B, 244** and thus, the top x-ray marker of the second row **258A, 244** is hidden from view. The third row has two points, which represents the x-ray markers located at the distal, unattached distal-pointing crowns **258C, 244** and **258D, 244;** in this x-ray view neither of the x-ray markers of the third row is hidden from view. The fourth row is, as always, a single point, which represents the x-ray marker located in the distal tube **236, 244.** The surgeon, thus, concludes that the second row of x-ray markers **258A, 244** and **258B, 244** (i.e., the x-ray markers at the proximal, unattached distal pointing-crowns) has converged. As shown in FIG. 18E, the surgeon then moves the distal body **216** proximally so that the distal, unattached distal-pointing crowns **258C, 244** and **258D, 244** are immediately distal to the clot **270B.** Unbeknownst to the surgeon, the clot **270B** enters the distal body interior **222** immediately distal to the top (in Orientation 2), proximal unattached distal-pointing crown **258A** and the distal body **216** is no longer collapsed. The surgeon then irradiates the x-markers again from the first vantage point. As shown in FIG. 18F, the first row is, as always, a single point, representing the x-ray marker located in the proximal tube **228, 244.** The second row has two x-ray markers because the distal body **216** is not collapsed and neither the top (in Orientation 2) **258A, 244** nor the bottom **258B, 244** (in Orientation 2) x-ray marker of the second row (i.e., the marker at the proximal, unattached distal-pointing crowns) is hidden from view. The third row has only one point because the rear (in Orientation 2), distal unattached distal-pointing crown **258D, 244** is hidden behind the front (in Orientation 2), distal, unattached distal pointing-crown **258C, 244.** The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body **216**). As shown, the first row is, as always, a single point, which represents the x-ray marker located in the proximal tube **228, 244.** The second row has a single point because the x-ray marker at the top (in Orientation 2), proximal, unattached distal-pointing crown **258A, 244** is hidden behind the bottom (in Orientation 2), proximal, unattached-distal pointing crown **258B, 244.** The third row has two points because neither the front nor the rear x-ray markers at the distal, unattached, distal-pointing crowns **258C, 244** and **258D, 244** is hidden from view. The fourth row is, as always, a single point, which represents the x-ray marker located in the distal tube **236, 244.** Based on the information from FIGs. 18D and 18F, the surgeon concludes that the clot **270B** has entered into the distal body interior **222.** The surgeon then removes the distal body **216** and the hard clot **270B,** captured by the distal body **216,** by moving the distal body **216** proximally out of the vessel **266,** as shown in FIG. 18G. Upon comparing FIGs. 16A-G and FIGs. 18A-G it will be appreciated that the orientation of the enlarged cells/drop zone **262A-D** relative to the orientation of a hard clot **270B** determine which enlarged cell/drop zone **262A, 262B, 262C,** or **262D,** the hard clot **270** enters the distal body interior **222** through. For example, in FIG. 16C, the hard clot **270B** is located above the distal body **216,** and thus, the hard clot **270B** must enter through the enlarged cell/drop zone located at the top of the distal body, which in the orientation of the distal body shown in FIGs. 16A-G, is the enlarged cell/drop zone **262C** immediately distal to the top, distal, unattached, distal-pointing crown **258C.** In FIG. 18C, the hard clot **270B** is again located above the distal body and, thus, the hard clot **270B** must enter through the enlarged cell/drop zone located at the top of the distal body. However, in FIG. 18C, the enlarged cell/drop zone located at the top of the distal body **216,** in the orientation of the distal body **216** shown in FIGs. 18A-G, is the enlarged cell/drop zone **262A** immediately distal to the top, proximal, unattached, distal-pointing crown **258A.**

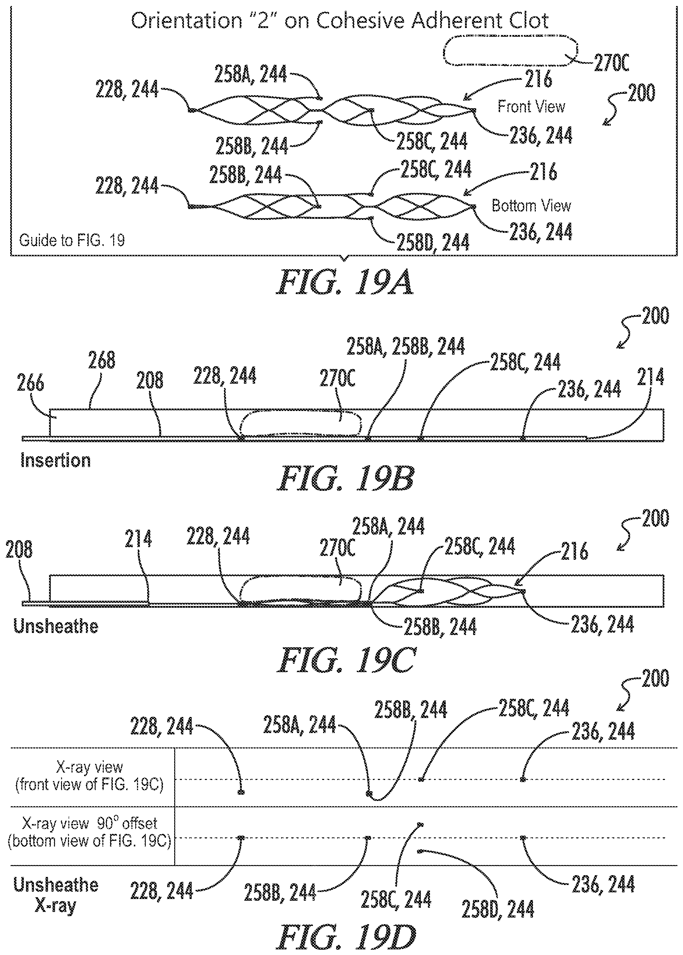

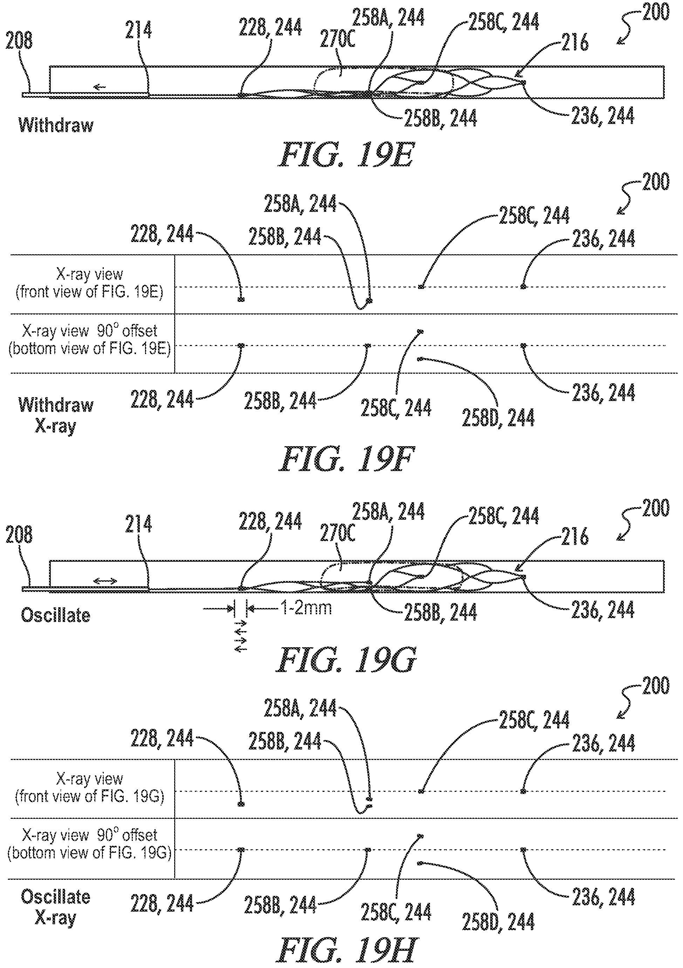

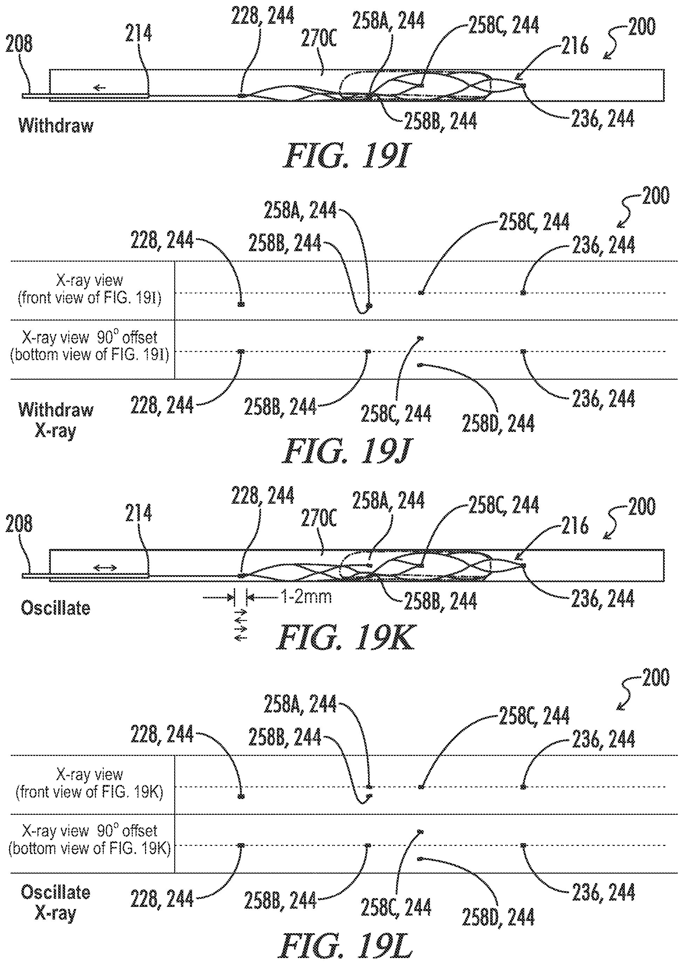

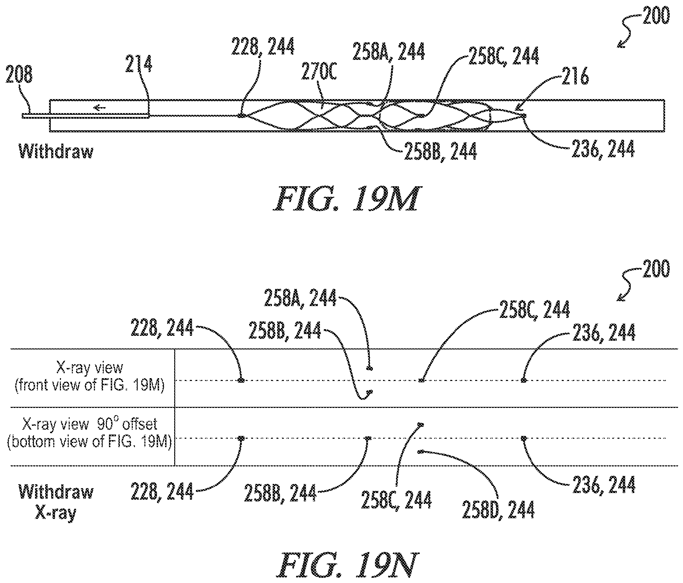

FIGs. 19A-N illustrate stepwise use of the distal body **216** in retrieving a deformable cohesive, adherent clot **270C-** i.e., a clot that is difficult to break up and is tightly adhered to the vessel wall **268** - in a human intracranial artery **266.** (In FIGS. 19A-N, the distal body **216** is in Orientation 2). First, as always, the surgeon determines the location of the clot **270C** in the vessel **266** using, for example, a contrast dye injected proximal and distal to the clot **270C.** Next, the delivery catheter **208,** which is enveloping the distal body **216,** is positioned in the blood vessel **266** so that the two proximal, unattached distal-pointing crowns **258A** and **258B** are immediately distal to the clot **270C.** See FIG. 19B. The distal body **216** is then deployed from the catheter **208** by moving the catheter **208** proximally. The deformable, cohesive adherent clot **270C,** which is located above the distal body **216,** collapses the distal body **216,** as shown in FIG. 19C. However, at this time, the surgeon is unaware that the clot **270C** has collapsed the distal body **216.** Thus, without moving the distal body **216,** the surgeon irradiates the x-ray markers at a first vantage point (i.e., from the front of the distal body; i.e., into the page). As shown in FIG. 19D, the first vantage point shows four rows of x-ray markers. The first row is, as always, a single point, representing the x-ray marker located in the proximal tube **228, 244.** The second row has a single point, corresponding to the top (in Orientation 2) and bottom (in Orientation 2), proximal, unattached distal-pointing crowns **258A, 244** and **258B, 244,** which have converged because the clot **270C** is collapsing the distal body **216.** The third row has a single point, which represents the x-ray marker located at the front (in Orientation 2), distal, unattached distal-pointing crown **258C, 244;** the x-ray marker located at the rear, distal, unattached distal-pointing crown **258D, 244** is hidden from view. The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body). As shown, the first row is, as always, a single point, which represents the x-ray marker located in the proximal tube **228, 244.** The second row has a single point, which corresponds to the bottom (in Orientation 2), proximal, unattached distal-pointing crown **258B, 244;** the top (in Orientation 2), proximal, unattached distal-pointing crown **258A, 244** is located behind the bottom, proximal, unattached distal-pointing crown **258B, 244** and hidden from view. The third row has two points, which correspond to the front (in Orientation 2) **258C, 244** and rear **258D, 244** (in Orientation 2), distal, unattached distal-pointing crowns, neither of which is blocked in this view. The fourth row is, as always, a single point, which represents the x-ray marker located in the distal tube **236, 244.** As shown in FIG. 19E, the surgeon then moves the distal body **216** proximally (i.e., slightly withdraws the distal body **216**). The surgeon then irradiates the x-markers again from the first and second vantage points. As shown in FIG. 19F, the results are exactly the same as in FIG. 19D. Based on the observation that the proximal, unattached distal-pointing crowns **258A, 244** and **258B, 244** have converged at both the original position (FIGs. 19C and 19D in which the proximal, unattached distal-pointing crowns **258A, 244** and **258B, 244** are immediately distal to the clot **270C**) and the second position (FIGs. 19E and 19F), the surgeon concludes that the clot **270C** is a deformable cohesive, adherent clot **270C.** The surgeon then oscillates the distal body **216** proximally and distally a small distance (e.g., about 1mm to about 2 mm) in the vessel **266,** and the clot **270C** begins to enter the distal body **216,** as shown in FIG. 19G. The surgeon then irradiates the x-markers again from the first and second vantage points. As shown in FIG. 19H, the results are exactly the same as in FIG. 19D and FIG. 19F except that the second row of markers **258A, 244** and **258B, 244** (at the proximal, unattached distal-pointing crowns) are beginning to move apart. The surgeon then moves the distal body **216** proximally again, as shown in FIG. 19I. The surgeon then irradiates the x-markers again from the first and second vantage points. As shown in FIG. 19J, the results are exactly the same as in FIGs. 19D and 19F, as the clot **270C** has caused the second row of markers **258A, 244** and **258B, 244** to re-converge. The surgeon then oscillates the distal body **216** proximally and distally a small distance (e.g., about 1mm to about 2 mm) in the vessel **266,** and the clot **270C** begins to further enter the distal body interior **222,** as shown in FIG. 19K. The surgeon then irradiates the x-markers again from the first and second vantage points. As shown in FIG. 19L, the results are the same as in FIG. 19H. The surgeon then moves the distal body **216** again proximally, and, instead of collapsing the second row of markers **258A, 244** and **258B, 244,** the clot **270C** fully enters the distal body interior **222,** as shown in FIG. 19M. The surgeon then irradiates the x-markers again from the first and second vantage points. As shown in FIG. 19N, the results show that the second row of markers **258A, 244** and **258B, 244** (at the proximal, unattached distal-pointing crowns) have moved apart. Satisfied that the x-ray markers in the second row **258A, 244** and **258B, 244** (at the proximal, unattached distal-pointing crowns) are sufficiently far apart and that the x-ray markers in the third row (at the distal, unattached distal-pointing crowns) **258C, 244** and **258D, 244** have stayed far apart, the surgeon concludes that the deformable cohesive, adherent clot **270C** has been sufficiently captured by the distal body **216** and the surgeon then removes the distal body **216** and the clot **270C,** captured by the distal body **216,** by moving the distal body **216** proximally out of the vessel **266.**

Several observations can be made from FIGs. 15-19, as indicated above. For example, the x-ray markers at the proximal and distal, unattached distal-pointing crowns **258A-D, 244** provide the surgeon feedback concerning the interaction between the distal body **216** and the clot **270** in the blood vessel **266.** In addition, the guiding principle of a soft clot **270A** is that the soft clot **270A** does not collapse the distal body **216,** and thus, x-ray markers at the proximal and distal, unattached distal-pointing crowns **258A-D, 244** always appear as two points except when a marker is hidden behind another marker (due to the view). When it comes to a hard clot **270B,** the hard clot **270B** is generally able to enter the distal body interior **222** without needing to oscillate the distal body **216** proximally and distally (unlike a deformable cohesive, adherent clot **270C**). However, to capture the hard clot **270B,** the hard clot **270B** must be oriented properly relative to the enlarged cell/drop zones **262A, 262B, 262C,** or **262D.** (This is the reason that the distal body **216** has four enlarged cells/drop zones: one enlarged cells/drop zone at 0 degrees **262B,** one enlarged cells/drop zone at 90 degrees **262C,** one enlarged cells/drop zone at 180 degrees **262A** and one enlarged cells/drop zone at 270 degrees **262D**). As a guiding principle, an enlarged cell/drop zone **262A, 262B, 262C,** or **262D** is properly oriented to the clot **270B** when the x-ray markers at the proximal, unattached distal-pointing crowns **258A, 244** and **258B, 244** or the distal, unattached distal pointing crowns **258C, 244** and **258D, 244** are together at both a first x-ray view and a second x-ray view 90 degrees relative to the first x-ray view, and the hard clot **270B** can enter the enlarged cell/drop zone **262A, 262B, 262C,** or **262D** by moving the distal body **216** proximally. See FIG. 16F and 18D. Finally, the guiding principal of retrieval of deformable cohesive, adherent clots **270C** is that oscillation of the distal body **216** causes the deformable cohesive, adherent clots **270C** to gradually enter the distal basket interior **222** over time.

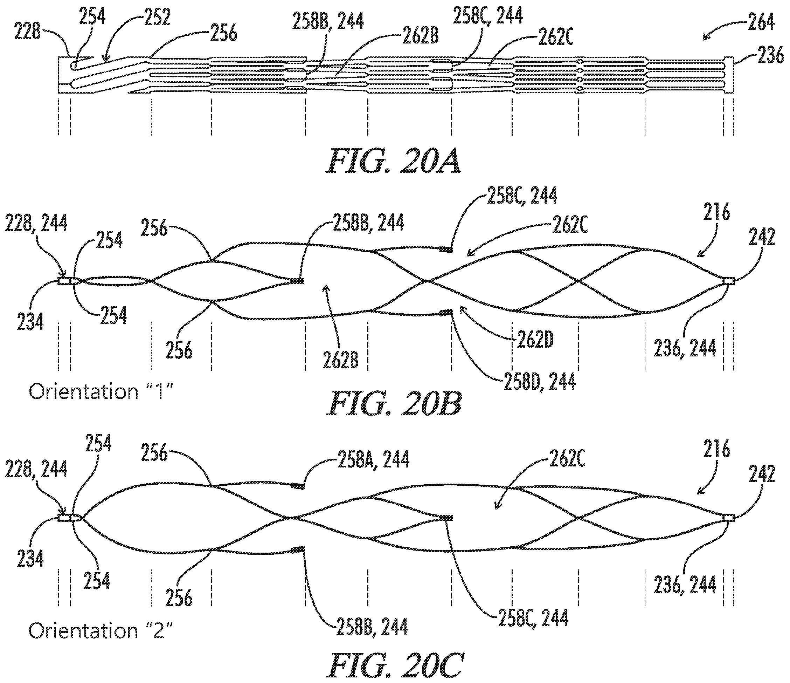

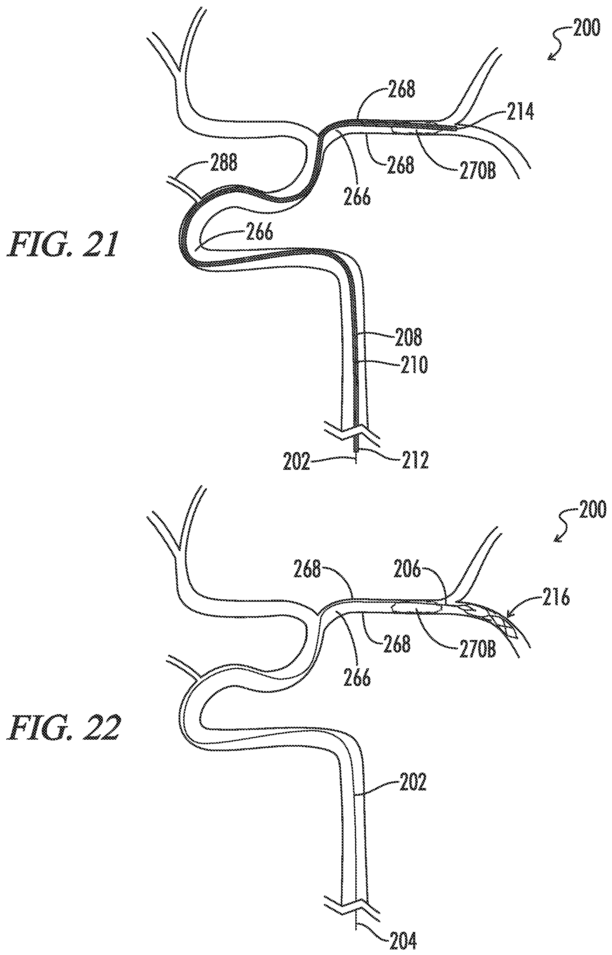

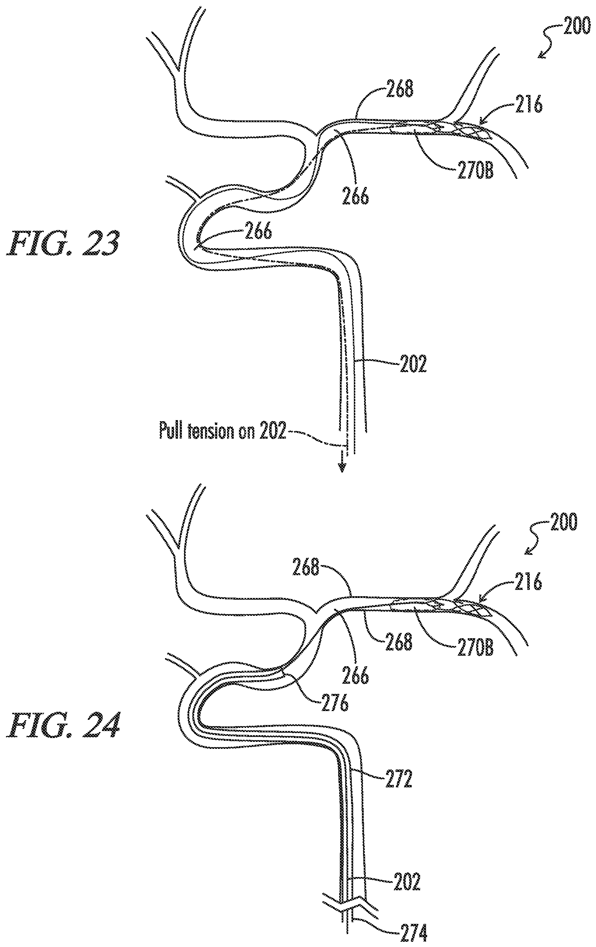

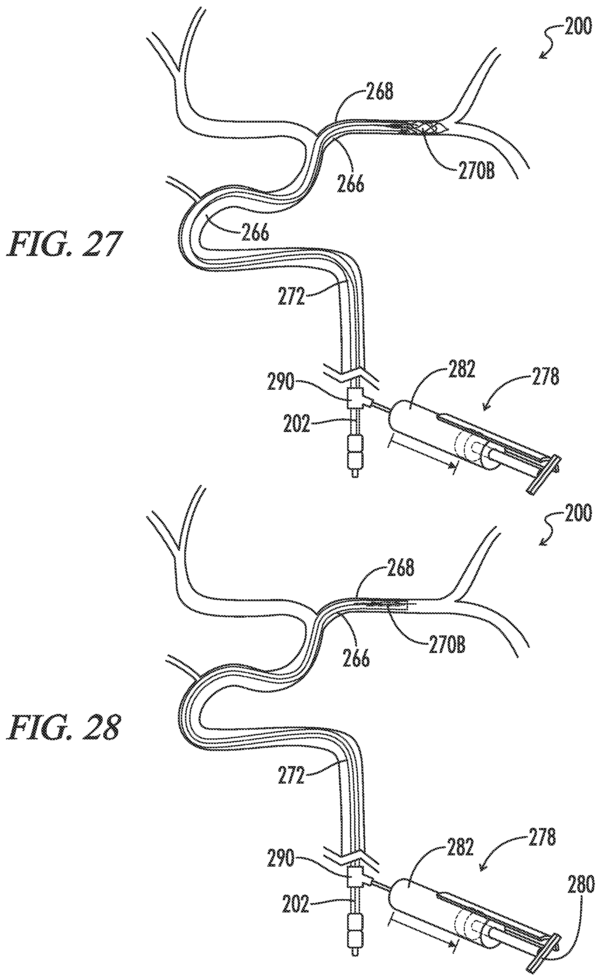

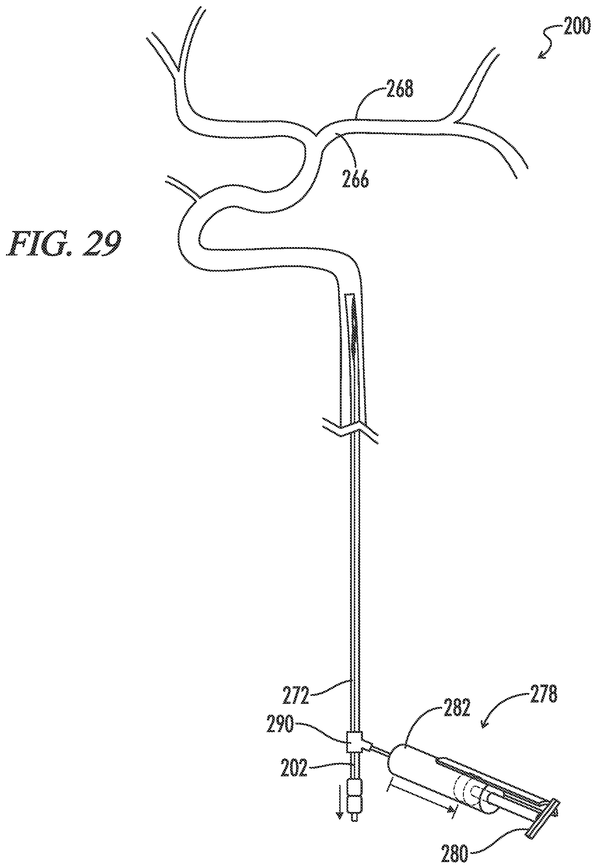

FIGs. 20A, 20B and 20C show a distal body **216** that is similar to the distal body **216** of FIGs. 14A, 14B and 14C except that the distal body **216** of FIGs. 20A, 20B and 20C is slightly shorter and its unattached, distal-pointing crowns **258A, 258B, 258C,** and **258D** are closer to the proximal tube **228.** The shortened distal body **216** of FIGs. 20A, 20B and 20C is particularly adapted for tortuous blood vessels **266.** FIG. 21-29 show stepwise deployment of the distal body **216** of FIGs. 20A, 20B and 20C in use with a manual (i.e., hand-operated), volume-dependent (i.e. volume locked) suction catheter **272** that is locked at between about 10 to about 60 cubic centimeters (cc). Optionally, the suction catheter **272** has an outer diameter of between about 0.05 inches and about 0.09 inches and its outer diameter is substantially larger than the outer diameter of the delivery catheter **208.** The clot **270** is located in the vessel **266** through the use of, for example, contrast dye injected proximal and distal to the clot **270.** As shown in FIG. 21, a delivery catheter **208** containing the distal body **216** of FIGs. 20A, 20B and 20C is positioned in the tortuous vessel **266** distal to the clot **270.** The delivery catheter **208** is withdrawn, deploying the distal body **216.** See FIG. 22. The distal body **216** is moved proximally relative to the clot **270** and tension is exerted on pull wire **202.** See FIG. 23. While maintaining tension on the pull wire **202,** a suction catheter **272** having a proximal end **274** and a distal end **276** is delivered over the pull wire **202** that is attached to the distal body **216.** See FIG. 24. (The reason for exerting tension on the pull wire **202** is that the pull wire **202** serves as the guide/track for the movement of the suction catheter **272** and without tension, the suction catheter **272** and pull wire **202** could end up in the ophthalmic artery **288**). The distal end **276** of the suction catheter **272** is positioned against the clot **270.** A syringe **278** is attached to the suction catheter **272** using a rotating hemostatic valve **290,** which allows the surgeon to aspirate while a pull wire **202** is in the system. The surgeon aspirates the syringe **278** by pulling back on the lever **280** to a mark on the base **282** corresponding to between about 10 and about 60 cubic centimeters of fluid. The surgeon then locks the lever **280** (and attached plunger) into place, leaving the suction catheter **272** under suction. The surgeon captures the clot **270** in the distal body **216** using the techniques described in FIGs. 15-19. The distal body **216** and clot **270** become captured by the suction catheter **272.** See FIGs. 27 and 28. The surgeon then removes the suction catheter **272** and the distal body **216** and the clot **270,** captured by the suction catheter **272,** by moving the suction catheter **272** proximally out of the vessel **266.** See FIG. 29. It is believed that the suction catheter **272** would be helpful in the event that a small portion of the clot **270** breaks off when retrieving the clot **270** using the distal body **216.**

To examine effectiveness of the systems **200,** the systems **200** of FIGs. 11-20, without the use of a suction catheter **272,** were used to retrieve soft and hard clots **270A** and **270B** induced in a pig weighing between 30 to 50 kg. The weight of the pig was chosen so that the size of its vessels **266** would be approximate to the size of a human vessel. The pig was anesthetized. Several hard clots **270B** were prepared by mixing pig blood and barium and incubating the mixture for 2 hours. Several soft clots **270A** were prepared by mixing pig blood, thrombin and barium and incubating the mixture for 1 hour. The clots **270A** and **270B,** each of which had a width of 4 to 6 mm and a length of 10 to 40 mm, were then inserted into a vessel **266** having a diameter of 2 to 4 mm. (Only one clot **270A** and **270B** was located in the vessel **266** at a time). Angiograms were then performed to confirm occlusion. After waiting ten minutes after confirming occlusion, the distal bodies **216** of FIGs. 11-20 were then delivered distal to the clots **270A** and **270B** as described above and were used to retrieve the clots **270A** and **270B** as described in FIGs. 11-19. In each case, the distal bodies **216** were successful in retrieving the clots **270A** and **270B.** As shown, the distal body height in the relaxed state tapers/decreases as the proximal strips **252** approach the proximal hub/junction/tube **228** and also tapers/decreases as the basket strips **291** located at the distal end **220** of the basket **246** converge at the distal hub/junction/tube **236.**

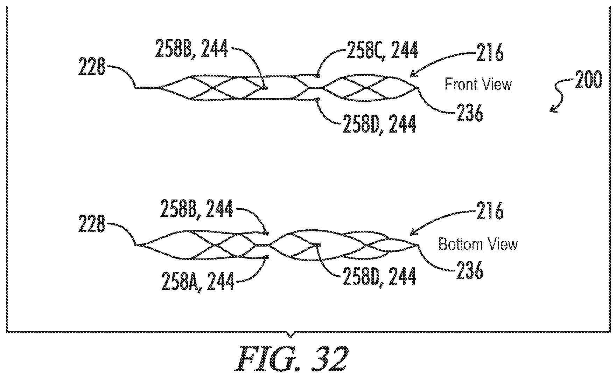

### The example shown in FIG. 32

FIG. 32 shown for reference only, shows a distal body **216** in which the proximal strips proximal ends **254** converge and are soldered or welded at the proximal hub/junction **228** and the basket strips **291** located at the distal end **220** of the basket **246** converge and are soldered or welded at the distal hub/junction **236.** To create such an example, the distal body **216** may be prepared from a single tube, as described above, and the proximal and distal tubes may be clipped and the proximal ends **254** of the proximal strips **252** soldered or welded together (and optionally to the pull wire **202**) and the basket strips **291** located at the distal end **220** of the basket **246** may also be welded or soldered or welded together. Optionally, the proximal and distal hubs/junctions **228** and **236** may include x-ray markers **244** as described above.

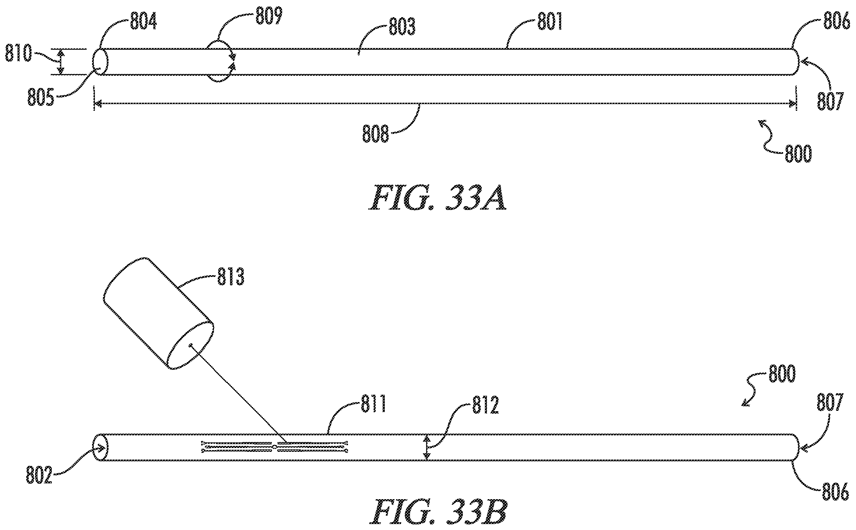

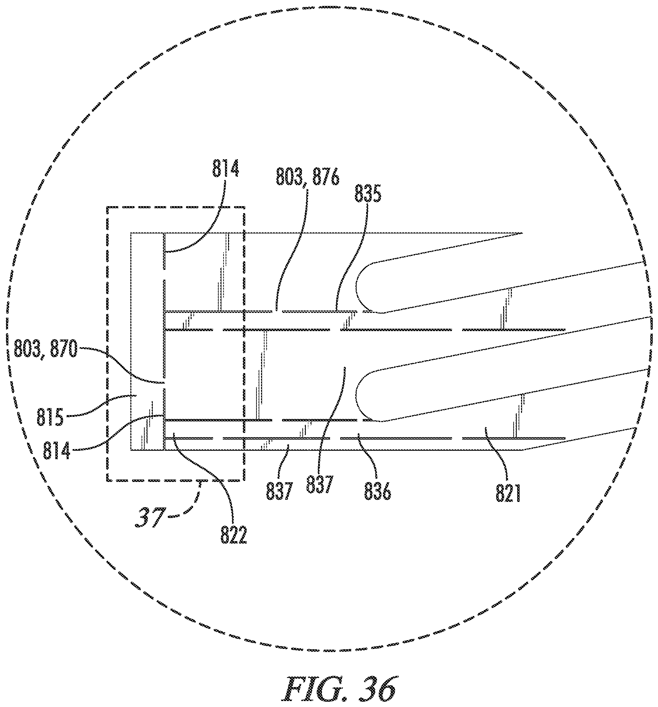

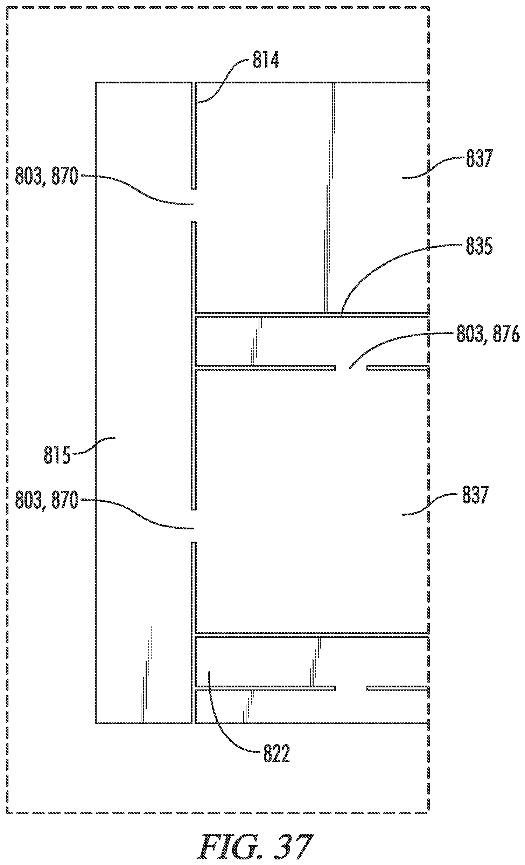

### The example shown in FIGs. 33A-49

During the development of the medical devices shown in FIGs. 11-20, it became apparent that it would be desirable to make devices from a single tube of memory metal (e.g., nitinol) that had a larger outer diameter than the inner diameter of the catheter. More particularly, it was desirable to create the baskets from a single tube having an outer diameter of 0.025 inches but deploy the baskets from a catheter having an inner diameter of 0.021 inches. This was not possible if the uncut proximal and distal ends of the tube were left intact in the device (as shown in FIG. 2 for example). Thus, a new method was developed to attain this objective, as shown in FIGs. 33-49. One method to achieve this was to create scoring lines (referred to below as perforations **814, 816, 835** and **838**) so that uncut excess material of first tube wall **803** would tend to tear cleanly and consistently along the scoring lines **814, 816, 835** and **838,** as described below.

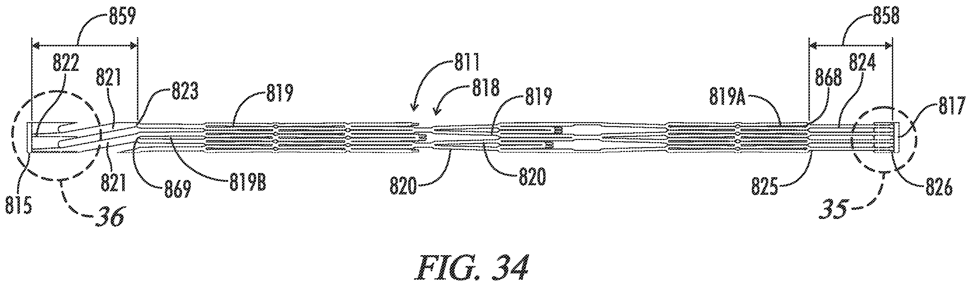

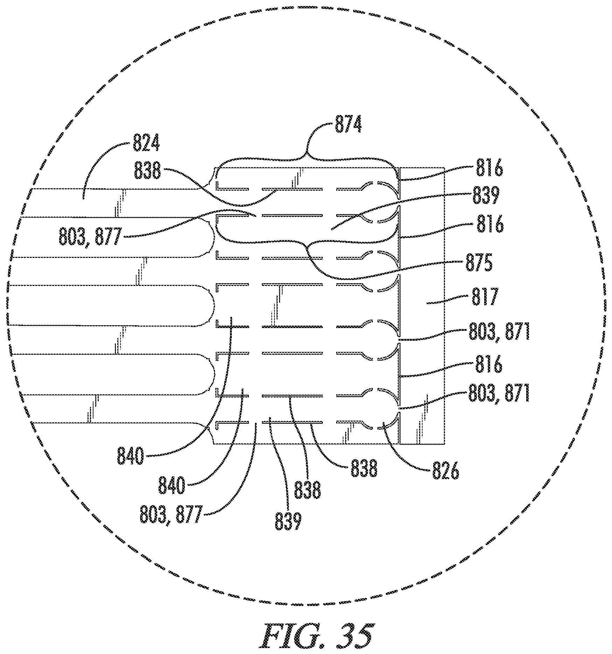

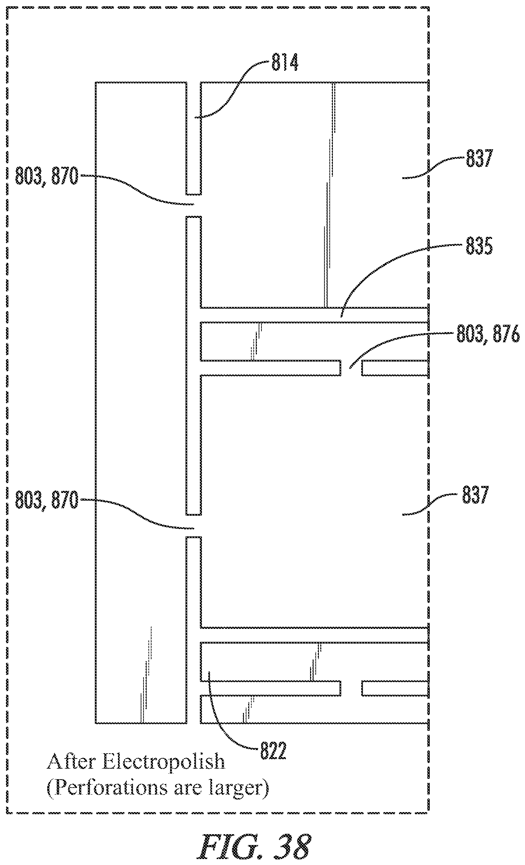

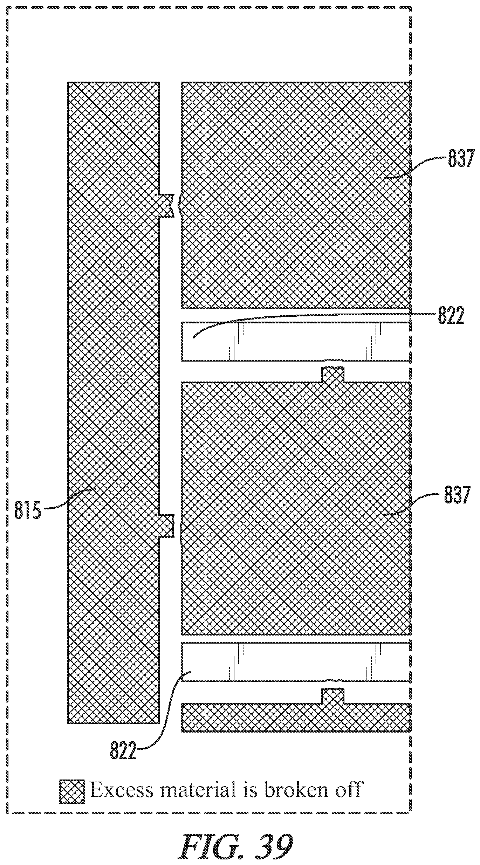

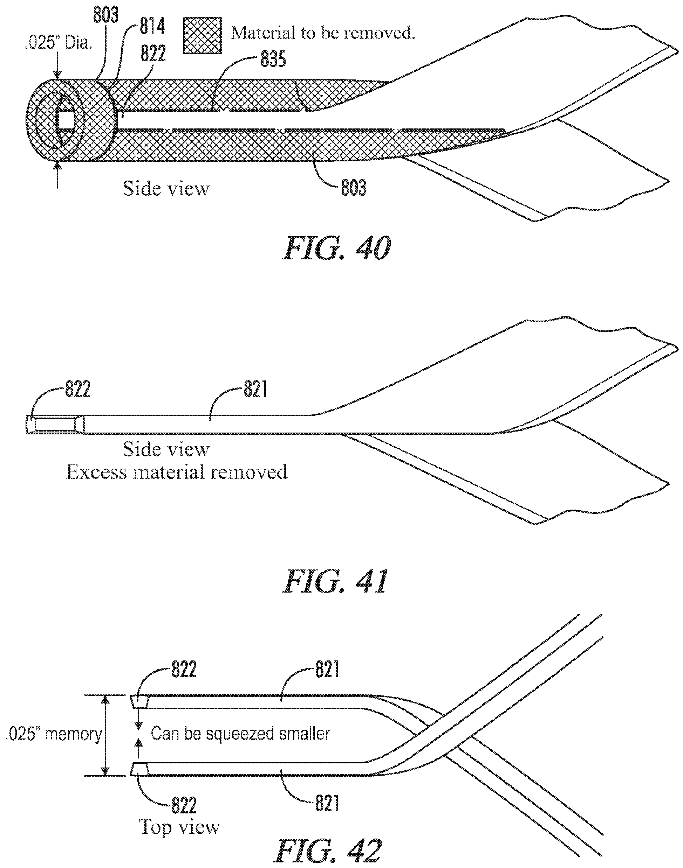

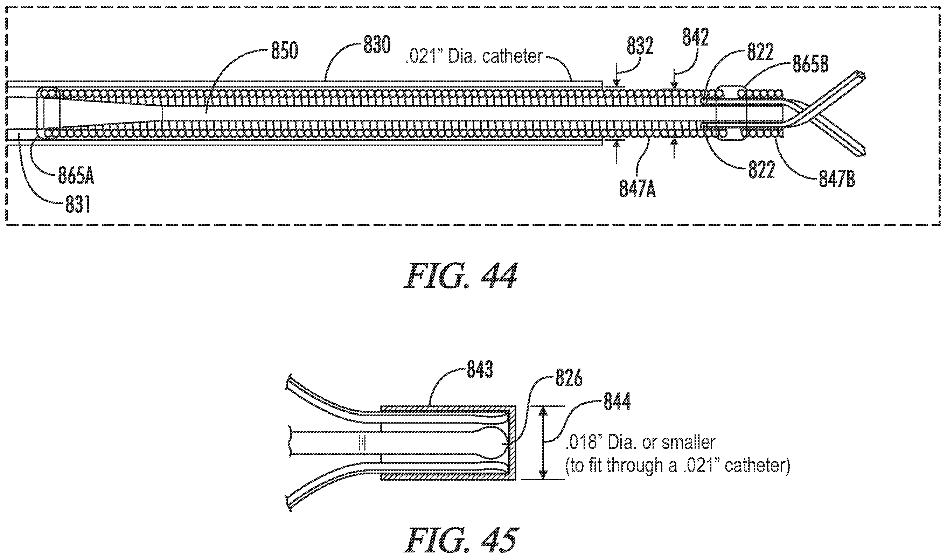

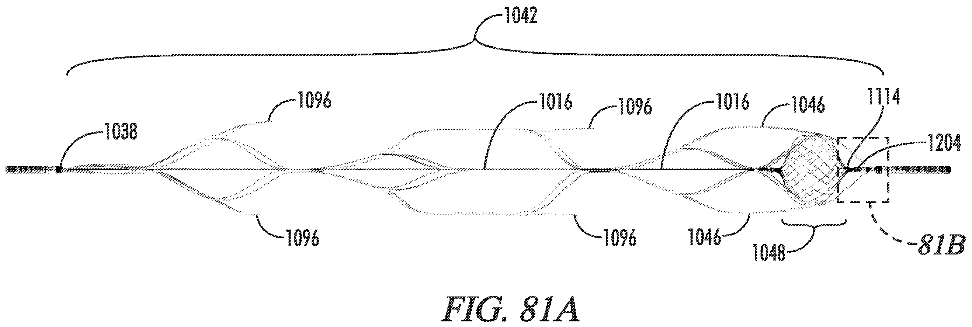

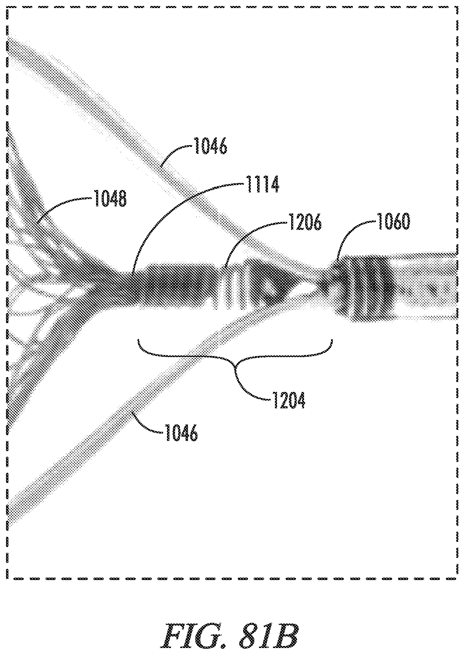

More particularly, as shown in FIGs. 33-49, the present disclosure provides for reference only: a method of manufacturing a medical device **827** comprising:
a) providing a first tube **800** comprised of a memory metal, the first tube **800** having a first tube exterior **801,** a first tube hollow interior **802,** a first tube wall **803** separating the first tube exterior **801** from the first tube hollow interior **802,** a first tube proximal end **804** comprising a first tube proximal aperture **805** leading to the first tube hollow interior **802,** a first tube distal end **806** comprising a first tube distal aperture **807** leading to the first tube hollow interior **802,** a first tube length **808** extending from the first tube proximal end **804** to the first tube distal end **806,** a first tube perimeter **809** (more particularly a circumference if first tube **800** is generally cylindrical) generally perpendicular to the first tube length **808,** a first tube width **810** (more particularly an outer diameter if first tube **800** is generally cylindrical) generally perpendicular to the first tube length **808,** and a middle portion **811** between the first tube proximal end **804** and the first tube distal end **806,** the middle portion **811** having a middle portion width **812** (more particularly an outer diameter if first tube **800** is generally cylindrical) generally parallel to the first tube width/diameter **810** (see FIG. 33A) (preferably the first tube width **810** is uniform along the first tube length **808** in step a) as shown in FIG. 33A);
b) using a cutting instrument **813** (e.g. a laser) to cut portions of the wall **803** of the first tube **800** (see FIG. 33B) and form i) a plurality of non-contiguous proximal perimeter perforations **814** located adjacent to the first tube proximal end **804** and spaced about the perimeter/circumference **809** of the first tube **800** and each proximal perimeter perforation **814** is separated by a proximal perimeter gap **870** (representing uncut portions of the wall **803**), the plurality of non-contiguous proximal perimeter perforations **814** and proximal perimeter gap **870** define a proximal end tab **815** located at the proximal end **804** of the first tube **800** (see FIGs. 34, 36, 37 and 40); ii) a plurality of non-contiguous distal perimeter perforations **816** located adjacent to the first tube distal end **806** and spaced about the perimeter/circumference **809** of the first tube **800** and each distal perimeter perforation **816** is separated by a distal perimeter gap **871** (representing uncut portions of the wall **803**), the plurality of non-contiguous distal perimeter perforations **816** and the distal perimeter gaps **871** defining a distal end tab **817** located at the distal end **806** of the first tube **800** (see FIGs. 34 and 35); iii) a matrix **818** in the middle portion **811** comprising a plurality of middle portion memory metal strips **820** forming a plurality of cells **819** (see FIG. 34); iv) a plurality of proximal memory metal strips **821** connecting the middle portion **811** to the proximal end tab **815,** each proximal memory metal strip **821** having a proximal memory metal strip proximal end **822** connected to the proximal end tab **815,** a proximal memory metal strip distal end **823** connected to a cell **819** of the middle portion **811** and a proximal memory metal strip length **859** extending from the proximal memory metal strip proximal end **822** to the proximal memory metal strip distal end **823** (see FIG. 34, 36, 37 and 40); and v) a plurality of distal memory metal strips **824** connecting the middle portion **811** to the distal end tab **817,** each distal memory metal strip **824** having a distal memory metal strip distal end **826** connected to the distal end tab **817,** a distal memory metal strip proximal end **825** connected to a cell **819** of the middle portion **811,** and a distal memory metal strip length **858** extending from the distal memory metal strip proximal end **825** to the distal memory metal strip distal end **826,** wherein the proximal end tab **815** connects the proximal ends **822** of the proximal memory metal strips **821** and the distal end tab **817** connects the distal ends **826** of the distal memory metal strips **824** (see FIGs. 34 and 35);
c) shape setting at least the middle portion **811** (e.g., the middle portion **811** and at least a portion of the proximal memory metal strips **821** and distal memory metal strips **824**) to expand the width/diameter **812** of the middle portion **811** (preferably by expanding the middle portion **811** using a mandrel such as that shown in FIGs. 30 and 31 to form a basket **851**);
d) after step c), polishing (e.g. electropolishing) the first tube **800,** wherein said polishing expands the plurality of proximal perimeter perforations **814** about the first tube perimeter/circumference **809** and expands the plurality of the distal perimeter perforations **816** about the first tube perimeter/circumference **809** (see FIG. 38, which shows expanding the proximal perimeter perforations **814** so that adjacent proximal perimeter perforations **814** approach each other and the proximal perimeter gaps **870** becoming smaller; the distal perimeter perforations **816** expand in a similar manner);
e) tearing along the plurality of proximal perimeter perforations **814** to free the proximal ends **822** of the proximal memory metal strips **821** from the proximal end tab **815** and each other and tearing along the plurality of distal perimeter perforations **816** to free the distal ends **826** of the distal memory metal strips **824** from the distal end tab **817** and each other (see FIGs. 39 and 41, which shows removing of the proximal end tab **815;** the distal end tab is **817** removed in a similar manner);
f) joining the free distal ends **826** of the distal memory metal strips **824** (see FIG. 45) and joining the free proximal ends **822** of the proximal memory metal strips **821** (see FIGs. 42, 43E-43G and 44) to form a medical device **827** comprised of the joined distal ends **826** of the distal memory metal strips **824,** the joined proximal ends **822** of the proximal memory metal strips **821,** and the shape set middle portion **811,** the medical device **827** having a medical device length **828** extending at least from the joined distal ends **826** of the distal memory metal strips **824** to at least the joined proximal ends **822** of the proximal memory metal strips **821** and a medical device width **829** generally perpendicular to the medical device length **828** (the term "at least" refers to the fact that the medical device **827** may include a lead wire at the distal end as described previously); and
g) inserting the medical device **827** into a catheter **830** comprising a catheter interior **831** having an interior width **832** (more particularly an inner diameter if the catheter **830** is generally cylindrical), an open catheter proximal end (not shown in FIGs. 33-49 but shown as **212** in FIG. 21) leading to the catheter interior **831,** an open catheter distal end **833** leading to the catheter interior **831,** the catheter **830** comprised of a biocompatible material, wherein the catheter interior width **832** (more particularly inner diameter if the catheter **830** is generally cylindrical) is less than the first tube width/outer diameter **810,** wherein the medical device **827** comprises a collapsed state wherein the medical device width **829** is less than the catheter interior width/diameter **832** and an expanded state wherein the medical device width **829** is greater than the catheter interior width/diameter **832,** and further wherein the catheter **830** is configured to envelope the medical device **827** when the medical device **827** is in the collapsed state (see FIG. 81).

Optionally, the first tube **800** is generally cylindrical in shape and comprises a first tube diameter **810** and a first tube circumference **809** and the proximal perimeter perforations **816** are arranged in a generally straight line about the circumference **809** of the first tube **800** (see FIGs. 34, 36, 37, 40 and 46) and the distal perimeter perforations **816** are arranged in a generally straight line about the circumference **809** of the first tube **800** (see FIGs. 34-35).

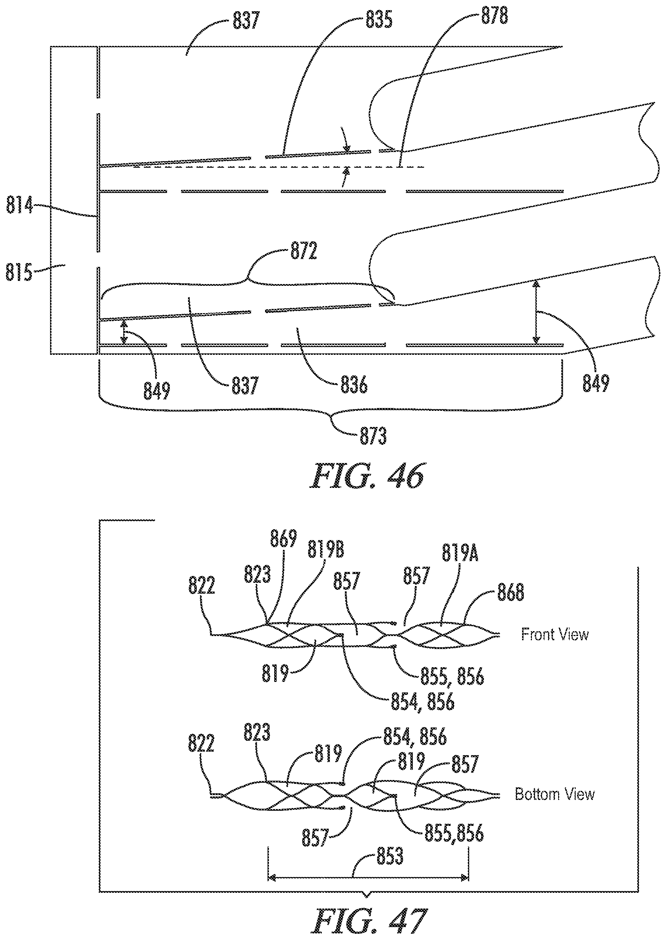

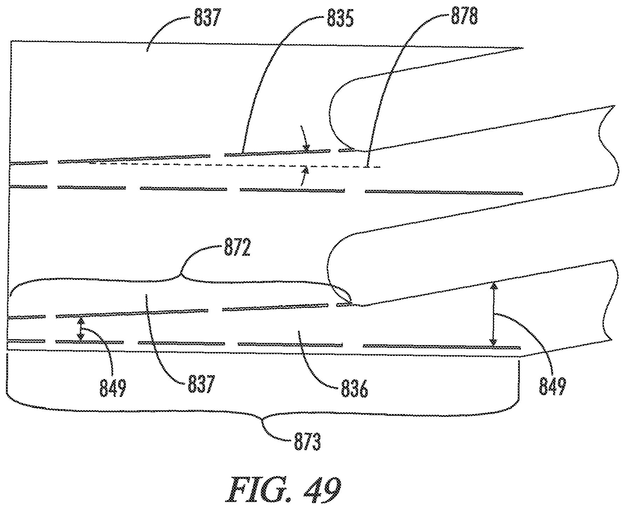

Optionally step b) further comprises using the cutting instrument **813** to cut additional portions of the wall **803** of the first tube **800** and form a plurality of non-contiguous proximal longitudinal perforations **835** located in a proximal segment **836** of each proximal memory metal strip **821** adjacent to the proximal end **822** of the respective proximal memory metal strip **821** and extending generally along the first tube length **808** (see FIGs. 34, 36, 37, 46 and 49). Each adjacent non-contiguous proximal longitudinal perforation **835** is separated by a proximal longitudinal gap **876** (representing uncut portions of the wall **803**). The proximal longitudinal perforations **835** and the proximal longitudinal gaps **876** form a first longitudinal side **872** and a second longitudinal side **873** of each proximal segment **836.** It will be understood that the non-contiguous proximal longitudinal perforations **835** extend generally along the first tube length **808** but are not necessarily parallel to the first tube length **808** as shown in FIGs. 46 and 49 as indicated by reference line **878;** the reference line **878** is not a component of the system but is merely drawn in the illustration to show the angle. A proximal longitudinal tab **837** is located between and connects adjacent proximal segments **836** of proximal memory metal strips **821** and is formed of uncut portions of the wall **803.**

Optionally step b) further comprises using the cutting instrument **813** to cut additional portions of the wall **803** of the first tube **800** and form a plurality of non-contiguous distal longitudinal perforations **838** located in a distal segment **839** of each distal memory metal strip **824** adjacent to the distal end **826** of the respective distal memory metal strip **824** and extending generally along the first tube length **808** (see FIGs. 34 and 35). Each adjacent non-contiguous distal longitudinal perforation **838** is separated by a distal longitudinal gap **877** (representing uncut portions of the wall **803**). The distal longitudinal perforations **838** and the distal longitudinal gaps **877** form a first longitudinal side **874** and a second longitudinal side **875** of each distal segment **839.** It will be understood that the non-contiguous distal longitudinal perforations **838** extend generally along the first tube length **808** but are not necessarily parallel to the first tube length **808** as best seen in FIGs. 35. A distal longitudinal tab **840** is located between and connects adjacent distal segments **839** of distal memory metal strips **824** and is formed of uncut portions of the wall **803.**

Preferably, the polishing expands the plurality of proximal longitudinal perforations **835** about the first tube length **808** (see FIG. 38) and expands the plurality of the distal longitudinal perforations **838** about the first tube length **808** (so that adjacent proximal longitudinal perforations **835** on the first longitudinal side **872** of the proximal segment **836** approach each other, so that adjacent proximal longitudinal perforations **835** on the second longitudinal side **873** of the proximal segment **836** approach each other, so that adjacent distal longitudinal perforations **838** on the first longitudinal side **874** of the distal segment **839** approach each other, and so that adjacent distal longitudinal perforations **838** on the second longitudinal side **875** of the distal segment **839** approach each other), and step e) further comprises tearing along the plurality of proximal longitudinal perforations **835** to remove the proximal longitudinal tabs **837** (see FIGs. 39 and 41) and disconnect the proximal segments **836** from each other and tearing along the plurality of distal longitudinal perforations **838** to remove the distal longitudinal tabs **840** and disconnect the distal segments **839** from each other.

Optionally, after step d), the plurality of proximal longitudinal perforations **835** become nearly continuous (see FIGs. 39 and 41), the plurality of distal longitudinal perforations **838** become nearly continuous, the plurality of proximal perimeter perforations **814** become nearly continuous (see FIGs. 39 and 41) and the plurality of distal perimeter perforations **816** become nearly continuous.

Optionally, the first tube **800** is generally cylindrical in shape and comprises a first tube outer diameter **810,** wherein said catheter **830** is generally cylindrical in shape and comprises a catheter inner diameter **832** (interior diameter), wherein said step of joining the free proximal ends **822** of the proximal memory metal strips **821** comprises attaching the free proximal ends **822** of the proximal memory metal strips **821** to a second tube **841,** the second tube **841** generally cylindrical in shape and comprising a second tube outer diameter **842,** wherein said step of joining the free distal ends **826** of the distal memory metal strips **824** comprises attaching the free distal ends **826** of the distal memory metal strips **824** to a third tube **843,** the third tube **843** generally cylindrical in shape and comprising a third tube outer diameter **844,** and further wherein said second tube outer diameter **842** and said third tube outer diameter **844** are less than said first tube outer diameter **810** and less than said catheter inner diameter **832** (see FIGs. 44 and 45).

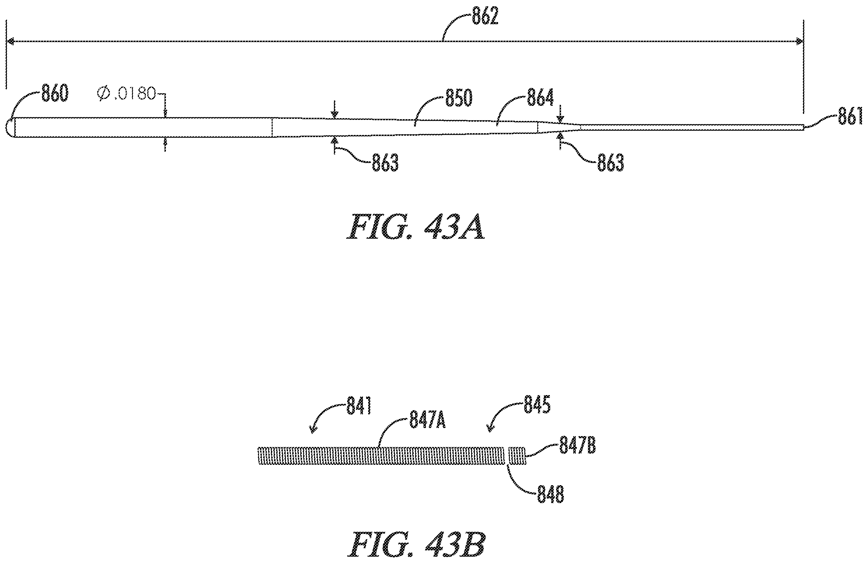

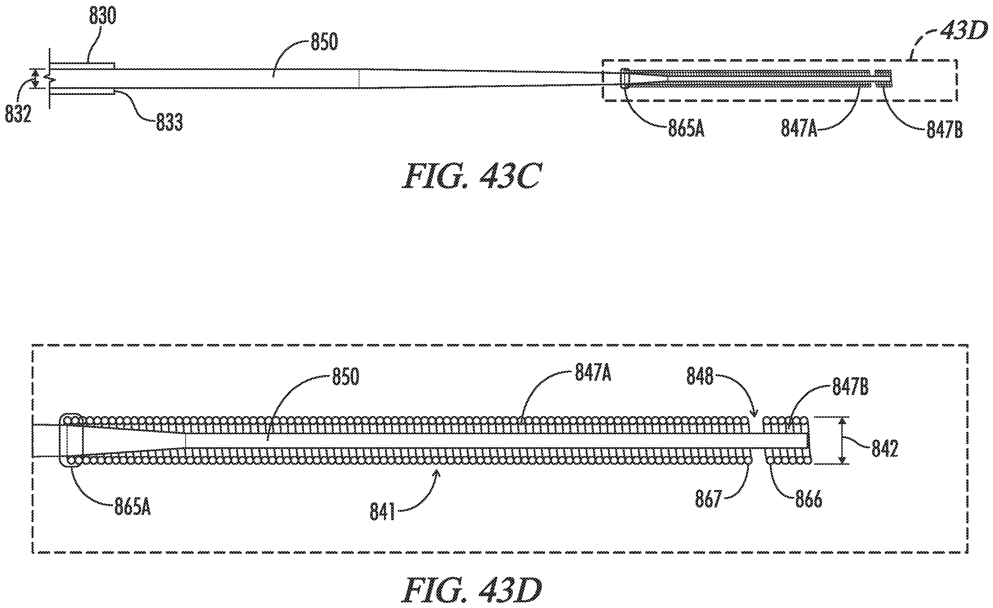

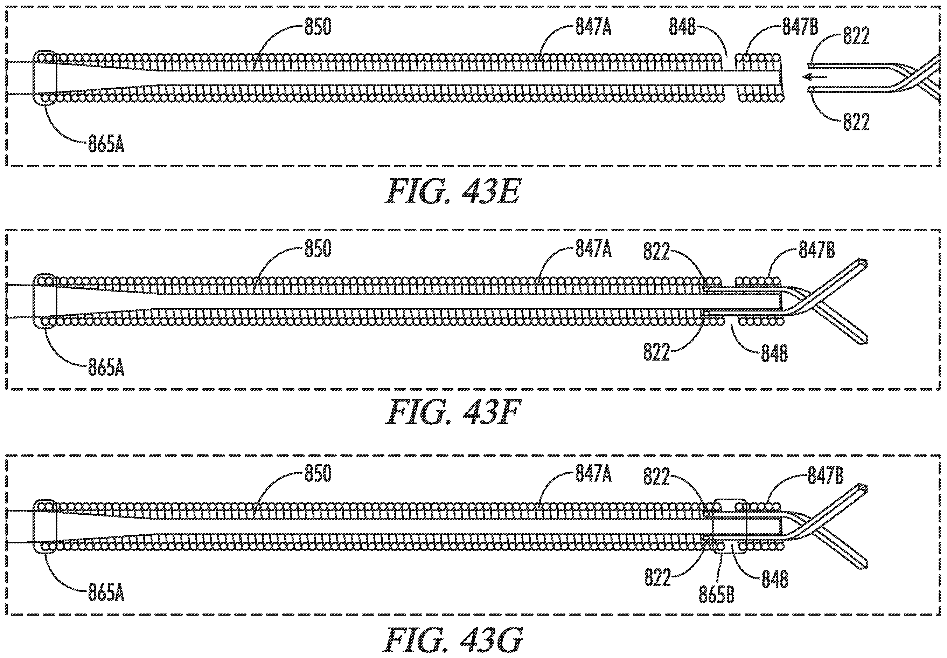

FIGs. 43A-43G illustrate an example where the second tube **841** is a coil system **845.** For example, the method may include providing a pull wire **850.** (See FIG. 43A). The next step may be providing a coil system **845** that includes a proximal coil **847A** and a distal coil **847B** separated by a longitudinal space **848** between the proximal end **866** of the distal coil **847B** and the distal end **867** of the proximal coil **847A.** (See FIG. 43B). The next step may involve soldering the pull wire **850** to the proximal coil **847A** so that the pull wire **850** is surrounded by the proximal coil **847A.** (See FIGs.43C and 43D; soldering denoted by the numeral **865A**). The next step may involve joining the proximal ends **822** of the proximal memory metal strips **821** by soldering the proximal ends **822** of the proximal memory metal strips **821** at the longitudinal space **848** between the coils **847A** and **847B.** (See FIGs. 43E-43G; soldering is denoted by the numeral **865B**). As shown in FIG. 43F, the proximal memory metal strips **821** are located between the pull wire **850** (which forms a core of the coil system **845**) and the proximal coil **847A.** Optionally, the pull wire **850** comprises a pull wire proximal end **860,** a pull wire distal end **861,** a pull wire length **862** extending from the pull wire proximal end **860** to the pull wire distal end **861** and a pull wire width **863** generally perpendicular to the pull wire length **862** and further wherein said pull wire width **863** comprises a segment **864** in which the pull wire width **863** tapers proximally along the pull wire length **862.** (See FIG. 43A).

Optionally, the proximal memory metal strips **821** comprise a width **849** generally perpendicular to the first tube length **808** and further wherein said widths **849** of said proximal memory metal strips **821** taper as the proximal memory metal strips **821** approach the proximal end tab **815** (see FIG. 46 and FIG. 49).

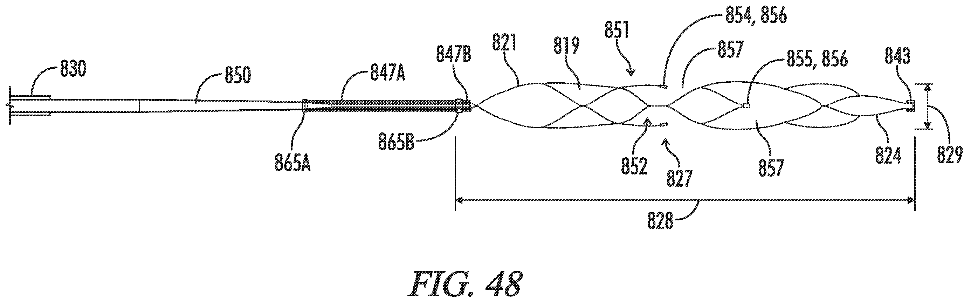

The middle portion **811** may be shape-set in any form. Preferably, the middle portion **811** is shape set in the form of a basket **851,** as described above, that is configured to capture a foreign object in a lumen of an animal such as an intracranial thrombus. For example, optionally the middle portion memory metal strips **820** of said shape set middle portion **811** form a basket **851** comprising a basket interior **852** and a basket length **853** generally parallel to the medical device length **828.** Optionally, in the expanded state, the basket **851** comprises a first pair of distal crowns **854** not attached to another cell **819** of the basket **851** and pointing generally in the distal direction, the distal crowns **854** in the first pair of distal crowns **854** located approximately the same distance along the basket length **853** and between 150 degrees and 180 degrees relative to each other, and further wherein the basket **851** further comprises a second pair of distal crowns **855** not attached to another cell **819** of the basket **851** and pointing generally in the distal direction, the second pair of distal crowns **855** located distally relative to the first pair of distal crowns **854,** each of the distal crowns in the second pair of distal crowns **855** located between 60 degrees and 90 degrees relative to a distal crown in the first pair of distal crowns **854,** the distal crowns in the second pair of distal crowns **855** located approximately the same distance along the basket length **853** and further wherein each of the distal crowns in the first and second pair of distal crowns **854** and **855** comprises an x-ray marker **856,** the x-ray maker **856** more visible under x-ray as compared to the middle portion strips **820** when the basket **851** is located in a cranial blood vessel inside the body of a human and the x-ray is taken from outside the human's body and further wherein each distal crown in the first and second pair of distal crowns **854** and **855** forms part of a cell **819.** Optionally, each distal crown in the first and second pair of distal crowns **854** and **855** forms part of an enlarged cell **857** and further wherein the surface area of the enlarged cells **857** in the relaxed state is greater than the surface area of the other cells **819** of the basket **811** and further wherein the enlarged cells **857** are configured to allow a thrombus to pass therethrough and into the basket interior **852,** and further wherein the basket **811** comprises a non-uniform outward radial force along the basket length **853** due to the offset enlarged cells **857.** (See FIG. 47). Optionally, in step b), each distal end **823** of each proximal memory metal strip **821** is connected to a proximal crown **869** of a proximal cell **819B** of the middle portion **811,** said proximal crown **869** of said proximal cell **819B** located at the proximal end of the basket **811** and pointing generally in the proximal direction, and each proximal end **825** of each distal memory metal strip **824** is connected to a distal crown **868** of a distal cell **819A,** each distal crown **868** pointing generally in the distal direction and located at the distal end of the basket **811** (see FIGs. 34 and 47). In other words, in the example the middle portion **811** preferably forms a basket **851** as described with the basket example shown in FIGs. 11-20. However, other basket designs are also possible. Preferably, in the medical device **827,** the middle portion width/diameter **812** in the expanded state tapers as the proximal memory metal strips **821** approach the second tube **841** and as the distal memory metal strips **824** approach the third tube **843.** (See FIG. 48). (Preferably, the proximal memory metal strips **821** twist as shown in FIGs. 40-42, 44 and 47-48 and as described above with respect to FIGs. 11 and 20 for example - i.e., each distal end **823** of the respective proximal memory metal strip **821** is 180 degrees offset from the proximal end **822** of the same respective proximal memory metal strip **821**).

Optionally, in the expanded state, the medical device width **829** is less than the medical device length **828.** Optionally, said catheter inner diameter **832** is at least about 0.001 inches (e.g, between 0.001 and 0.015 inches, preferably between 0.003 and 0.015 inches) less than said first tube outer diameter **810.** The medical device **827** may further include a lead wire at the distal end as described previously.

After step e), the proximal end tab **815,** the distal end tab **817,** the proximal longitudinal tabs **837** and the distal longitudinal tabs **840** are discarded.

Optionally, after step e), the proximal memory metal strips **821** comprise a smooth periphery and the distal memory metal strips **824** comprise a smooth periphery. In other words, preferably, the proximal end tabs **815** tear cleanly along the proximal perimeter perforations **814,** the distal end tabs **817** tear cleanly along the distal perimeter perforations **816,** the proximal longitudinal tabs **837** tear cleanly along the proximal longitudinal perforations **835** and the distal longitudinal tabs **840** tear cleanly along the distal longitudinal perforations **838.**

The steps of the method described above with reference to FIGs. 33-49 may be performed simultaneously or in any suitable order. In addition, one or more of the steps, such as step d) may be omitted. Further, step c) (expanding the middle portion **811**) may be performed using methods now known or hitherto developed. Moreover, the first tube **800** may only include proximal perimeter perforations **814,** proximal longitudinal perforations **835,** distal perimeter perforations **816** and/or distal longitudinal perforations **838.** In other words, the first tube **800** may be cut to include only perimeter perforations **814** and/or **816** or only longitudinal perforations **835** and/or **838** as shown in FIG. 49 which only includes proximal longitudinal perforations **835** that extend to the proximal end **804** of the first tube **800**). Preferably, the first tube **800** is cut to include at least proximal longitudinal perforations **835** and distal longitudinal perforations **838.**

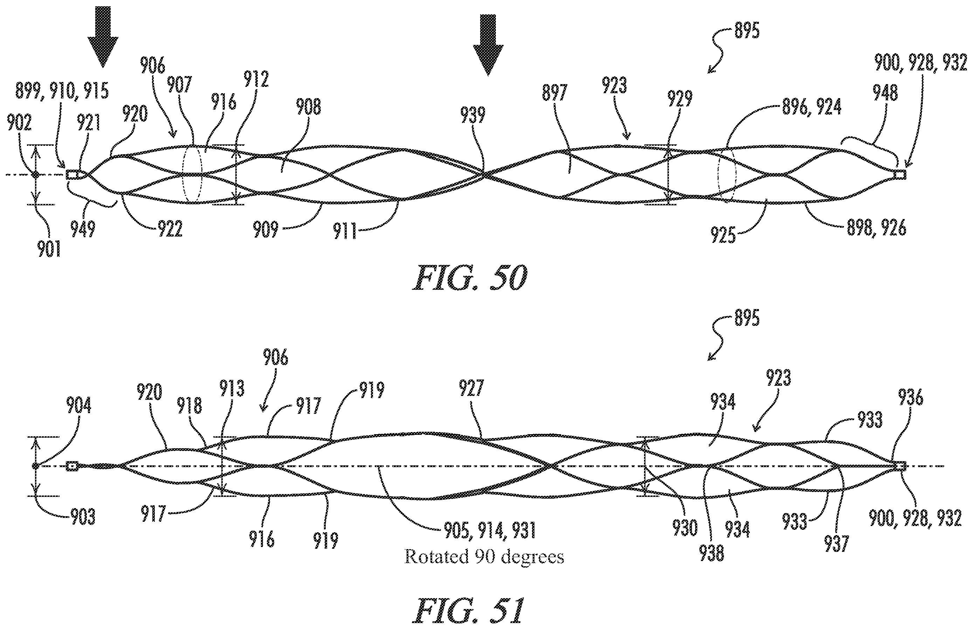

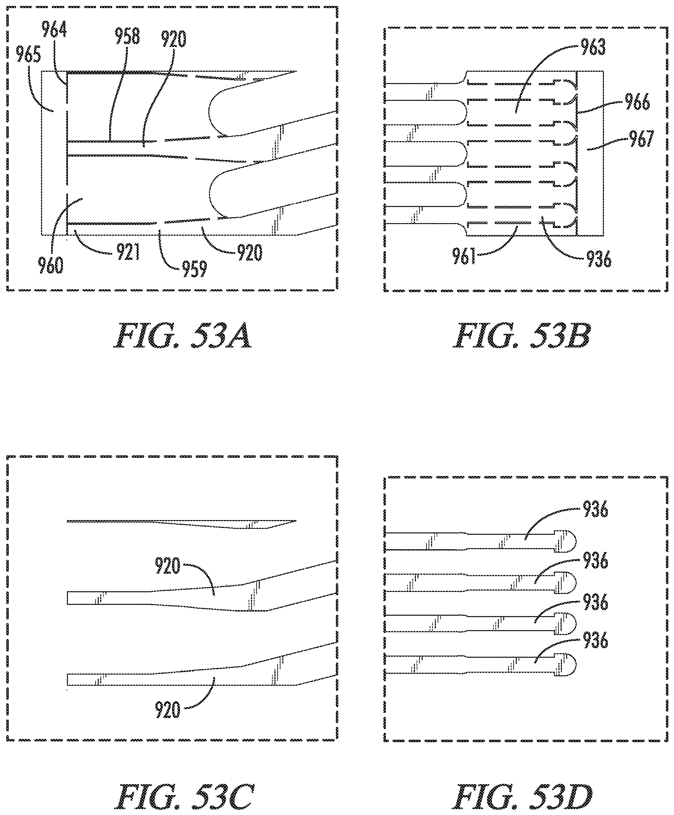

### The examples shown in FIGs. 50-56

FIGs. 50-56 illustrate another catheter-delivered endovascular device. The catheter-delivered endovascular device **890** of FIGs. 50-56 may be used to retrieve a clot or other foreign object from a lumen of an animal. In addition, the catheter-delivered endovascular device **890** of FIGs. 50-56 may be used to open a constricted blood vessel **950** in the case of a subarrachnoid hemorrhage induced vasospasm or other vasospasm.

The catheter-delivered endovascular device **890** of FIGS. 50-56 includes a pull wire **891** having a proximal end, a distal end **892** and a pull wire longitudinal axis **894** extending from the proximal end to the distal end **892.** The pull wire **891** may have one or more features described above with respect to the systems of FIGs. 1-49, and may be comprised of a biocompatible metallic material for example.

Optionally, the catheter-delivered endovascular device **890** further includes a deployable dual basket system **895** attached to the pull wire **891** and comprising a system perimeter/circumference **896** separating a system interior **897** from a system exterior **898,** a system proximal end **899,** a system distal end **900,** a system height **901** having a system height center **902,** a system width **903** perpendicular to the system height **901** and having a system width center **904,** a system longitudinal axis **905** from the system proximal end **899** to the system distal end **900** and extending through the system height center **902** and system width center **904.** The system height **901** and width **903** may vary along the system longitudinal axis **905,** as seen in FIGs. 50-51, e.g., a smaller height and width at the proximal end **899,** the distal end **900,** and the middle of the system as seen in FIGs. 50-51. The system **895** is preferably generally in the form of a tapered cylinder with a variable diameter constituting the system height **901** and system width **903,** and accordingly, the system perimeter **896** is preferably a system circumference.

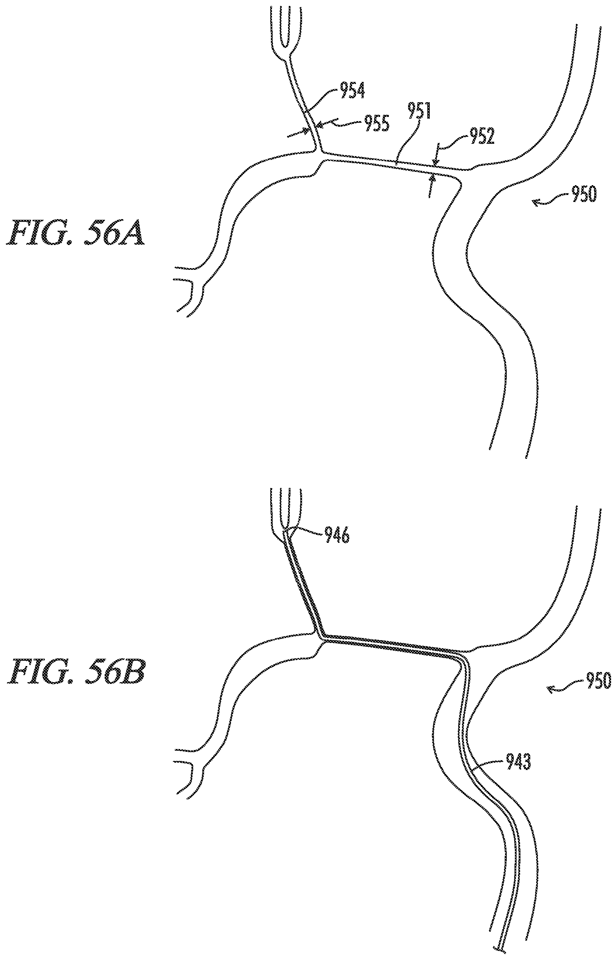

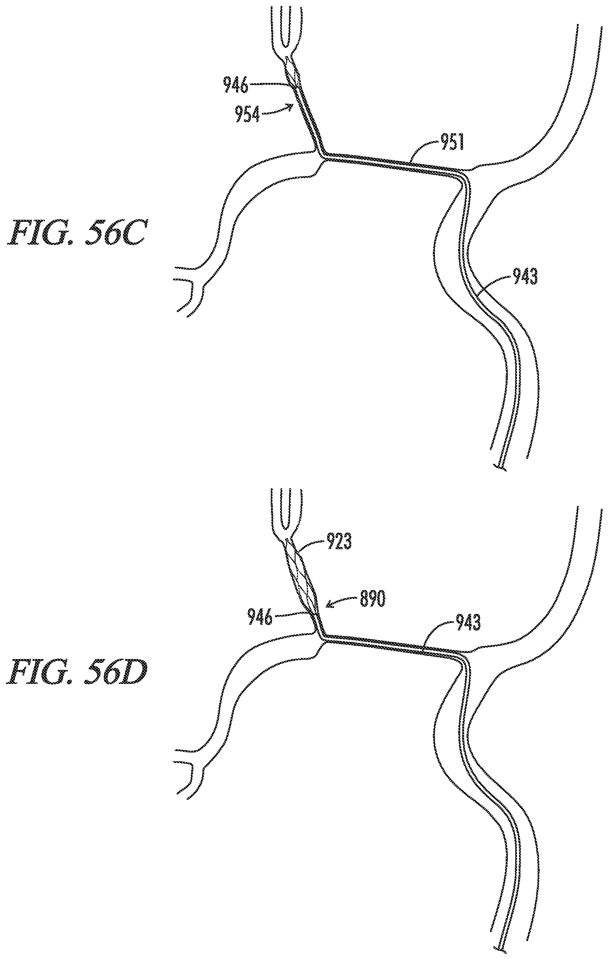

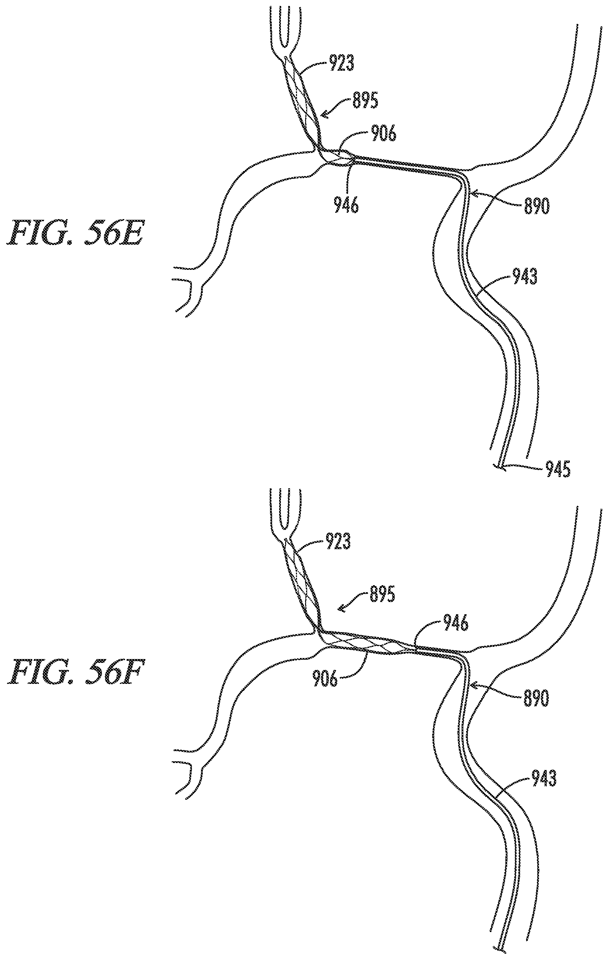

Optionally, the deployable dual basket system **895** includes a proximal basket 906 attached to the pull wire **891,** the proximal basket **906** comprising a proximal basket perimeter/circumference **907** separating a proximal basket interior **908** from a proximal basket exterior **909,** a proximal end **910** forming the system proximal end **899,** a distal end **911,** a proximal basket height **912** generally parallel to the system height **901,** a proximal basket width **913** generally parallel to the system width **903** and perpendicular to the proximal basket height **912,** a proximal basket longitudinal axis **914** extending from the proximal basket proximal end **910** to the distal end **911** and generally parallel to the system longitudinal axis **905** and generally perpendicular to the proximal basket height **912** and proximal basket width **913,** a proximal junction **915** located at the proximal end **910** of the proximal basket **906,** a plurality of proximal cells **916** distal to the proximal junction **915** and defined by a plurality of proximal basket memory metal strips **917,** each proximal cell **916** comprising a proximal crown **918** located at the proximal end of the proximal cell **916** and pointing generally in the proximal direction and a distal crown **919** located at the distal end of the proximal cell **916** and pointing generally in the distal direction, a plurality of proximal tether memory metal strips **920** located between the proximal junction **915** and the proximal cells **916** and connecting the proximal cells **916** to the proximal junction **915,** each proximal tether memory metal strip **920** having a proximal end **921** attached to the proximal junction **915,** a distal end **922** attached to a proximal crown **918** of a proximal cell **916.** Due to the fact that the proximal basket **906** is preferably formed from a memory metal tube, as with the prior examples, the proximal basket **906** preferably has a relaxed/expanded state (as shown in FIGs. 50, 51, 56F, 56G, and 56H) wherein the proximal basket **906** has a first height **912** and a first width **913,** and a collapsed state (see FIG. 56B, 56C and 56D, in which the proximal basket **906** is in the catheter interior **944**) wherein the proximal basket **906** has a second height and a second width, the second height less than the first height **912** and the second width less than the first width **913.** (FIGs. 56E shows the distal end **911** of the proximal basket **906** in the relaxed state and the proximal end **910** (which is not clearly visible) is in the collapsed state.

Optionally, the deployable dual basket system **895** further includes: a distal basket **923** distal to the proximal basket **906** and comprising a distal basket circumference **924** separating a distal basket interior **925** from a distal basket exterior **926,** a proximal end **927,** a distal end **928** forming the system distal end **900,** a distal basket height **929** generally parallel to the system height **901,** a distal basket width **930** generally parallel to the system width **903** and generally perpendicular to the distal basket height **929,** a distal basket longitudinal axis **931** extending from the distal basket proximal end **927** to the distal basket distal end **928** and generally parallel to the system longitudinal axis **905,** a distal junction **932** located at the distal end **928** of the distal basket **923,** a plurality of distal cells **934** proximal to the distal junction **932** and defined by a plurality of distal basket memory metal strips **933,** each distal cell **934** comprising a proximal crown **938** located at the proximal end of the distal cell **934** and pointing generally in the proximal direction and a distal crown **937** located at the distal end of the distal cell **934** and pointing generally in the distal direction. Due to the fact that the distal basket **923** is preferably formed from a memory metal tube, as with the prior examples, the distal basket **923** preferably has a relaxed/expanded state (as shown in FIGs. 50, 51, and 56E-56H) wherein the distal basket **923** has a first height **929** and a first width **930,** and a collapsed state (see FIG. 56B in which the distal basket **923** is in the catheter interior **944**) wherein the distal basket **923** has a second height and a second width, the second height less than the first height **929** and the second width less than the first width **930.** (FIGs. 56C shows the distal end **928** of the distal basket **923** in the expanded state and the proximal end **927** (which is in the catheter interior **944**) is in the collapsed state).

Optionally, the deployable dual basket system **895** further includes a plurality of basket connector tether memory metal strips **939** located between the proximal basket **906** and the distal basket **923** and connecting the proximal basket **906** to the distal basket **923** and located between the proximal basket **906** and the distal basket **923.** Optionally, each basket connector tether memory metal strip **939** has a proximal end **940** attached to a distal crown **919** of a cell **916** located at the distal end of the proximal basket **906** and a distal end **941** attached to a proximal crown **938** of a cell **934** located at the proximal end of the distal basket **923,** and a basket connector tether memory metal strip longitudinal axis extending from the proximal end **940** of the basket connector tether memory metal strip **939** to the distal end **941** of the basket connector tether memory metal strip **939.**

As previously mentioned, the catheter-delivered endovascular device **890** further includes a catheter **943** having an interior **944,** a proximal end **945** leading to the interior **944** and a distal end **946** leading to the interior **944,** the catheter **943** comprised of a biocompatible material and configured to envelope the deployable dual basket system **895** when the proximal basket **906** and distal basket **923** are in the collapsed state. The catheter **943** may have one or more features described above with respect to the catheters of the systems shown in FIGs. 1-49 and may be polymeric as described above.

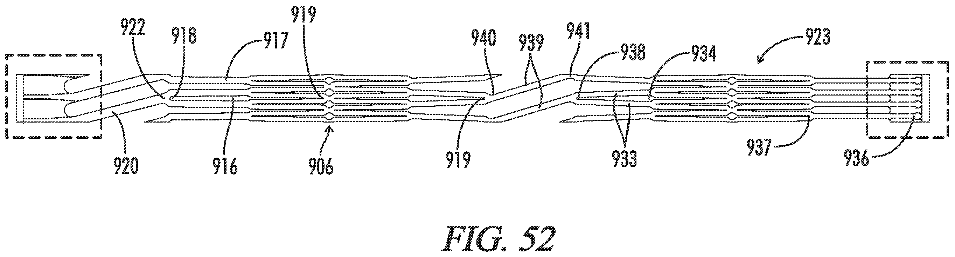

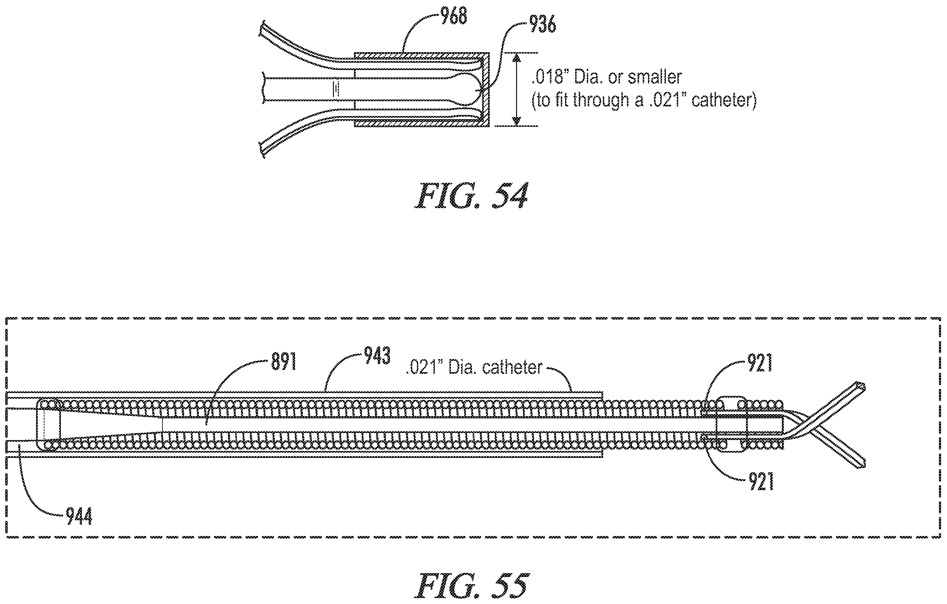

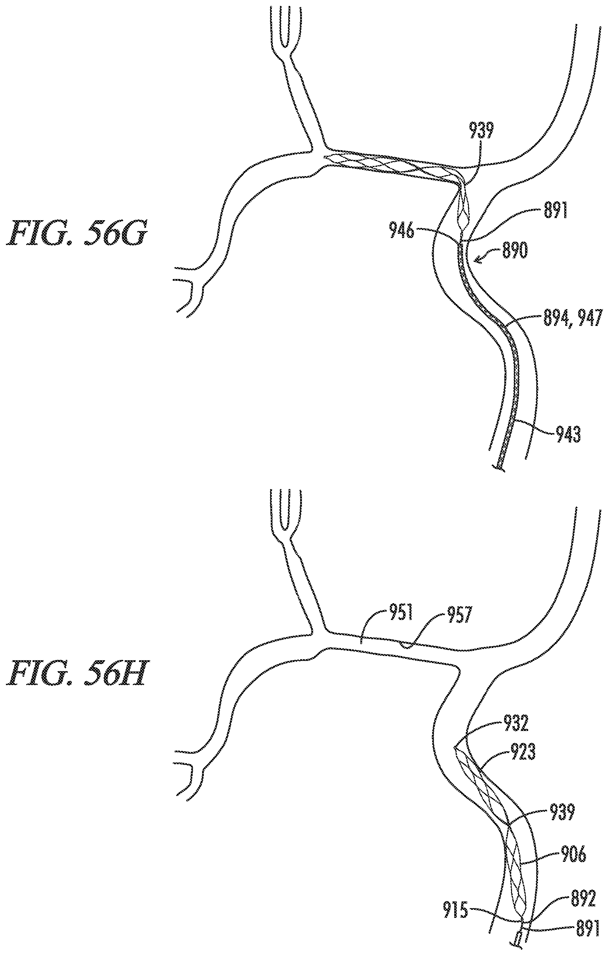

Optionally, in the relaxed state and the collapsed state, each basket connector tether memory metal strip **939** rotates a degree of rotation about the system circumference **896** relative to the proximal basket longitudinal axis **914,** the distal basket longitudinal axis **931** and the system longitudinal axis **905.** Optionally, each basket connector tether memory metal strip **939** rotates in the same direction; for example, if the deployable dual basket system **895** has two basket connector tether memory metal strips **939** both will rotate clockwise or both will rotate counterclockwise as viewed from the system proximal end 899. The reason that the basket connector tether memory metal strips **939** both preferably rotate in the same direction is that the deployable dual basket system **895** is preferably initially made from a single memory metal tube using the cut pattern for the basket connector tether memory metal strips **939** shown in FIG. 52 (the memory metal tube is shown flat in FIG. 52 for illustration purposes). As discussed below, after cutting the tube and removing the proximal end of the tube and the distal end of the tube, the proximal tether memory metal strips **920** may be re-joined as shown in FIG. 55 using coil and the distal basket memory metal strips distal ends **936** may be rejoined using third tube **968** as shown in FIG. 54. The rotating basket connector tether memory metal strips **939** preferably provide a flex point so that the deployable dual basket system **895** may navigate tortuous blood vessels **950,** as shown in FIG. 56. It will be understood that the rotation is a characteristic of the connector tether memory metal strips **939** and does not refer to user manipulation of the connector tether memory metal strips **939** - i.e., the connector tether memory metal strips **939** rotate without user manipulation.

Optionally, each basket connector tether memory metal strip **939** rotates a greater degree of rotation in the collapsed state as compared to the degree of rotation of the same basket connector tether memory metal strip **939** in the relaxed state if the basket connector tether memory metal strips **939** are prepared from a single memory metal tube that is expanded and shape set. The reason for this is that the collapsed state mimics the native portion and has the diameter of the tube from which the deployable dual basket system **895** is cut, whereas the relaxed state has a greater diameter, and accordingly, the basket connector tether memory metal strips **939** must travel a greater distance in the relaxed state. Thus, for example, a given basket connector tether memory metal strip **939** may rotate 180 degrees for example in the collapsed state but only 90 degrees in the relaxed state. Optionally, in the relaxed state, the basket connector tether memory metal strips **939** each rotate at least about fifteen degrees in the same direction relative to the proximal basket longitudinal axis **914** and the distal basket longitudinal axis **931.** In the collapsed state, the distal end **941** of a first basket connector tether memory metal strip **939** is located between about 90 degrees and about 270 degrees relative to the proximal end **940** of the same basket connector tether memory metal strip **939,** and further wherein in the collapsed state, the distal end **941** of a second basket connector tether memory metal strip **939** is located between about 90 degrees and about 270 degrees relative to the proximal end **940** of the same basket connector tether memory metal strip **939.**

Due to the fact that the basket connector tether memory metal strips **939** rotate, in the relaxed state and the collapsed state, a distal crown **919** of the proximal basket **906** attached to the proximal end **940** of a basket connector tether memory metal strip **939** is offset about the system circumference **896** relative to the proximal crown **938** of the distal basket **923** attached to the distal end **941** of the same basket connector tether memory metal strip **939,** and accordingly, the distal crown **919** of the proximal basket **906** will rotate a greater extent in the collapsed state as compared to the relaxed state.

Optionally, at least some of the distal basket memory metal strips **933** are located at the distal end **928** of the distal basket **923,** wherein each of the distal basket memory metal strips **933** located at the distal end **928** of the distal basket **923** have a distal end **936,** wherein each of the distal ends **936** of the distal basket memory metal strips **933** located at the distal end **928** of the distal basket **923** converge at the distal junction **932** and further wherein the distal basket **923,** in the relaxed state, comprises a tapered region **948** in which the distal basket height **929** and width **930** decrease as the distal basket memory metal strips **933** located at the distal end **928** of the distal basket **923** approach the distal junction **932.** Likewise, optionally, the proximal basket **906,** in the relaxed state, comprises a tapered region **949** in which the proximal basket height **912** and width **913** decrease as the proximal tether memory metal strips **920** approach the proximal junction **915.** In other words, the proximal tapered region **949** represents a low point in the proximal basket width **913** and height **912** and the distal tapered region **948** represents a low point in the distal basket width **930** and height **929,** which prevents the device **890** from injuring a blood vessel **950** when used to treat vasospasm, as shown in FIGs. 56A-56H for example.

Optionally, in the relaxed state, the radial force of the deployable dual basket system **895** from the proximal ends **940** of the basket connector tether memory metal strips **939** to the distal ends **941** of the basket connector tether memory metal strips **939** is less than the radial force of the proximal basket **906,** as measured from the proximal crowns **918** of the cells **916** of the proximal basket **906** attached to the plurality of proximal memory metal strips **920** to the distal crowns **919** of the cells **916** of the proximal basket **906** attached to the plurality of basket connector tether memory metal strips **939.** The decreased radial force of the basket tether memory metal strips **939** is designed to allow the deployable dual basket system **895** to navigate the tortuous blood vessels **950,** as previously mentioned.

Optionally, the system **895** has only two basket connector tether memory metal strips **939.**

Optionally, in the relaxed state, the height **912** of the proximal basket **906** is greater than the height **929** of the distal basket **923** and further wherein the width **913** of the proximal basket **906** is greater than the width **930** of the distal basket **923.** Optionally, in the relaxed state, the radial force of the distal basket **923,** as measured from the proximal crowns **938** of the cells **934** of the distal basket **923** attached to the plurality of basket connector tether memory metal strips **939** to the distal-most crown **937** of the distal cells **934** of the distal basket **923,** is less than the radial force of the proximal basket **906** as measured from the proximal crowns **918** of the cells **916** of the proximal basket **906** attached to the plurality of proximal memory metal strips **920** to the distal crowns **919** of the cells **916** of the proximal basket **906** attached to the plurality of basket connector tether memory metal strips **919.** The decreased height **929,** width **930** and radial force of the distal basket **923,** as compared to the proximal basket **906,** is designed to prevent vessel damage given that blood vessels **950** generally taper from the proximal end to the distal end. Optionally, in the relaxed state, the radial force of the proximal basket **906** is substantially uniform from the proximal crowns

**918** of the cells **916** of the proximal basket **906** attached to the plurality of proximal memory metal strips **920** to the distal crowns **919** of the cells **916** of the proximal basket **906** attached to the plurality of basket connector tether memory metal strips **939** (i.e., substantially uniform along the length of the proximal basket **906**). Similarly, optionally, in the relaxed state, the radial force of the distal basket **923** is substantially uniform from the proximal crowns **938** of the cells **934** of the distal basket **923** attached to the plurality of basket connector tether memory metal strips **939** to the distal-most crown **937** of the distal cells **934** of the distal basket **923.**

Optionally, the proximal basket interior **908** and the distal basket interior **925** are generally hollow and the proximal basket cells **916** are spaced about the circumference of the proximal basket **906** and the distal basket cells **934** are spaced about the circumference **924** of the distal basket **923.**

Optionally, the basket connector tether memory metal strips **939** do not traverse the system interior **897.** In other words, the connector tether memory metal strips **939,** the proximal basket cells **916** and the distal basket cells **934** each define a portion of the perimeter of the deployable dual basket system **895.**

Optionally, each of the distal crowns **919** of the proximal basket **906** connected to the basket connector tether memory metal strips **939** are approximately the same distance from the proximal junction **915** and further wherein each of the proximal crowns **938** of the distal basket **923** connected to the basket connector tether memory metal strips **939** are approximately same distance from the distal junction **932.**

Optionally, each of the proximal crowns **918** and **938** are connected to a memory metal strip extending proximally from the proximal crowns **918** and **938** and each of the distal crowns **919** and **937** are connected to a memory metal strip extending distally from the distal crowns **919** and **937** (i.e., the proximal crowns **918** and **938** and distal crowns **919** and **937** are connected to either the proximal tether memory metal strips **920,** the proximal basket memory metal strips **917,** the distal basket memory metal strips **933** or the basket connector tether memory metal strips **939**). In other words, there are no free crowns and the proximal basket **906** and distal basket **923** have a closed cell design to prevent vessel injury.

Optionally, the proximal tether memory metal strips form **920** flex points of the deployable dual basket system **895.** The proximal tether memory metal strips **920** may also rotate. For example, in the collapsed state, the distal end **922** of a first proximal tether memory metal strip **920** may be located between about 90 degrees and about 270 degrees relative to the proximal end **921** of the same proximal tether memory metal strip **920,** and further wherein in the collapsed state, the distal end **922** of a second proximal tether memory metal strip **920** may be located between about 90 degrees and about 270 degrees relative to the proximal end **921** of the same proximal tether memory metal strip **920.** Optionally, the first and second proximal memory metal strips **920** intersect/cross adjacent and distal to the proximal junction **915,** as seen in FIGs. 50 and 51. In other words, the length/longitudinal axis of the proximal tether memory metal strips **920** (and the length/longitudinal axis of the basket connector tether memory metal strips **939**) is preferably angled relative to the system longitudinal axis **905,** the proximal basket longitudinal axis **914** or the distal basket longitudinal axis **931.**

Optionally, the basket connector tether memory metal strips **939** form the sole attachment of the proximal basket **906** to the distal basket **923.**

As mentioned, the device **890** of FIGs. 50-56 may be used to open a constricted blood vessel in the case of a subarrachnoid hemorrhage induced vasospasm, as seen in FIGs. 56. It will be understood that the term "blood vessel" includes more than one vessel, as four artery branches are shown in FIG. 56, namely, the M2 middle cerebral artery (MCA), the M1 middle cerebral artery (MCA), the internal carotid artery (ICA) and the A1 anterior cerebral artery (ACA).

For example, the device **890** may be used in a method of treating a human having a subarrachnoid hemorrhage induced vasospasm in a constricted blood vessel **950** having a proximal region **951** having a constricted height **952** and a constricted width and a distal region **954** having a constricted height **955** and a constricted width, the method comprising the steps of:
a) providing the deployable dual basket system **895,** wherein the distal basket **923** and the proximal basket **906** are in the collapsed state and located in the catheter interior **944;**
b) positioning the deployable dual basket system **895** in the blood vessel **950** so that the distal end **946** of the catheter **943** is distal to the distal region **954** of the blood vessel **950;**
c) deploying the proximal basket **906** and the distal basket **923** from the distal end **946** of the catheter **943** into the distal region **954** of the blood vessel **950;** and
d) allowing the height **929** and width **930** of the distal basket **923** and the proximal basket **906** to increase and cause the height **955** and width of the distal region **954** of the blood vessel **950** to increase. Optionally, the method further includes e) moving the deployable dual basket system **895** proximally in the relaxed state within the blood vessel **950** and into the proximal region **951** to cause the height **952** and width of the proximal region **951** of the blood vessel **950** to increase; and f) withdrawing the deployable dual basket system **895** from the blood vessel **950** and out of the human.

As mentioned above, the term "blood vessel" may or may not include multiple blood vessels. For example, in FIG. 56, the constricted distal region **954** of the blood vessel **950** is the M2 of the middle cerebral artery and the constricted proximal region **951** of the blood vessel **950** is the M1 segment of the middle cerebral artery. Alternatively, the proximal region **951** and distal region **954** may be two discrete (albeit connected) blood vessels.

The blood vessel **950** is lined with endothelium **957** and preferably the method comprises performing steps a) - f) without damaging the endothelium **957.**

The devices **895** of FIGS. 50-56 may be manufactured by any suitable method. In an example, the device **895** is assembled in a method similar to FIGs. 33A-49. The method may include: a) providing a first tube comprised of a memory metal as previously described with respect to FIGs. 33A-49;b) using a cutting instrument to cut portions of the first tube wall and form a proximal matrix (i.e., the precursor to proximal basket **906**) in the proximal middle portion comprising a plurality of proximal middle portion memory metal strips forming a plurality of proximal matrix cells, each proximal matrix cell having a proximal crown pointing generally in the proximal direction and a distal crown pointing generally in the distal direction and a proximal matrix cell length extending from the proximal crown to the distal crown and generally parallel to the first tube longitudinal axis; ii) a plurality of proximal tether memory metal strips **920,** each proximal tether memory metal strip **920** having a proximal tether memory metal strip proximal end **921,** a proximal tether memory metal strip distal end **922** connected to a proximal crown of a proximal matrix cell and a proximal memory metal strip length extending from the proximal tether memory metal strip proximal end **921** to the proximal tether memory metal strip distal end **922,** the proximal tether memory metal strips **920** formed by moving the cutting instrument at an angle (e.g.., between about 90 degrees and 270 degrees relative to the first tube longitudinal axis); iii) a distal matrix (i.e., the precursor to the distal basket **923**) in the proximal middle portion comprising a plurality of distal middle portion memory metal strips forming a plurality of distal matrix cells, each distal matrix cell having a proximal crown pointing generally in the proximal direction and a distal crown pointing generally in the distal direction and a distal matrix cell length extending from the proximal crown to the distal crown and generally parallel to the first tube longitudinal axis; iv) a plurality of basket connector tether memory metal strips **939,** each basket connector tether memory metal strip **939** having a basket connector tether memory metal strip proximal end **940** connected to a distal crown of a proximal matrix cell, a basket connector tether memory metal strip distal end **941** connected to a proximal crown of a distal matrix cell and a basket connector tether memory metal strip length extending from the basket connector tether memory metal strip proximal end **940** to the basket connector tether memory metal strip distal end **941,** the basket connector tether memory metal strips **939** formed by rotating the first tube about the first tube longitudinal axis relative to the cutting instrument so that the proximal end **940** of a basket connector tether memory metal strip **939** is located between about 90 degrees and about 270 degrees relative to the distal end **941** of the same basket connector tether memory metal strip **939;** and v) a plurality of proximal longitudinal perforations **958** as described previously, wherein a proximal longitudinal tab **960** is located between and connects adjacent proximal segments **959** of adjacent proximal tether memory metal strips **920** and is formed from uncut portions of the first tube wall; c) shape setting at least the proximal middle portion and the distal middle portion to expand the width of the proximal middle portion and the distal middle portion and form a proximal basket **906** comprised of the proximal matrix cells and a distal basket **923** comprised of the distal matrix cells, the proximal basket **906** and the distal basket **923** connected by the basket connector tether memory metal strips **939;** d) after step c), polishing the first tube, wherein said polishing expands the plurality of proximal longitudinal perforations **958** so that the proximal longitudinal gaps become smaller and adjacent proximal longitudinal perforations **958** approach each other; e) tearing along the plurality of proximal longitudinal perforations **958** to free the proximal segments **959** of the proximal tether memory metal strips **920** from the proximal longitudinal tabs **960** and each other; f) joining the free proximal segments **959** of the proximal tether memory metal strips **920** (e.g., using a coil as shown in FIG. 55) to form a medical device comprised of the joined proximal segments **959** of the proximal tether memory metal strips **920,** the proximal basket **906,** the basket connector tether memory metal strips **939,** and the distal basket **923,** the medical device having a medical device length extending at least from the distal basket **923** to at least the joined proximal segments **959** of the proximal tether memory metal strips **920** and a medical device width generally perpendicular to the medical device length; and g) inserting the medical device into a catheter **943** comprising a catheter interior **944** having an interior width, an open catheter proximal end **945** leading to the catheter interior **944,** an open catheter distal end **946** leading to the catheter interior **944,** the catheter **943** comprised of a biocompatible material, wherein the medical device comprises a collapsed state wherein the medical device width is less than the catheter interior width and a relaxed state wherein the medical device width is greater than the catheter interior width, wherein the catheter **943** is configured to envelope the medical device when the medical device is in the collapsed state, and further wherein the catheter interior width is less than the first tube outer width.

Optionally, the process further includes forming distal longitudinal perforations **961,** distal longitudinal tabs **963** and rejoining the distal basket memory metal strip distal ends **936** using a third tube **968** as described previously and shown in FIG. 54. In addition, the process may include forming proximal perimeter perforations **964,** proximal end tab **965,** distal perimeter perforations **966** and distal end tab **967.** It will be appreciated that the manufacturing process has been described and illustrated in abbreviated form due to the similarities to FIGs. 33A-49. As with FIGs. 33A-49, the process of FIGs. 52-55 allows one to form the proximal and distal baskets **906** and **923** from a tube having a first tube diameter, and then removing the proximal and distal ends of the first tube (and attaching coil and third tube **968,** which have a smaller diameter than the first tube diameter) in order to allow the deployable dual basket system **895** to fit inside a catheter having a diameter less than the first tube diameter.

Optionally, the cells **916** of the proximal basket **906** are substantially equal in size to each other and to the cells **934** of the distal basket **923** in the relaxed state - e.g., the surface area of the cells **916** and **934** may vary by no more than 5%.

The deployable dual basket system **895** of FIGs. 50-56 may have a length of, for example, between about 10 mm (millimeters) and 60 mm, more preferably between about 30 mm and about 60 mm.

The system of FIGs. 50-56 may include a lead wire extending from the distal junction **932,** as described above with respect to the systems of FIGS. 1-49.

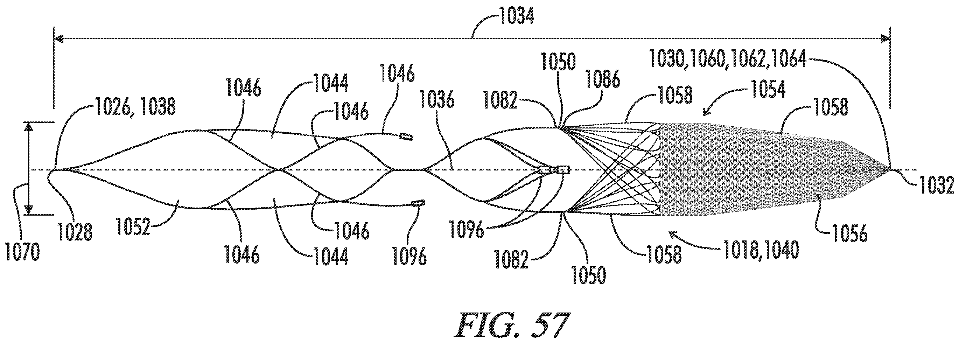

### The examples shown in FIGs. 57-72, 81A, and 81B

FIGs. 57-72, 81A and 81B illustrate an embodiment of the present invention in which the distal body **1018** includes a proximal portion **1042** that has cells **1044** and a distal portion **1048** that has mesh openings **1056.** The proximal portion **1042** may be similar to the baskets shown in FIGs. 11-56 above. With respect to the distal portion **1048,** the mesh openings **1056** may be small openings that serve to impede blood flow, as well as to capture any small emboli captured by the basket **1040** from escaping through the basket **1040.**

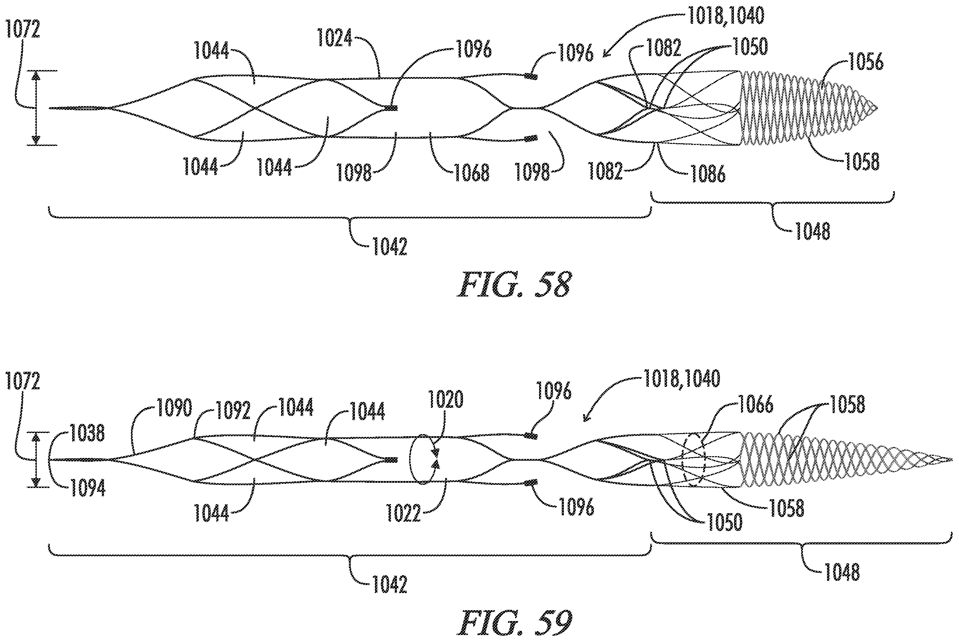

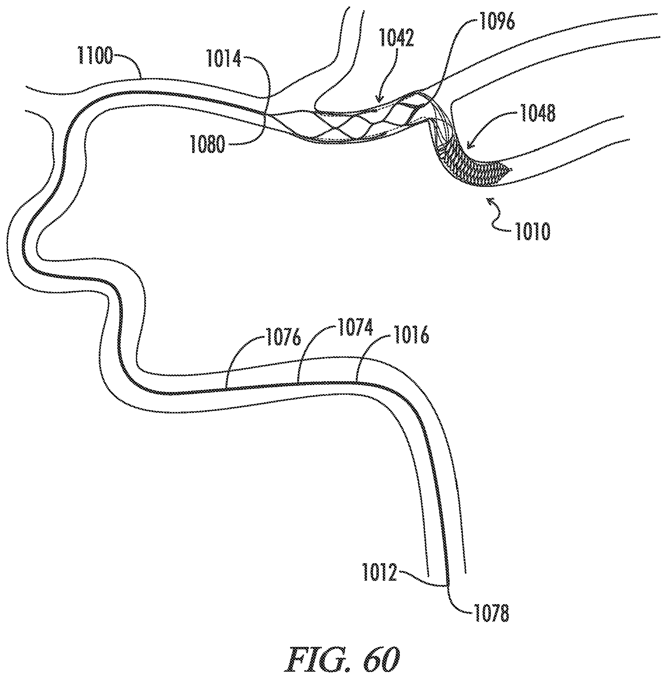

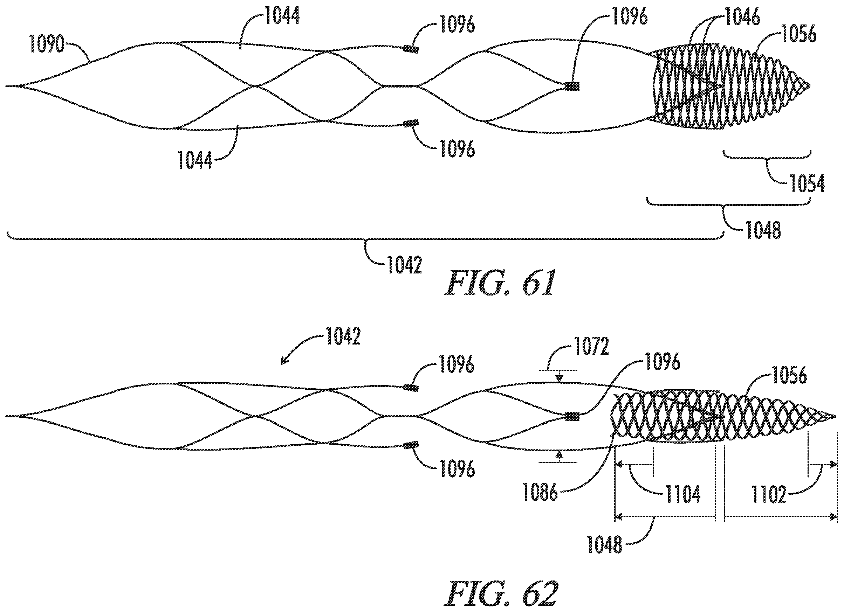

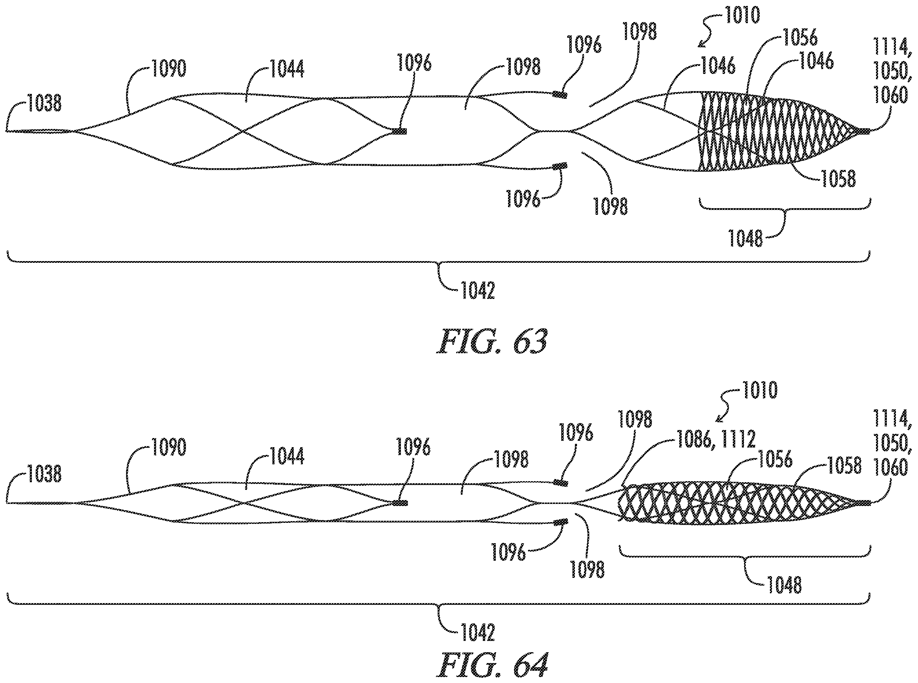

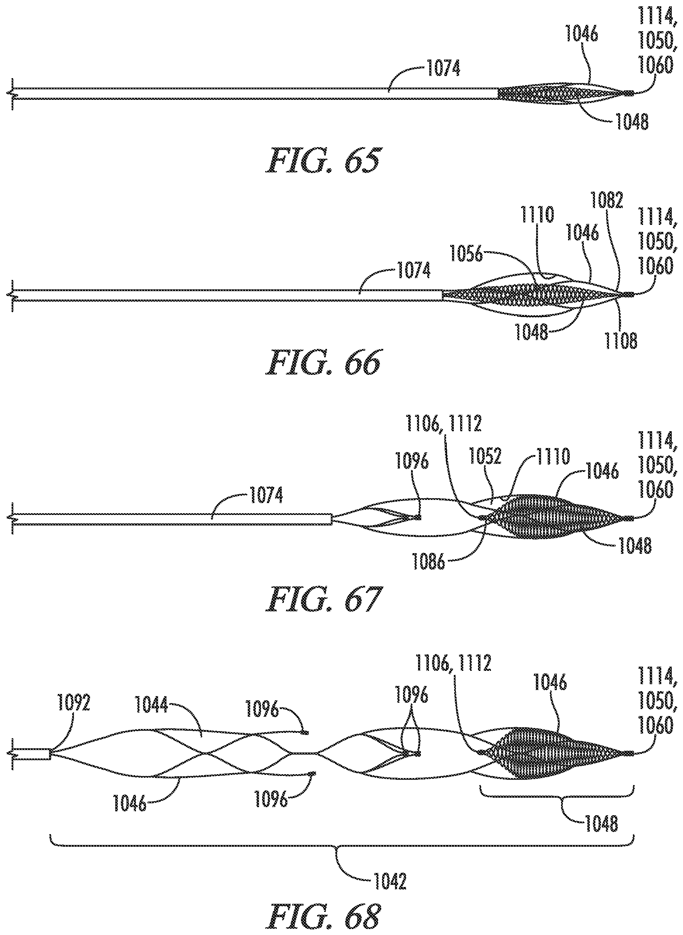

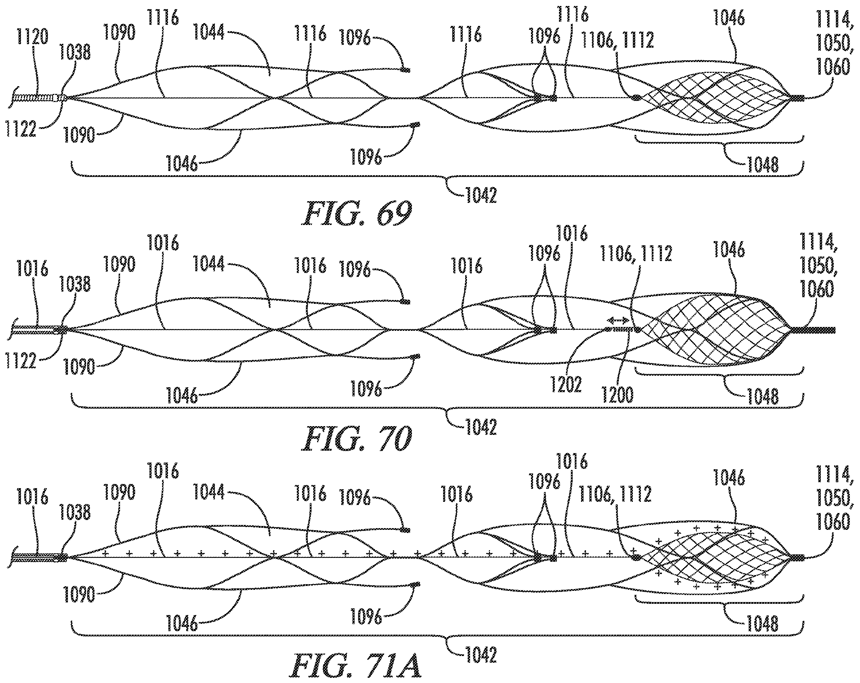

Six examples of the design are shown in FIGs. 57-72, 81A and 81B. FIGs. 57-60 show an example where the proximal end **1086** of a woven linear strand **1058** of a distal portion **1048** is attached to the distal end **1082** of a basket memory metal strip **1046** of the proximal portion **1042.** In such case, the distal portion **1048** may elongate as shown by comparing FIG. 58 (relaxed state) and FIG. 59 (partially collapsed state) when the distal body **1018** moves to the collapsed state. FIG. 60 shows how the distal portion **1048** of some examples of the present invention is able to navigate tortuous blood vessel's **1100** due to the increased flexibility and decreased radial force of the distal portion **1048** as compared to the proximal portion **1042** in some examples of the present invention. The distal ends **1082** of the basket memory metal strips **1046** may be attached to the proximal ends **1086** of the woven linear strands **1058** by welding, soldering or a crimp for example. FIGs. 61-62 show an example in which the distal portion **1048** is attached to the interior of the proximal portion **1042** (e.g., by welding, soldering or the like) at multiple connection points **1050** and the distal portion **1048** and the proximal portion **1042** partially overlap. As shown by comparing FIG. 61 (relaxed state) and FIG. 62 (partially collapsed state), the distal portion **1048** elongates distal and proximal to the connection points **1050** in moving from the relaxed state to the collapsed state. Meanwhile, the segment at the connection points **1050** preferably does not elongate as shown in FIG. 62. FIGs. 63-64 show an example in which the distal portion **1048** is fully located in the proximal portion interior **1052.** In FIGs. 63-64, the sole connection point **1050** of the proximal portion **1042** and the distal portion **1048** is the distal body distal junction **1060,** which may be in the form of a distal tube, including a coil, as previously described. More particularly, the distal ends **1082** of the basket memory metal strips **1046** located at the distal end **1064** of the basket **1040** and the distal ends **1108** of the woven linear strands **1058** meet at the distal body distal junction **1060.** FIGs. 65-68 show an example. Similar to FIGs. 63-64, the design shown in FIGs. 65-68 includes the distal portion **1048** fully located within the proximal portion interior **1052** and the sole connection point **1050** of the proximal portion **1042** and the distal portion **1048** is the distal body distal junction **1060,** which may be in the form of a distal tube. Again, more particularly, the distal ends **1082** of the basket memory metal strips **1046** located at the distal end **1064** of the basket **1040** and the distal ends **1108** of the woven linear strands **1058** meet at the distal body distal junction **1060.** In FIGs. 65-68, the proximal ends **1086** of the woven linear strands **1058** converge at and are attached to a free-floating distal portion proximal junction **1106** that forms the proximal end **1112** of the distal portion **1048.** By contrast, in FIGs. 63-64, the proximal ends **1086** of the woven linear strands do not converge and instead are preferably located adjacent to an interior surface **1110** of one or more of the basket memory metal strips **1046.** An example is shown in FIGs. 69-72. In FIGs. 69-72, the distal portion **1048** is fully located within the proximal portion interior **1052** and the distal ends **1108** of the woven linear strands **1058** meet at the distal body distal junction **1060.** However, in FIGs. 69-72, the distal portion **1048** is attached to the distal body proximal junction **1038** by a proximal tether, which among other things, is believed to assist in re-sheathing the distal body **1018** into the catheter **1074** (i.e., repositioning the distal body **1018** into the catheter **1074** after the clot has been retrieved) as well as to keep the distal portion **1048** centered and away from the vessel wall when the distal body **1018** moves around a curved vessel **1100.** The proximal tether is preferably located in the center of the height **1070** and width **1072** of the distal body **1018** in the relaxed state and preferably is parallel to the distal body longitudinal axis **1036.** The proximal tether may also slightly stretch the distal portion **1048** during the re-sheathing process. The proximal tether may be a suture or other thin material **1116** that has a proximal end attached to the distal body proximal junction **1038** and a distal end attached to the distal portion proximal junction **1106,** as shown in FIG. 69. Alternatively, the proximal tether may be a segment of the pull wire **1016,** as shown in FIGs. 70-72, in which case the proximal tether may be comprised of stainless steel or nitinol for example. If the proximal tether is conductive, a positive or negative charge (a current) may be propagated along the tether to the distal portion **1048** in order to interact with a blood clot captured in the distal body **1018.** (For example, depending on the charge propagated, the charge may assist in clotting or in attraction to a charged blood clot). If the proximal tether is comprised of suture material, it may be proline or nylon and nonabsorbable for example and may be size 4-0 to size 1-0. If the proximal tether is a segment of the pull wire **1016,** it may have an outer diameter of 0.002 inches to about 0.010 inches for example. FIG. 70 illustrates a particular example in which a segment of the proximal tether is in the form of a proximal helical coil/coil spring **1200.** The proximal helical coil **1200** has a coil length generally parallel to the distal body length **1034,** the proximal helical coil **1200** has an expanded/elongated state in which the proximal helical coil **1200** has a first length and a relaxed state in which the proximal helical coil **1200** has a second length, the first length greater than the second length. In other words, the proximal helical coil **1200** may stretch as illustrated by the arrows in FIG. 70 if tension is exerted on the proximal tether in an effort to avoid damage to the proximal tether. The proximal helical coil **1200** is preferably adjacent to the distal portion proximal junction **1106.** In FIG. 70, the helical coil **1200** is a radiopaque stretch coil soldered at the proximal end to the pull wire **1016** and epoxied at the distal end to the distal portion proximal junction **1106.** The point of solder is denoted by numeral **1202.** A sixth iteration is shown in FIGs. 81A and 81B. The embodiment of FIGs. 81A and 81B is similar to the example of FIG. 70 except that the embodiment of FIGs. 81A and 81B includes a distal tether **1204** attaching the distal portion/distal body inner body distal end **1114** to the distal junction **1060.** In the illustration of FIGs. 81A-81B, a segment of the distal tether **1204** is a distal helical coil/coil spring **1206.** The illustration of FIGs. 81A-81B does not include a proximal helical coil/coil spring. However, as the distal body **1018** is re-sheathed by a catheter **1074** (e.g, after clot capture), like the previously-described proximal helical coil/coil spring **1200,** the distal helical coil/coil spring **1206** may elongate (as the distal portion/distal body inner body **1048** elongates) during the re-sheathing process and, thus, the distal helical coil/coil spring **1206** can be used to prevent damage during the re-sheathing process. In particular, in experimental testing, it was shown that the distal helical coil/coil spring **1206** prevented damage of the distal portion/distal body inner body **1048** during the re-sheathing process.

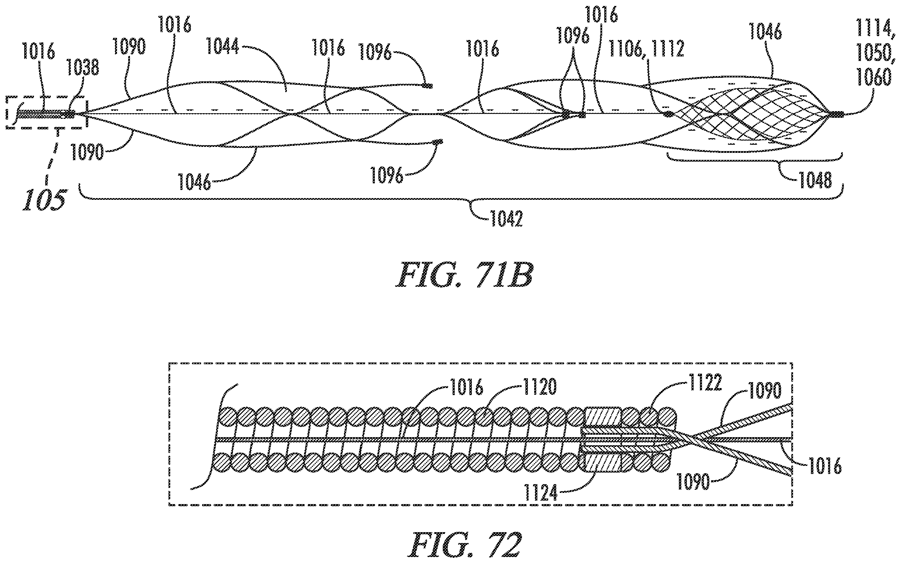

The examples of FIGs. 57-72 and 81A-81B are drawn to scale. However, it will be understood that the dimensions provided are merely exemplary. It will also be appreciated that distal portion **1048** has a reduced height and width as compared to the proximal portion **1042** in the relaxed state in the illustrations of FIGs. 69 and 71. It will be appreciated that FIGs. 69-71 and 81A show the proximal end of the distal portion **1048** as being closed, as the proximal ends **1086** of the woven linear strands **1058** converge at and are attached to the distal portion proximal junction **1106.** The convergence, which is also shown in FIGs. 67-68, is thought to prevent the distal portion woven linear strands **1058** from unraveling.

Given that, in FIGs. 63-72, 81A, and 81B, the distal portion **1048** is fully located within the proximal portion interior **1052,** the distal portion **1048** is also referred to herein as the "distal body inner body" and the proximal portion **1042** is also referred to herein as the "distal body outer body" to more accurately reflect the fact that the woven linear strands **1058** are located within the basket memory metal strips **1046** of the proximal portion interior **1052.** Optionally, as demonstrated in FIGs. 63 and 68, at least some the woven linear strands **1058** contact the interior surface **1110** of at least some of the basket memory metal strips **1046** in the relaxed state. For example, a segment of all the woven linear strands **1058** may contact the interior surface **1110** of at least some of the basket memory metal strip **1046** in the relaxed state, as shown in FIG. 63 and 68.

In some of the examples of FIGs. 57-72 and 81A-81B, the proximal ends **1086** of the woven linear strands **1058** may be free; however, it is believed that they will not damage the vessel **1100** because they are located in the proximal portion/distal body outer body interior **1052.**

As shown in FIGs. 57-72 and 81A-81B the distal portion/distal body inner body **1048** is located adjacent (i.e., at or near the distal end **1064** of the distal basket **1040**). In some examples, i.e., FIGs. 57-62, at least a segment **1054** of the distal portion/distal body inner body **1048** is located distal to the proximal portion **1042.**

More particularly, as shown in FIGs. 57-72 and 81A-81B, the present disclosure provides a system **1010** for removing objects from an interior lumen **1100** of an animal. The system **1010** includes a pull wire **1016** having a proximal end **1012** and a distal end **1014,** as previously described.

The system **1010** may further include a distal body **1018** attached to the pull wire **1016,** the distal body **1018** comprising a distal body perimeter **1020** separating a distal body interior **1022** from a distal body exterior **1024,** a proximal end **1026** having a proximal end center **1028,** a distal end **1030** having distal end center **1032,** a distal body length **1034** extending from the proximal end **1026** to the distal end **1030,** a longitudinal axis **1036** extending through the proximal end center **1028** and the distal end center **1032** and parallel to the distal body length **1034,** and a proximal junction **1038** forming the proximal end of the distal body **1026.**

The distal body **1018** may further include a proximal portion/distal body outer body **1042** comprising a basket **1040** comprised of a plurality of cells **1044** spaced about the distal body perimeter (e.g., circumference) **1020** and formed by a plurality of basket memory metal strips **1046** and a distal portion/distal body inner body **1048** connected to the proximal portion/distal body outer body **1042** at one or more connection points **1050,** the proximal portion/distal body outer body **1042** comprising a proximal portion/distal body outer body interior **1052.** The distal portion/distal body inner body **1048** is preferably located at the distal end **1064** of the basket **1040** and may or may not have at least a segment **1054** distal to the proximal portion **1042.** The distal portion/distal body inner body **1048**comprises a plurality of distal braided mesh openings **1056** formed by a plurality of woven linear strands **1058.** The system may further include a distal body distal junction **1060** comprising a proximal end **1062.** The proximal end **1062** of the distal body distal junction **1060** may form a distal end **1064** of the basket **1040.** The distal portion/distal body inner body **1048** may have a perimeter **1066** and each woven linear strand **1058** may rotate about the distal portion/distal body inner body perimeter **1066** relative to the distal body longitudinal axis **1036** a plurality of times in a helical fashion. The helical rotation is best seen in FIGs. 58-68. In some examples, at least some of the distal braided mesh openings **1056** are distal to the cells **1044** as shown in FIGs. 57-62. The basket **1040** may comprise a basket interior **1068.** The distal body **1018** may have a relaxed state wherein the distal body **1018** has a first height **1070** and a first width **1072,** and a collapsed state wherein the distal body **1018** has a second height **1070** and a second width **1072,** the second height less than the first height, the second width less than the first width.

The system further includes a catheter **1074,** as previously described, having an interior **1076,** a proximal end **1078** leading to the interior **1076** and a distal end **1080** leading to the interior **1076,** the catheter **1074** comprised of a biocompatible material and configured to envelope the distal body **1018** when the distal body **1018** is in the collapsed state. In the relaxed state, the median surface area of the cells **1044** is larger than the median surface area of the distal braided mesh openings **1056.** In other words, the average surface area of the cells **1044** is preferably greater (preferably substantially greater) than the average surface area of the distal mesh openings **1056** in the relaxed state, as shown in FIGs. 57-58, 61, 63 and 68 and 81A. Optionally, in the relaxed state, the median radial force of the distal portion/distal body inner body **1048** is substantially less than the median radial force of the proximal portion/distal body outer body **1042** (e.g., 25% or less of the radial force of the proximal portion/distal body outer body **1042**), it being understood that the radial force of the proximal portion/distal body outer body **1042** may vary along its length due to the free distal crowns **1096,** which may create enlarged cells **1098** as previously described.

Optionally, the radial force of the proximal portion/distal body outer body 1042 through its connection to the distal portion/distal body inner body **1048** at the connection point(s) **1050** is configured to cause the distal portion/distal body inner body **1048** to move to the relaxed state when the proximal portion/distal body outer body **1042** moves from the collapsed state to the relaxed state. The aforementioned phenomena is not present in FIGs. 63-68, where the sole connection point **150** of the distal portion/distal body inner body **1048** and the proximal portion/distal body outer body **1042** is the distal body distal junction **1060.**

The proximal portion/distal body outer body **1042** and the distal portion/distal body inner body **1048** each have a length generally parallel to the distal body length **1034,** the proximal portion/distal body outer body **1042** and distal portion/distal body inner body **1048** lengths configured to elongate upon moving from the relaxed state to the collapsed state. Optionally, upon moving from the relaxed state to the collapsed state, the length of the distal portion/distal body inner body **1048** is configured to elongate a greater percentage as compared to the elongation of the proximal portion/distal body outer body **1042** as shown by comparing FIG. 66 with FIG. 68, by comparing FIG. 59 with FIG. 58, by comparing FIG. 62 with FIG. 61, and by comparing FIG. 64 with FIG. 63. Optionally, the woven linear strands **1058** rotate about the distal body distal portion/inner body perimeter **1066** relative to the distal body longitudinal axis **1036** a fewer number of times per unit of distance/length in the collapsed state as compared to the relaxed state, similar to what is seen when stretching a phone cord.

Optionally, in the relaxed state, the proximal portion/distal body outer body **1042,** but not the distal portion/distal body inner body **1048,** is configured to alter the shape of a curved intracranial artery, allowing the distal portion/distal body inner body **1048** to be used in tortuous vessels **1110** as shown in FIG. 60. Optionally, in the relaxed state, the distal portion/distal body inner body **1048** is more flexible than the proximal portion/distal body outer body **1042,** again allowing the distal portion/distal body inner body **1048** to be used in tortuous vessels **1110** as shown in FIG. 60. Optionally, the woven linear strands **1058** are comprised of a biocompatible material such as suture, a metallic material, Dacron, Teflon or vascular graft material. The woven linear strands **1058** may be comprised of a memory metal. In some examples, the woven linear strands **1058** are braided filaments that have the same diameter. In some examples, the woven linear strands **1058** are comprised of a material similar to the PIPELINE embolization device (ev3, Plymouth, Minnesota), which is a flow diverter and is said to be comprised of a 75% cobalt chromium 25% platinum tungsten bimetallic design, or the SPIDER FX embolic protection device (also made by ev3). Similar devices are made by other companies.

Optionally, the distal portion/distal body inner body **1048** in the relaxed state comprises a tapered region in which the distal body height **1070** and width **1072** decrease as the woven linear strands **1058** approach the distal body distal junction **1060** as shown in FIGs. 63 and 68-71. Optionally, in the relaxed state, the basket interior **1068** is substantially hollow.

Optionally, the proximal portion **1042** comprises a distal end comprising between two and four basket memory metal strip distal ends **1082** and further wherein each woven linear strand **1058** comprises a proximal end **1086** attached to a basket memory metal strip distal end **1082,** as shown in FIGs. 57-59. Optionally, the distal portion/distal body inner body **1048** comprises at least two woven linear strands **1058** attached to each basket memory metal strip distal end **1082.** Optionally, in the relaxed state, the basket memory metal strips **1046** of the proximal portion/distal body outer body **1042** comprises an interior surface **1110** facing the distal body interior **1022** and the distal portion/distal body inner body **1048** comprises an outer/exterior surface facing and connected to the basket memory metal strips interior surface **1046,** and further wherein at least a segment of the distal portion/distal body inner body **1048** is interior to the proximal portion/distal body outer body **1042,** as shown in Figs. 61 and 62. Optionally, each woven linear strand **1058** comprises a free proximal end **1086** and further wherein all free proximal ends **1086** of the woven linear strands **1058** are located in the proximal portion/distal body outer body interior **1052,** as shown in FIGs. 61-68. Optionally, the distal portion/distal body inner body **1048** is configured to elongate proximally and distally relative to the proximal portion/distal body outer body **1048** and the plurality of connection points **1050** upon moving from the relaxed state to the collapsed state, as shown in FIG. 62.

Optionally, the distal portion/distal body inner body **1048** is attached to the proximal portion/distal body outer body **1042** by at least two connection points **1050,** and further wherein said at least two connection points **1050** are located a slightly different distance from the proximal junction **1038** in the relaxed state. Optionally, said at least two connection points **1050** are located a slightly different distance from the proximal junction **1038** in the collapsed state. In other words, the connection points **1050** may be staggered slightly in the relaxed and collapsed states to aid collapsing of the distal body **1018.**

Optionally, a plurality of woven linear strand proximal ends **1088** are connected to each basket memory metal strip distal end **1082.**

Optionally, in the relaxed state, the distal portion/distal body inner body **1048** impedes blood flow to a greater extent than the proximal portion/distal body outer body **1042** when the proximal portion/distal body outer body **1042** and the distal portion/distal body inner body **1048** are placed in a blood vessel **1100.**

Optionally, the distal portion/distal body inner body **1048** is configured to reduce blood flow by at least 25% (preferably at least 50%) when the distal portion/distal body inner body **1048** is placed in a blood vessel **1100,** which may obviate the need for a suction catheter.

Optionally, the distal portion/distal body inner body **1048** is radiopaque.

Optionally, the proximal portion/distal body outer body **1042** of the distal body **1018** further comprises a plurality of proximal strips **1090,** each proximal strip **1090** having a distal end **1092** attached to a cell **1044** (more particularly a proximal crown of a cell **1044**) and a proximal end **1094,** the proximal ends **1094** of the proximal strips **1090** converging at the proximal junction **1038.** Preferably, in the relaxed state, the length of the distal portion/distal body inner body **1048** is no more than 33% of the length of the proximal portion/distal body outer body **1042** (e.g., the length of the distal portion/distal body inner body **1048** may be about 2% to about 33% of the length of the proximal portion/distal body outer body **1042**).

Optionally, in the relaxed state, as previously described, the proximal portion/distal body outer body may include offset free distal crowns **1096** with x-ray markers and offset enlarged cells **1098.** More particularly, the proximal portion/distal body outer body **1042** may comprise a first pair of distal crowns **1096** not attached to another cell of the basket **1040** and pointing generally in the distal direction, the distal crowns **1096** in the first pair of distal crowns **1096** located approximately the same distance from the proximal junction **1038** and between 150 degrees and 180 degrees relative to each other, and further wherein the basket **1040** further comprises a second pair of distal crowns **1096** not attached to another cell of the basket **1040** and pointing generally in the distal direction, the second pair of distal crowns **1096** located distally relative to the first pair of distal crowns **1096,** each of the distal crowns **1096** in the second pair of distal crowns **1096** located between 60 degrees and 90 degrees relative to a distal crown **1096** in the first pair of distal crowns **1096,** the distal crowns **1096** in the second pair of distal crowns **1096** located approximately the same distance from the distal body proximal junction **1038,** each of the distal crowns **1096** forming a portion of a cell **1044.** Optionally, each distal crown **1096** in the first and second pair of distal crowns **1096** forms part of a different enlarged cell/drop zone **1098,** each enlarged cell/drop zone **1098** having a center and the centers of the enlarged cells **1098** of the first pair of distal crowns **1096** located approximately 180 degrees relative to each other (e.g., between 150 and 180 degrees) and approximately 90 degrees (e.g., between 60 and 90 degrees) relative to the centers of the enlarged cells/drop zones **1098** of the second pair of distal crowns 1096. Optionally, the surface area of the enlarged cells/drop zones **1098** in the relaxed state is greater than the surface area of the other cells **1044** of the basket 1040. Optionally, the enlarged cells/drop zones **1098** are configured to allow a thrombus to pass therethrough and into the basket interior **1068.** The distal crowns **1096** may include x-ray markers as previously described. Optionally, the distal portion/distal body inner body **1046** is located fully distal relative to all free distal crowns **1096** and enlarged cells/drop zones **1098** as shown in FIGs. 81A-81B for example.

The proximal portion/distal body outer body **1042** differs from the distal portion/distal body inner body **1048** in several physical characteristics. For example, the proximal portion/distal body outer body **1042** is preferably prepared by using a laser to cut a single memory metal tube similar to the examples of FIGs. 11-20, for example (e.g., as shown in FIGs. 1A, 1B 33A and 33B); whereas the distal portion/distal body inner body **1048** is preferably prepared from woven linear strands **1058.** In addition, the woven linear strands **1058** preferably slide relative to each other, whereas the basket memory metal strips **1046** of the proximal portion/distal body outer body **1042** meet at fixed nodes (crowns). In addition, the woven linear strands **1058** may be cylindrical in shape, whereas the basket memory metal strips **1046** may be trapezoidal in shape, and the width/diameter of the woven linear strands **1058** may be substantially smaller (e.g., five times or ten times smaller) than the maximum width of the basket memory metal strips **1046.** FIG. 72 illustrates coupling of the proximal strip proximal ends **1094** using a coil comprising a proximal coil **1120** and a distal coil **1122** separated by a gap **1124,** similar to FIGs. 43A-G, 44 and 55. FIG. 72 also illustrates rotation of the proximal strips **1090.**

Optionally, the system **1010** may be used method of removing a blood clot from a blood vessel **1100** of an animal, the method comprising the steps of: a) providing the system **1010;** b) positioning the system 1010 in the blood vessel **1100;** c) deploying the distal body **1018** from the distal end **1080** of the catheter **1074;** d) allowing the height 1070 and width **1072** of the distal body **1018** to increase; e) moving the blood clot into the basket interior **1068;** and f) moving the distal body **1018** (and captured blood clot) proximally out of the blood vessel **1100.**

Optionally, the method further includes applying contrast dye proximally and distally to the blood clot.

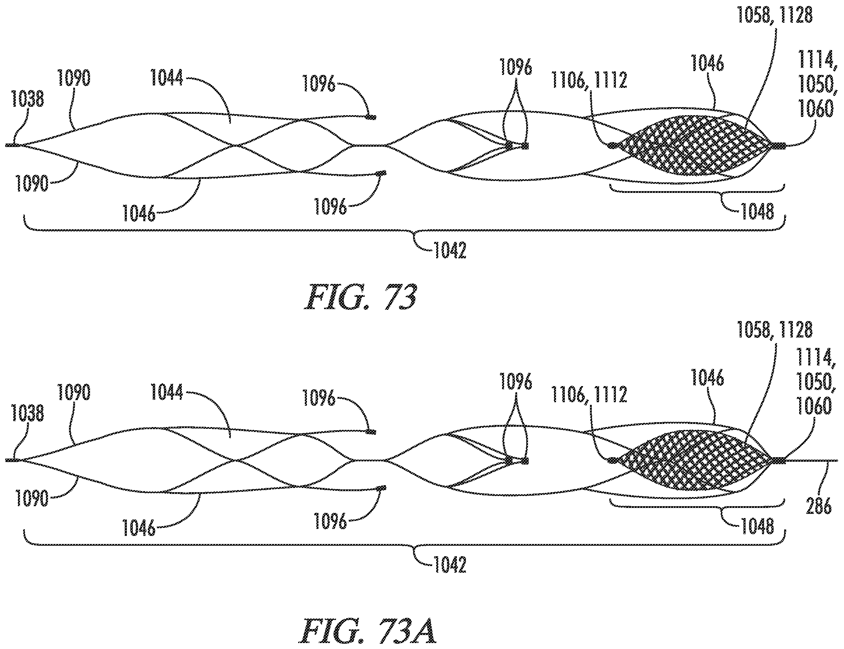

The examples of FIGs. 57-72 and 81A-81B may include a lead wire **286** as described previously. The lead wire **286** may extend from the distal end **1030** of the distal body **1018** and the distal body distal junction **1060** as shown in FIG. 73A. Alternatively, the distal body distal junction **1060** may be elongated, as shown in FIG. 70, which depicts the distal body distal junction **1060** as an elongated coil to prevent damage to the vessel.

### The examples shown in FIGs. 73-80

FIGs. 73-80 illustrate how an active agent **1128** can be used with the examples of FIG. 57-72. The active agent **1128** may be a pharmaceutical or biologic that is configured to dissolve in the blood vessel **1100** and has therapeutic efficacy in the case of an ischemic stroke. For example, the active agent **1128** may be a reloytic (clot dissolving agent) such as tissue plasminogen activator (TPA), abciximab or urokinase for example. The active agent **1128** may also be an reo-adhesive agent to allow the woven linear strands **1058** to swell when contracting blood to further reduce porosity of the distal body inner body **1048.** The active agent **1128** may also be a neuroprotective agent such as minocycline. The term active agent **1128** includes those now known and later developed.

More particularly, FIG. 73 illustrates active agent **1128** that coats the woven linear strands **1058.** In further detail, the distal body inner body **1048** has an increased surface area due to the number of woven linear strands **1058.** For example, the distal body inner body **1048** is comprised of between thirty six and sixty woven linear strands **1058.** This increased surface area allows for a high concentration of active agent **1128** per unit length. The location of the active agent **1128** at the distal body inner body **1048** may have several advantages including but not limited to 1) run off of active agent **1128** at the distal end **1030** of the distal body **1018** into stroke territory where ischemia exists; 2) to prevent formation of new clot on woven linear strands **1058** during deployment and retrieval; 3) to increase adherence/stickiness of the distal body inner body **1048** to trap/adhere to the clot **1126;** and 4) so that the active agent **1128** is located in the distal capture portion of the distal body **1018.** Though not shown, the distal body **1018** of FIG. 73 may include a tether as previously described.

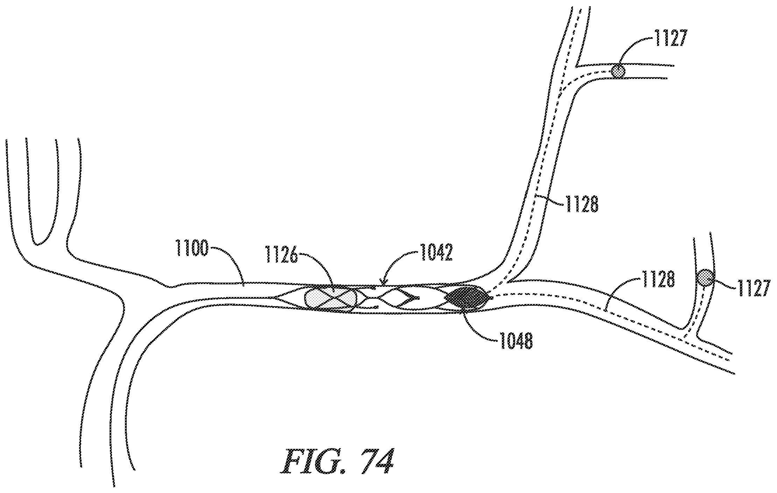

FIG. 74 illustrates use of the system of FIG. 73 in a blood vessel **1100.** As shown in FIG. 74, the main blood clot **1126** causing the ischemia is captured by the distal body outer body **1042.** The active agent **1128,** which may be a reolytic agent, may be used to dissolve the secondary clot/distal emboli 1127.

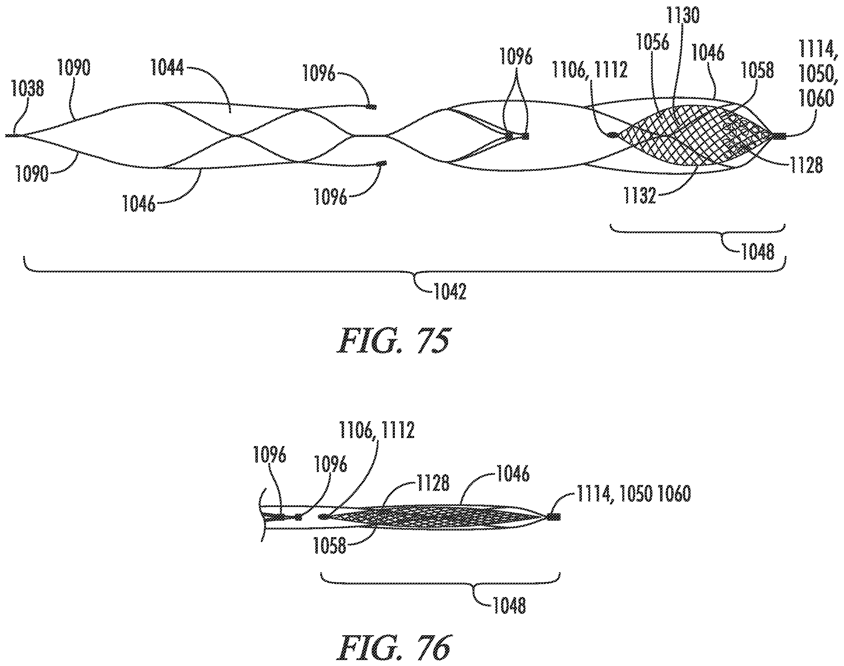

FIG. 75 illustrates active agent **1128** that are located in the distal body inner body interior **1130.** More particularly, the active agent **1128** may in the form of particles that are trapped in the distal body inner body interior **1130** by the woven linear strands **1058.** Each distal braided mesh opening **1056** may have a width of less than 100 microns and the D90 particle size diameter/width of the active agent **1128** (prior to dissolving) may be larger than 200 microns for example so the particles are trapped in the distal body inner body interior **1130.** The particles may then slowly dissolve in the presence of blood flow through the distal portion of the distal body **1018** over a period of minutes before dissolving to a size that allows the dissolved particles to flow to the blood vessels **1110** within the stroke territory where they completely dissolve. As the distal body inner body **1048** is preferably tapered at its proximal end **1112** and distal end **1114** (e.g., in the shape of an American football), the distal braided mesh openings **1056** may be exponentially smaller at the distal body inner body proximal end **1112** and distal body inner body distal end **1114** than the distal braided mesh openings **1056** along the middle portion of the distal body inner body **1132.** FIG. 75 shows the particles of active agent **1128** congregating at the distal body inner body distal end **1114** where the width of the distal braided mesh openings **1056** is significantly less than 100 microns. Though not shown, the distal body **1018** of FIG. 75 may include a tether as previously described.

FIG. 76 shows the distal body **1018** in the collapsed state with drug particles distributed evenly in nearly a single file line.

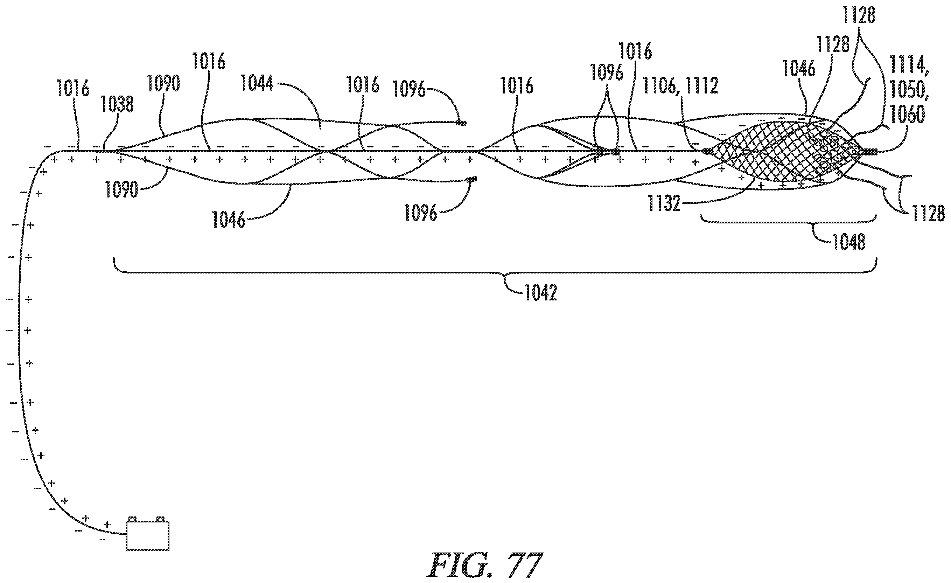

FIG. 77 illustrates electrolysis to release the active agent **1128** from the distal body inner body interior **1130.** (A similar method may be used to release the active agent coating of FIG. 73). For example, a positive or negative charge may be propagated along the pull wire **1016** to cause elution of the active agent **1128** due to the presence of the positive or negative charge. The system may take advantage of the "floating"/middle portion of the distal body inner body **1132** allowing build up of selective charge without grounding on the wall of the blood vessel **1100.**

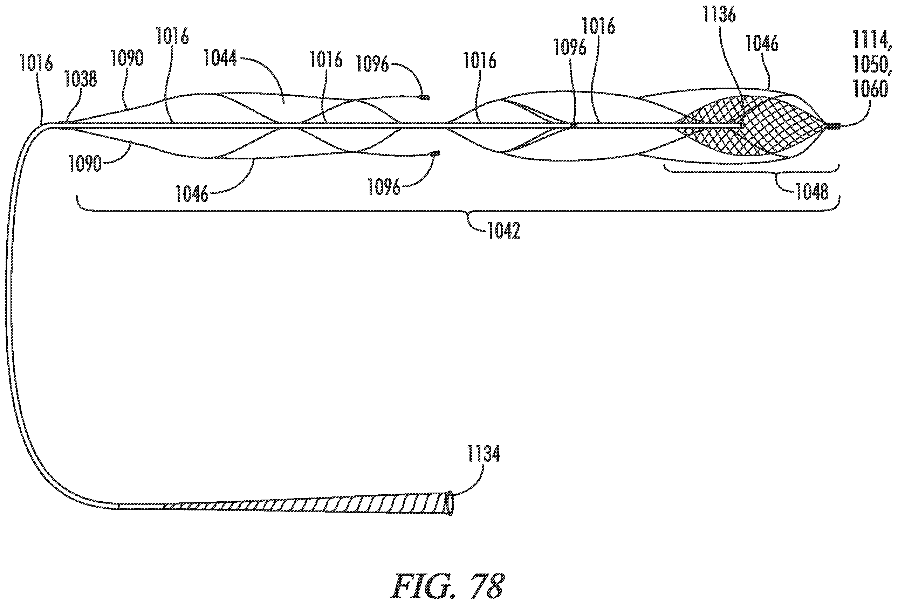

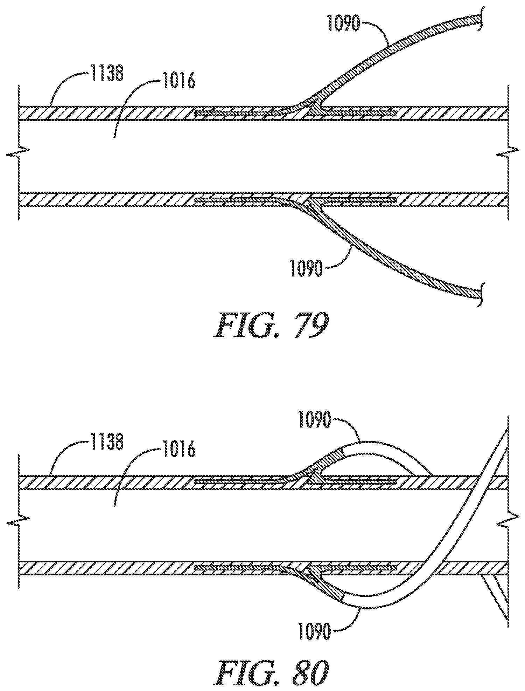

FIG. 78 illustrates an example where the pull wire **1016** is in the form of a catheter that may be used to deliver the active agent **1128.** For sake of labelling and differentiating from the previous catheter **1074,** the pull wire **1016** that is in the form of a catheter and used to deliver the active agent **1128** is labelled with the numeral **1016** and is called the active agent delivery catheter. The active agent delivery catheter **1016** may have an open proximal end **1134** for receiving the active agent **1128** and an open distal end **1136** for delivering the active agent **1128.** The active agent delivery catheter **1016** may be attached to the distal body **1018** at at least the distal body proximal junction **1038** and may be a braided design and proximally stiff with a distal progression of flexibility matching a typical core-coil delivery wire. The catheter distal end **1136** may be positioned at the distal body proximal junction **1038** (not shown), in the basket interior **1068** proximal to the distal body inner body **1048** (not shown), within the distal body inner body interior **1130** (the example shown in FIG. 78), or at the distal body distal junction **1060** (not shown), depending on where the user desires to deliver the active agent **1128.** The proximal strips **1090** may be mounted within the wall **1138** of the active agent delivery catheter **1016,** as shown in FIGs. 79-80, so as not to interfere with the delivery of the active agent **1128.** The active agent delivery catheter **1016** may be wider at the proximal end **1134** as shown in FIG. 78 and reinforced with nitinol or other support material for pushability. The active agent delivery catheter **1016** may be no wider than 0.027 inches so that the active agent delivery catheter **1016** may be delivered through a standard microcatheter **1074.** If desired the active agent delivery catheter **1016** may be perforated to allow delivery of the active agent **1128** along the distal body length **1034.**

The examples of FIGs. 73-81 may include a lead wire **286,** as shown in FIG. 73A, or an elongated distal body distal junction **1060,** as described previously.

### Part List for FIGs. 57-81

| | |
|---|---|
| System | 1010 |
| pull wire proximal end | 1012 |
| pull wire distal end | 1014 |
| pull wire | 1016 |
| Pull wire wall | 1017 |
| distal body | 1018 |
| distal body perimeter | 1020 |
| distal body interior | 1022 |
| distal body exterior | 1024 |
| proximal end | 1026 |
| proximal end center | 1028 |
| distal end | 1030 |
| distal end center | 1032 |
| distal body length | 1034 |
| longitudinal axis | 1036 |
| proximal junction | 1038 |
| basket | 1040 |
| proximal portion/distal body outer body | 1042 |
| cells | 1044 |
| basket memory metal strips | 1046 |
| distal portion/distal body inner body | 1048 |
| connection points | 1050 |
| proximal portion/distal body outer body interior | 1052 |
| distal segment | 1054 |
| distal braided mesh openings | 1056 |
| woven linear strands | 1058 |
| distal junction | 1060 |
| distal junction proximal end | 1062 |
| basket distal end | 1064 |
| distal portion perimeter | 1066 |
| basket interior | 1068 |
| distal body height | 1070 |
| distal body width | 1072 |
| catheter | 1074 |
| catheter interior | 1076 |
| catheter proximal end | 1078 |
| catheter distal end | 1080 |
| basket memory metal strips distal end | 1082 |
| proximal end of strand | 1086 |
| proximal strip | 1090 |
| proximal strip distal end | 1092 |
| proximal strip proximal end | 1094 |
| distal crowns | 1096 |
| enlarged cells | 1098 |
| vessel/lumen | 1100 |
| distal elongation | 1102 |
| proximal elongation | 1104 |
| distal portion/distal body inner body proximal junction | 1106 |
| distal end of strand | 1108 |
| basket memory metal strip interior surface | 1110 |
| distal portion/distal body inner body proximal end | 1112 |
| distal portion/distal body inner body distal end | 1114 |
| Suture tether | 1116 |
| Proximal coil | 1120 |
| Distal coil | 1122 |
| Gap | 1124 |
| Main Clot | 1126 |
| Secondary clot/distal emboli | 1127 |
| Active agent | 1128 |
| distal portion/distal body inner body interior | 1130 |
| distal portion/distal body inner body middle portion | 1132 |
| Active agent delivery catheter open proximal end | 1134 |
| Active agent delivery catheter open distal end | 1136 |
| Active agent delivery catheter wall | 1138 |
| Proximal helical coil | 1200 |
| Solder location | 1202 |
| Distal tether | 1204 |
| Distal helical coil | 1206 |

Having now described the invention in accordance with the requirements of the patent statutes, those skilled in the art will understand how to make changes and modifications to the disclosed examples to meet their specific requirements or conditions. Changes and modifications may be made without departing from the scope of the invention, as defined and limited solely by the following claims. In particular, although the system has been exemplified for use in retrieving blood clots, the system may be used to retrieve other objects from animal lumens. In addition, the steps of any method described herein may be performed in any suitable order and steps may be performed simultaneously if needed.

Terms of degree such as "substantially", "about" and "approximately" as used herein mean a reasonable amount of deviation of the modified term such that the end result is not significantly changed. For example, these terms can be construed as including a deviation of at least ± 5% of the modified term if this deviation would not negate the meaning of the word it modifies.

## Claims

1. A system (1010) for removing objects from an interior lumen of an animal, the system (1010) comprising:
a pull wire (1016) having a proximal end (1012) and a distal end (1014);
a distal body (1018) attached to the pull wire (1016) and comprising a distal body proximal end (1026) comprising a distal body proximal junction (1038), a distal body distal end (1030) comprising a distal body distal junction (1060), a distal body length (1034) extending from the distal body proximal end (1026) to the distal body distal end (1030), a distal body longitudinal axis (1036) extending from the distal body proximal junction (1038) to the distal body distal junction (1060), and a distal body height (1070) and width (1072) perpendicular to the distal body length (1034), the distal body (1018) comprising:
a distal body outer body (1042) extending from the distal body proximal end (1026) to the distal body distal end (1030), the distal body outer body (1042) comprising the distal body proximal junction (1038) and the distal body distal junction (1060), the distal body outer body (1042) comprising a distal body outer body perimeter separating a distal body outer body interior (1052) from a distal body outer body exterior, the distal body outer body (1042) comprising a basket (1040) comprised of a plurality of cells (1044) spaced about the distal body outer body perimeter and formed by a plurality of basket memory metal strips (1046), wherein at least some of the basket memory metal strips (1046) are located at a distal end of the basket (1064), wherein each of the basket memory metal strips (1046) located at the distal end of the basket (1064) have a distal end (1082), and wherein each of the distal ends (1082) of the basket memory metal strips located at the distal end of the basket (1064) converge at, and are attached to, the distal body distal junction (1060);
a distal body inner body (1048) comprised of a plurality of braided mesh openings (1056) formed by a plurality of woven linear strands (1058), the distal body inner body (1048) having a distal body inner body perimeter, each woven linear strand (1058) rotating about the distal body inner body perimeter relative to the distal body longitudinal axis (1036) a plurality of times in a helical fashion, the distal body inner body (1048) comprising a distal body inner body proximal end (1112) and a distal body inner body distal end (1114),
wherein the distal body (1018) has a relaxed state wherein the distal body (1018) has a first height and a first width, and a collapsed state wherein the distal body (1018) has a second height and a second width, the second height less than the first height, the second width less than the first width,
wherein the system (1010) further comprises a catheter (1074) having an interior (1076), a proximal end (1078) leading to the interior (1076) and a distal end (1080) leading to the interior (1076), the catheter (1074) comprised of a biocompatible material and configured to envelope the distal body (1018) when the distal body (1018) is in the collapsed state,
wherein the woven linear strands (1058) comprise a proximal end and a distal end, and at least some of the distal ends of the woven linear strands (1058) are attached to the distal body distal junction (1060),
wherein, in the relaxed state, the median surface area of the cells (1044) is larger than the median surface area of the braided mesh openings (1056), wherein, the distal body inner body (1048) and the distal body outer body (1042) each have a length generally parallel to the distal body length (1034), the distal body inner body and distal body outer body lengths configured to elongate upon moving from the relaxed state to the collapsed state,
wherein, in the collapsed state and in the relaxed state, the distal body inner body (1048) is located in the distal body outer body interior (1052),
wherein the woven linear strands (1058) rotate about the distal body inner body perimeter relative to the distal body longitudinal axis (1036) a fewer number of times per unit of length in the collapsed state as compared to the relaxed state,
wherein the proximal ends of at least some of the woven linear strands (1058) converge at and are attached to a distal body inner body proximal junction (1106), and
wherein the distal body inner body proximal junction (1106) forms the proximal end of the distal body inner body (1112),
wherein the system (1010) further comprises a proximal tether connecting the distal body proximal junction (1038) to the distal body inner body proximal junction (1106), and
**characterised in that** the system (1010) further comprises a distal tether (1204) connecting the distal body inner body distal junction to the distal body distal junction (1060),
wherein the distal tether (1204) comprises a distal helical coil (1206), the distal helical coil (1206) having a coil length generally parallel to the distal body length (1034), the distal helical coil (1206) having an expanded state in which the distal helical coil (1206) has a first length and a relaxed state in which the distal helical coil (1206) has a second length, the first length greater than the second length, and
wherein the distal helical coil (1206) is configured to move to the expanded state when tension is exerted on the distal body inner body (1048).

2. The system (1010) of claim 1, wherein the proximal tether is a segment of the pull wire (1016).

3. The system (1010) of claim 1, wherein the proximal tether comprises a proximal end attached to the distal body proximal junction (1038) and a distal end attached to the distal body inner body proximal junction (1106).

4. The system (1010) of claim 1, wherein the proximal and distal tethers located approximately in the center of the distal body height (1070) and the distal body width (1072) when the distal body (1018) is in the relaxed state and the proximal and distal tethers are generally parallel to the distal body longitudinal axis (1036) when the distal body (1018) is in the relaxed state.

5. The system (1010) of claim 1, wherein the basket memory metal strips (1046) are located on the distal body outer body perimeter and comprise an interior surface facing the distal body outer body interior (1052) and an exterior surface opposite the interior surface, and further wherein in the relaxed state, at least some of the woven linear strands (1058) contact the interior surface of at least some of the basket memory metal strips (1046).

6. The system (1010) of claim 1, wherein the distal body inner body (1048) comprises a distal body inner body height and a distal body inner body width and wherein the distal body inner body in the relaxed state comprises a distal body inner body proximal tapered region in which the distal body inner body height and the distal body inner body width decrease as the proximal ends of the woven linear strands (1058) approach the distal body inner body proximal junction.

7. The system (1010) of claim 1 wherein in the relaxed state, the basket does not have any free crowns that point generally in the proximal direction.

8. The system (1010) of claim 1 wherein the distal body outer body (1042) further comprises a plurality of proximal strips (1090), each proximal strip (1090) having a distal end (1092) attached to a proximal crown of a cell and a proximal end (1094), the proximal ends (1094) of the proximal strips converging at the distal body proximal junction (1038), or wherein the proximal ends of each of the woven linear strands converge at and are attached to the distal body inner body proximal junction (1106) and further wherein the distal ends of each of the woven linear strands converge at and are attached to the distal body distal junction (1060).

9. The system (1010) of claim 1, wherein in the relaxed state, the distal body inner body (1048) is more flexible than the distal body outer body (1042) and wherein, in the relaxed state, the median radial force of the distal body inner body is substantially less than the median radial force of the distal body outer body.

10. The system (1010) of claim 1, wherein the distal body inner body (1048) comprises a distal body inner body height and a distal body inner body width, wherein the distal body inner body (1048) in the relaxed state comprises a distal body inner body distal tapered region in which the distal body inner body height and the distal body inner body width decrease as the woven linear strand distal ends (1058) approach the distal body distal junction (1060), wherein the distal body outer body (1042) comprises a distal body outer body height and a distal body outer body width, and further wherein the distal body outer body (1042) comprises a tapered region in which the distal body outer body height and the distal body outer body width decrease as the distal ends of the basket memory metal strips (1046) located at the distal end of the basket approach the distal body distal junction (1060).

11. The system (1010) of claim 1, wherein, in the relaxed state, the distal body inner body (1048) impedes blood flow to a greater extent than the distal body outer body (1042) when the distal body outer body (1042) and the distal body inner body (1048) are placed in a blood vessel, or
wherein, prior to removal of an obstruction, the distal body inner body (1048) is configured to automatically reduce blood flow when the distal body inner body (1048) is placed in a blood vessel.

12. The system (1010) of claim 1, wherein, in the relaxed state, the distal body outer body (1042) comprises a first pair of distal crowns (1096) not attached to another cell (1044) of the basket (1040) and pointing generally in the distal direction, the distal crowns (1096) in the first pair of distal crowns located approximately the same distance from the distal body proximal junction (1038) and located between 150 degrees and 180 degrees relative to each other, and further wherein the basket (1040) further comprises a second pair of distal crowns (1096) not attached to another cell (1044) of the basket (1040) and pointing generally in the distal direction, the second pair of distal crowns (1096) located distally relative to the first pair of distal crowns (1096), each of the distal crowns (1096) in the second pair of distal crowns (1096) located between 60 degrees and 90 degrees relative to a distal crown (1096) in the first pair of distal crowns (1096), the distal crowns (1096) in the second pair of distal crowns (1096) located approximately the same distance from the distal body proximal junction (1038), each of the distal crowns (1096) forming a portion of a cell (1044),
wherein each distal crown (1096) in the first and second pair of distal crowns (1096) forms part of a different enlarged cell, each enlarged cell having a center,
wherein the centers of the enlarged cells of the first pair of distal crowns (1096) are between 150 degrees and 180 degrees relative to each other and between 60 degrees and 90 degrees relative to the centers of the enlarged cells of the second pair of distal crowns (1096),
wherein the enlarged cells are configured to allow a thrombus to pass therethrough and into the basket interior, and
optionally wherein, in the relaxed state, the distal body inner body proximal junction (1106) is located distally relative to the first and second pair of distal crowns (1096), or
wherein, in the relaxed state, the distal body inner body length is no more than about 33% of the distal body outer body length.

13. The system (1010) of claim 1 wherein the system (1010) further comprises a lead wire extending distally from the distal body distal junction (1060).

14. The system (1010) of claim 1 wherein the distal body inner body proximal end (1112) is substantially closed.

15. The system (1010) of claim 1,
wherein, upon moving from the relaxed state to the collapsed state, the length of the distal body inner body is configured to elongate a greater percentage than the length of the distal body outer body,
wherein, upon moving from the relaxed state to the collapsed state, the distal body inner body (1048) is configured to elongate proximally within the distal body outer body interior (1052) toward the distal body proximal junction (1038),
wherein, in the relaxed state, the distal body inner body proximal end (1112) is located a first distance distal from the distal body proximal junction,
wherein, in the collapsed state, the distal body inner body proximal end (1112) is located a second distance distal from the distal body proximal junction, the second distance less than the first distance.

## Patentansprüche

1. System (1010) zum Entfernen von Objekten aus einem inneren Lumen eines Tieres, wobei das System (1010) Folgendes umfasst:
einen Zugdraht (1016) mit einem proximalen Ende (1012) und einem distalen Ende (1014);
einen distalen Körper (1018), der an dem Zugdraht (1016) befestigt ist und ein proximales Ende (1026) des distalen Körpers, das einen proximalen Knotenpunkt (1038) des distalen Körpers umfasst, ein distales Ende (1030) des distalen Körpers, das einen distalen Knotenpunkt (1060) des distalen Körpers umfasst, eine Länge (1034) des distalen Körpers, die sich von dem proximalen Ende (1026) des distalen Körpers zu dem distalen Ende (1030) des distalen Körpers erstreckt, eine Längsachse (1036) des distalen Körpers, die sich von dem proximalen Knotenpunkt (1038) des distalen Körpers zu dem distalen Knotenpunkt (1060) des distalen Körpers erstreckt, und eine Höhe (1070) und Breite (1072) des distalen Körpers senkrecht zu der Länge (1034) des distalen Körpers umfasst, wobei der distale Körper (1018) Folgendes umfasst:
einen Außenkörper (1042) des distalen Körpers, der sich von dem proximalen Ende (1026) des distalen Körpers zu dem distalen Ende (1030) des distalen Körpers erstreckt, wobei der Außenkörper (1042) des distalen Körpers den proximalen Knotenpunkt (1038) des distalen Körpers und den distalen Knotenpunkt (1060) des distalen Körpers umfasst, wobei der Außenkörper (1042) des distalen Körpers einen Umfang des Außenkörpers des distalen Körpers umfasst, der einen Innenraum (1052) des Außenkörpers des distalen Körpers von einem Außenraum des Außenkörpers des distalen Körpers trennt, wobei der Außenkörper (1042) des distalen Körpers einen Korb (1040) umfasst, der aus einer Vielzahl von Zellen (1044) besteht, die um den Umfang des Außenkörpers des distalen Körpers herum beabstandet sind, und durch eine Vielzahl von Gedächtnismetallstreifen (1046) des Korbs gebildet werden, wobei sich zumindest einige der Gedächtnismetallstreifen (1046) des Korbs an einem distalen Ende des Korbs (1064) befinden, wobei jeder der Gedächtnismetallstreifen (1046) des Korbs, die sich an dem distalen Ende des Korbes (1064) befinden, ein distales Ende (1082) aufweist, und wobei alle distalen Enden (1082) der Gedächtnismetallstreifen des Korbs, die sich an dem distalen Ende des Korbes (1064) befinden, an dem distalen Knotenpunkt (1060) des distalen Körpers zusammenlaufen und daran befestigt sind;
einen Innenkörper (1048) des distalen Körpers, der aus einer Vielzahl von geflochtenen Maschenöffnungen (1056) besteht, die durch eine Vielzahl von gewebten linearen Strängen (1058) gebildet werden, wobei der Innenkörper (1048) des distalen Körpers einen Umfang des Innenkörpers des distalen Körpers aufweist, wobei sich jeder gewebte lineare Strang (1058) um den Umfang des Innenkörpers des distalen Körpers relativ zu der Längsachse (1036) des distalen Körpers mehrere Male schraubenförmig dreht, wobei der Innenkörper (1048) des distalen Körpers ein proximales Ende (1112) des Innenkörpers des distalen Körpers und ein distales Ende (1114) des Innenkörpers des distalen Körpers umfasst,
wobei der distale Körper (1018) einen entspannten Zustand aufweist, in dem der distale Körper (1018) eine erste Höhe und eine erste Breite aufweist, und einen zusammengefallenen Zustand, in dem der distale Körper (1018) eine zweite Höhe und eine zweite Breite aufweist, wobei die zweite Höhe geringer ist als die erste Höhe und die zweite Breite geringer als die erste Breite,
wobei das System (1010) weiter einen Katheter (1074) mit einem Innenraum (1076), einem proximalen Ende (1078), das zu dem Innenraum (1076) führt, und einem distalen Ende (1080), das zu dem Innenraum (1076) führt, umfasst, wobei der Katheter (1074) aus einem biokompatiblen Material besteht und so konfiguriert ist, dass er den distalen Körper (1018) umhüllt, wenn sich der distale Körper (1018) in dem zusammengefallenen Zustand befindet,
wobei die gewebten linearen Stränge (1058) ein proximales Ende und ein distales Ende umfassen und mindestens einige der distalen Enden der gewebten linearen Stränge (1058) an dem distalen Knotenpunkt (1060) des distalen Körpers befestigt sind,
wobei im entspannten Zustand der mittlere Oberflächenbereich der Zellen (1044) größer ist als der mittlere Oberflächenbereich der geflochtenen Maschenöffnungen (1056), wobei der Innenkörper (1048) des distalen Körpers und der Außenkörper (1042) des distalen Körpers jeweils eine Länge aufweisen, die im Allgemeinen parallel zu der Länge (1034) des distalen Körpers verläuft, wobei die Längen des Innenkörpers des distalen Körpers und des Außenkörpers des distalen Körpers so konfiguriert sind, dass sie sich bei der Bewegung von dem entspannten Zustand in den zusammengefallenen Zustand verlängern,
wobei sich der Innenkörper (1048) des distalen Körpers in dem zusammengefallenen Zustand und in dem entspannten Zustand in dem Innenraum (1052) des Außenkörpers des distalen Körpers befindet,
wobei die gewebten linearen Stränge (1058) sich um den Umfang des Innenkörpers des distalen Körpers relativ zu der Längsachse (1036) des distalen Körpers im zusammengefallenen Zustand im Vergleich zu dem entspannten Zustand weniger oft pro Längeneinheit drehen,
wobei die proximalen Enden von mindestens einigen der gewebten linearen Stränge (1058) an einem proximalen Knotenpunkt (1106) des Innenkörpers des distalen Körpers zusammenlaufen und daran befestigt sind, und
wobei der proximale Knotenpunkt (1106) des Innenkörpers des distalen Körpers das proximale Ende des Innenkörpers (1112) des distalen Körpers bildet,
wobei das System (1010) weiter eine proximale Halteleine umfasst, die den proximalen Knotenpunkt (1038) des distalen Körpers mit dem proximalen Knotenpunkt (1106) des Innenkörpers des distalen Körpers verbindet, und **dadurch gekennzeichnet, dass** das System (1010) weiter eine distale Halteleine (1204) umfasst, die den distalen Knotenpunkt des Innenkörpers des distalen Körpers mit dem distalen Knotenpunkt (1060) des distalen Körpers verbindet,
wobei die distale Halteleine (1204) eine distale schraubenförmige Windung (1206) umfasst, wobei die distale schraubenförmige Windung (1206) eine Windungslänge aufweist, die im Allgemeinen parallel zu der Länge (1034) des distalen Körpers ist, wobei die distale schraubenförmige Windung (1206) einen expandierten Zustand, in dem die distale schraubenförmige Windung (1206) eine erste Länge aufweist, und einen entspannten Zustand aufweist, in dem die distale schraubenförmige Windung (1206) eine zweite Länge aufweist, wobei die erste Länge größer als die zweite Länge ist, und
wobei die distale schraubenförmige Windung (1206) so konfiguriert ist, dass sie sich in den expandierten Zustand bewegt, wenn Spannung auf den Innenkörper (1048) des distalen Körpers ausgeübt wird.

2. System (1010) nach Anspruch 1, wobei die proximale Halteleine ein Segment des Zugdrahts (1016) ist.

3. System (1010) nach Anspruch 1, wobei die proximale Halteleine ein proximales Ende, das an dem proximalen Knotenpunkt (1038) des distalen Körpers befestigt ist, und ein distales Ende, das an dem proximalen Knotenpunkt (1106) des Innenkörpers des distalen Körpers befestigt ist, umfasst.

4. System (1010) nach Anspruch 1, wobei die proximalen und distalen Leinen ungefähr in der Mitte der Höhe (1070) des distalen Körpers und der Breite (1072) des distalen Körpers angeordnet sind, wenn sich der distale Körper (1018) in dem entspannten Zustand befindet, und die proximalen und distalen Leinen im Allgemeinen parallel zu der Längsachse (1036) des distalen Körpers verlaufen, wenn sich der distale Körper (1018) in dem entspannten Zustand befindet.

5. System (1010) nach Anspruch 1, wobei die Gedächtnismetallstreifen (1046) des Korbs an dem Umfang des Außenkörpers des distalen Körpers angeordnet sind und eine Innenfläche, die dem Innenraum (1052) des Außenkörpers des distalen Körpers zugewandt ist, und eine Außenfläche gegenüber der Innenfläche umfassen, und weiter wobei in dem entspannten Zustand mindestens einige der gewebten linearen Stränge (1058) die Innenfläche von mindestens einigen der Gedächtnismetallstreifen (1046) des Korbes kontaktieren.

6. System (1010) nach Anspruch 1, wobei der Innenkörper (1048) des distalen Körpers eine Höhe des Innenkörpers des distalen Körpers und eine Breite des Innenkörpers des distalen Körpers umfasst und wobei der Innenkörper des distalen Körpers in dem entspannten Zustand einen proximalen verjüngten Bereich des Innenkörpers des distalen Körpers umfasst, in dem die Höhe des Innenkörpers des distalen Körpers und die Breite des Innenkörpers des distalen Körpers abnehmen, wenn sich die proximalen Enden der gewebten linearen Stränge (1058) dem proximalen Knotenpunkt des Innenkörpers des distalen Körpers nähern.

7. System (1010) nach Anspruch 1, wobei der Korb in dem entspannten Zustand keine freien Kronen aufweist, die allgemein in die proximale Richtung weisen.

8. System (1010) nach Anspruch 1, wobei der Außenkörper (1042) des distalen Körpers weiter eine Vielzahl von proximalen Streifen (1090) umfasst, wobei jeder proximale Streifen (1090) ein distales Ende (1092), das an einer proximalen Krone einer Zelle befestigt ist, und ein proximales Ende (1094) aufweist, wobei die proximalen Enden (1094) der proximalen Streifen an dem proximalen Knotenpunkt (1038) des distalen Körpers zusammenlaufen, oder wobei die proximalen Enden jedes der gewebten linearen Stränge an dem proximalen Knotenpunkt (1106) des Innenkörpers des distalen Körpers zusammenlaufen und daran befestigt sind, und weiter wobei die distalen Enden jedes der gewebten linearen Stränge an dem distalen Knotenpunkt (1060) des distalen Körpers zusammenlaufen und daran befestigt sind.

9. System (1010) nach Anspruch 1, wobei in dem entspannten Zustand der Innenkörper (1048) des distalen Körpers flexibler ist als der Außenkörper (1042) des distalen Körpers und wobei in dem entspannten Zustand die mittlere Radialkraft des Innenkörpers des distalen Körpers wesentlich geringer ist als die mittlere Radialkraft des Außenkörpers des distalen Körpers.

10. System (1010) nach Anspruch 1, wobei der Innenkörper (1048) des distalen Körpers eine Höhe des Innenkörpers des distalen Körpers und eine Breite des Innenkörpers des distalen Körpers umfasst, wobei der Innenkörper (1048) des distalen Körpers in dem entspannten Zustand einen sich verjüngenden Bereich eines Innenkörpers des distalen Körpers umfasst, in dem die Höhe des Innenkörpers des distalen Körpers und die Breite des Innenkörpers des distalen Körpers abnehmen, wenn sich die distalen Enden (1058) des gewebten linearen Strangs dem distalen Knotenpunkt (1060) des distalen Körpers nähern, wobei der Außenkörper (1042) des distalen Körpers eine Höhe des Außenkörpers des distalen Körpers und eine Breite des Außenkörpers des distalen Körpers umfasst, und weiter wobei der Außenkörper (1042) des distalen Körpers einen verjüngten Bereich umfasst, in dem die Höhe des Außenkörpers des distalen Körpers und die Breite des Außenkörpers des distalen Körpers abnehmen, wenn sich die distalen Enden der Gedächtnismetallstreifen (1046) des Korbes, die sich an dem distalen Ende des Korbes befinden, dem distalen Knotenpunkt (1060) des distalen Körpers nähern.

11. System (1010) nach Anspruch 1, wobei in dem entspannten Zustand der Innenkörper (1048) des distalen Körpers den Blutfluss in einem größeren Ausmaß behindert als der Außenkörper (1042) des distalen Körpers, wenn der Außenkörper (1042) des distalen Körpers und der Innenkörper (1048) des distalen Körpers in einem Blutgefäß platziert sind, oder
wobei der Innenkörper (1048) des distalen Körpers so konfiguriert ist, dass er vor der Entfernung eines Hindernisses den Blutfluss automatisch reduziert, wenn der Innenkörper (1048) des distalen Körpers in einem Blutgefäß platziert wird.

12. System (1010) nach Anspruch 1, wobei der Außenkörper (1042) des distalen Körpers im entspannten Zustand ein erstes Paar distaler Kronen (1096) umfasst, die nicht an einer anderen Zelle (1044) des Korbes (1040) befestigt sind und im Allgemeinen in die distale Richtung zeigen, wobei die distalen Kronen (1096) in dem ersten Paar distaler Kronen ungefähr den gleichen Abstand von dem proximalen Knotenpunkt (1038) des distalen Körpers haben und zwischen 150 Grad und 180 Grad relativ zueinander angeordnet sind, und weiter wobei der Korb (1040) weiter ein zweites Paar distaler Kronen (1096) umfasst, die nicht an einer anderen Zelle (1044) des Korbes (1040) befestigt sind und im Allgemeinen in die distale Richtung zeigen, wobei das zweite Paar von distalen Kronen (1096) distal relativ zu dem ersten Paar von distalen Kronen (1096) angeordnet ist, wobei jede der distalen Kronen (1096) in dem zweiten Paar von distalen Kronen (1096) zwischen 60 Grad und 90 Grad relativ zu einer distalen Krone (1096) in dem ersten Paar von distalen Kronen (1096) angeordnet ist, wobei die distalen Kronen (1096) in dem zweiten Paar distaler Kronen (1096) sich ungefähr in dem gleichen Abstand von dem proximalen Knotenpunkt (1038) des distalen Körpers befinden, wobei jede der distalen Kronen (1096) einen Teil einer Zelle (1044) bildet,
wobei jede distale Krone (1096) in dem ersten und zweiten Paar distaler Kronen (1096) einen Teil einer anderen vergrößerten Zelle bildet, wobei jede vergrößerte Zelle ein Zentrum hat,
wobei die Zentren der vergrößerten Zellen des ersten Paars distaler Kronen (1096) zwischen 150 Grad und 180 Grad relativ zueinander und zwischen 60 Grad und 90 Grad relativ zu den Zentren der vergrößerten Zellen des zweiten Paars distaler Kronen (1096) liegen,
wobei die vergrößerten Zellen so konfiguriert sind, dass ein Thrombus durch sie hindurch und in das Innere des Korbes gelangen kann, und
optional wobei in dem entspannten Zustand der proximale Knotenpunkt (1106) des Innenkörpers des distalen Körpers distal relativ zu dem ersten und zweiten Paar distaler Kronen (1096) angeordnet ist, oder
wobei in dem entspannten Zustand die Länge des Innenkörpers des distalen Körpers nicht mehr als etwa 33 % der Länge des Außenkörpers des distalen Körpers beträgt.

13. System (1010) nach Anspruch 1, wobei das System (1010) weiter einen Leitungsdraht umfasst, der sich distal von dem distalen Knotenpunkt (1060) des distalen Körpers erstreckt.

14. System (1010) nach Anspruch 1, wobei das proximale Ende (1112) des Innenkörpers des distalen Körpers im Wesentlichen geschlossen ist.

15. System (1010) nach Anspruch 1,
wobei beim Bewegen von dem entspannten Zustand in den zusammengefallenen Zustand die Länge des Innenkörpers des distalen Körpers so konfiguriert ist, dass sie sich um einen größeren Prozentsatz verlängert als die Länge des Außenkörpers des distalen Körpers,
wobei beim Bewegen von dem entspannten Zustand in den zusammengefallenen Zustand der Innenkörper (1048) des distalen Körpers so konfiguriert ist, dass er sich in proximaler Richtung innerhalb des Innenraumes (1052) des Außenkörpers des distalen Körpers in Richtung des proximalen Knotenpunkts (1038) des distalen Körpers verlängert,
wobei in dem entspannten Zustand das proximale Ende (1112) des Innenkörpers des distalen Körpers in einem ersten Abstand distal von dem proximalen Knotenpunkt des distalen Körpers angeordnet ist,
wobei in dem zusammengefallenen Zustand das proximale Ende (1112) des Innenkörpers des distalen Körpers sich in einem zweiten Abstand distal von dem proximalen Knotenpunkt des distalen Körpers befindet, wobei der zweite Abstand geringer ist als der erste Abstand.

## Revendications

1. Système (1010) de retrait d'objets d'une lumière intérieure d'un animal, le système (1010) comprenant :
un fil de traction (1016) présentant une extrémité proximale (1012) et une extrémité distale (1014) ;
un corps distal (1018) fixé au fil de traction (1016) et comprenant une extrémité proximale (1026) de corps distal comprenant une jonction proximale (1038) de corps distal, une extrémité distale (1030) de corps distal comprenant une jonction distale (1060) de corps distal, une longueur (1034) de corps distal s'étendant de l'extrémité proximale (1026) de corps distal à l'extrémité distale (1030) de corps distal, un axe longitudinal (1036) de corps distal s'étendant de la jonction proximale (1038) de corps distal à la jonction distale (1060) de corps distal, et une hauteur (1070) et une largeur (1072) de corps distal perpendiculaires à la longueur (1034) de corps distal, le corps distal (1018) comprenant :
un corps externe (1042) de corps distal s'étendant de l'extrémité proximale (1026) de corps distal à l'extrémité distale (1030) de corps distal, le corps externe (1042) de corps distal comprenant la jonction proximale (1038) de corps distal et la jonction distale (1060) de corps distal, le corps externe (1042) de corps distal comprenant un périmètre de corps externe de corps distal séparant un intérieur (1052) de corps externe de corps distal d'un extérieur de corps externe de corps distal, le corps externe (1042) de corps distal comprenant un panier (1040) composé d'une pluralité de cellules (1044) espacées autour du périmètre de corps externe de corps distal et formées par une pluralité de bandes métalliques (1046) de mémoire de panier, dans lequel au moins certaines des bandes métalliques (1046) de mémoire de panier sont situées au niveau d'une extrémité distale du panier (1064), dans lequel chacune des bandes métalliques (1046) de mémoire de panier situées au niveau de l'extrémité distale du panier (1064) présente une extrémité distale (1082), et dans lequel chacune des extrémités distales (1082) des bandes métalliques de mémoire de panier situées au niveau de l'extrémité distale du panier (1064) convergent au niveau de la jonction distale (1060) de corps distal et y sont fixées ;
un corps interne (1048) de corps distal composé d'une pluralité d'ouvertures de mailles tressées (1056) formées par une pluralité de brins linéaires tissés (1058), le corps interne (1048) de corps distal présentant un périmètre de corps interne de corps distal, chaque brin linéaire tissé (1058) tournant autour du périmètre de corps interne de corps distal par rapport à l'axe longitudinal (1036) de corps distal plusieurs fois de manière hélicoïdale, le corps interne (1048) de corps distal comprenant une extrémité proximale (1112) de corps interne de corps distal et une extrémité distale (1114) de corps interne de corps distal,
dans lequel le corps distal (1018) présente un état détendu dans lequel le corps distal (1018) présente une première hauteur et une première largeur, et un état replié dans lequel le corps distal (1018) présente une seconde hauteur et une seconde largeur, la seconde hauteur étant inférieure à la première hauteur, la seconde largeur étant inférieure à la première largeur,
dans lequel le système (1010) comprend en outre un cathéter (1074) présentant un intérieur (1076), une extrémité proximale (1078) menant à l'intérieur (1076) et une extrémité distale (1080) menant à l'intérieur (1076), le cathéter (1074) étant composé d'un matériau biocompatible et étant configuré pour envelopper le corps distal (1018) lorsque le corps distal (1018) est dans l'état replié,
dans lequel les brins linéaires tissés (1058) comprennent une extrémité proximale et une extrémité distale, et au moins certaines des extrémités distales des brins linéaires tissés (1058) sont fixées à la jonction distale (1060) de corps distal,
dans lequel, dans l'état détendu, la surface médiane des cellules (1044) est plus grande que la surface médiane des ouvertures de mailles tressées (1056), dans lequel le corps interne (1048) de corps distal et le corps externe (1042) de corps distal présentent chacun une longueur généralement parallèle à la longueur (1034) de corps distal, les longueurs de corps interne de corps distal et de corps externe de corps distal sont configurées pour s'allonger lors du déplacement de l'état détendu vers l'état replié,
dans lequel, dans l'état replié et dans l'état détendu, le corps interne (1048) de corps distal est situé dans l'intérieur (1052) de corps externe de corps distal,
dans lequel les brins linéaires tissés (1058) tournent autour du périmètre de corps interne de corps distal par rapport à l'axe longitudinal (1036) de corps distal un nombre de fois inférieur par unité de longueur dans l'état replié par rapport à l'état détendu,
dans lequel les extrémités proximales d'au moins certains des brins linéaires tissés (1058) convergent au niveau d'une jonction proximale (1106) de corps interne de corps distal et sont fixées à ladite jonction, et
dans lequel la jonction proximale (1106) de corps interne de corps distal forme l'extrémité proximale (1112) du corps interne de corps distal,
dans lequel le système (1010) comprend en outre une longe proximale reliant la jonction proximale (1038) de corps distal à la jonction proximale (1106) de corps interne de corps distal, et **caractérisé en ce que**
le système (1010) comprend en outre une longe distale (1204) reliant la jonction distale de corps interne de corps distal à la jonction distale (1060) de corps distal,
dans lequel la longe distale (1204) comprend une bobine hélicoïdale distale (1206), la bobine hélicoïdale distale (1206) présentant une longueur de bobine généralement parallèle à la longueur (1034) de corps distal, la bobine hélicoïdale distale (1206) présentant un état déployé dans lequel la bobine hélicoïdale distale (1206) présente une première longueur et un état détendu dans lequel la bobine hélicoïdale distale (1206) présente une seconde longueur, la première longueur étant supérieure à la seconde longueur, et
dans lequel la bobine hélicoïdale distale (1206) est configurée pour se déplacer vers l'état déployé lorsqu'une tension est exercée sur le corps interne (1048) de corps distal.

2. Système (1010) selon la revendication 1, dans lequel la longe proximale est un segment du fil de traction (1016).

3. Système (1010) selon la revendication 1, dans lequel la longe proximale comprend une extrémité proximale fixée à la jonction proximale (1038) de corps distal et une extrémité distale fixée à la jonction proximale (1106) de corps interne de corps distal.

4. Système (1010) selon la revendication 1, dans lequel les longes proximale et distale sont situées approximativement au centre de la hauteur (1070) de corps distal et de la largeur (1072) de corps distal lorsque le corps distal (1018) est dans l'état détendu et les longes proximale et distale sont généralement parallèles à l'axe longitudinal (1036) de corps distal lorsque le corps distal (1018) est dans l'état détendu.

5. Système (1010) selon la revendication 1, dans lequel les bandes métalliques (1046) de mémoire de panier sont situées sur le périmètre de corps externe de corps distal et comprennent une surface intérieure faisant face à l'intérieur (1052) de corps externe de corps distal et une surface extérieure opposée à la surface intérieure, et en outre dans lequel, dans l'état détendu, au moins certains des brins linéaires tissés (1058) entrent en contact avec la surface intérieure d'au moins certaines des bandes métalliques (1046) de mémoire de panier.

6. Système (1010) selon la revendication 1, dans lequel le corps interne (1048) de corps distal comprend une hauteur de corps interne de corps distal et une largeur de corps interne de corps distal et dans lequel le corps interne de corps distal dans l'état détendu comprend une région conique proximale de corps interne de corps distal dans laquelle la hauteur de corps interne de corps distal et la largeur de corps interne de corps distal diminuent à mesure que les extrémités proximales des brins linéaires tissés (1058) s'approchent de la jonction proximale de corps interne de corps distal.

7. Système (1010) selon la revendication 1, dans lequel, dans l'état détendu, le panier ne présente pas de couronnes libres qui pointent généralement dans la direction proximale.

8. Système (1010) selon la revendication 1, dans lequel le corps externe (1042) de corps distal comprend en outre une pluralité de bandes proximales (1090), chaque bande proximale (1090) présentant une extrémité distale (1092) fixée à une couronne proximale d'une cellule et une extrémité proximale (1094), les extrémités proximales (1094) des bandes proximales convergeant au niveau de la jonction proximale (1038) de corps distal, ou
dans lequel les extrémités proximales de chacun des brins linéaires tissés convergent au niveau de la jonction proximale (1106) de corps interne de corps distal et sont fixées à ladite jonction, et en outre dans lequel les extrémités distales de chacun des brins linéaires tissés convergent au niveau de la jonction distale (1060) de corps distal et sont fixées à ladite jonction.

9. Système (1010) selon la revendication 1, dans lequel, dans l'état détendu, le corps interne (1048) de corps distal est plus flexible que le corps externe (1042) de corps distal et dans lequel, dans l'état détendu, la force radiale médiane du corps interne de corps distal est sensiblement inférieure à la force radiale médiane du corps externe de corps distal.

10. Système (1010) selon la revendication 1, dans lequel le corps interne (1048) de corps distal comprend une hauteur de corps interne de corps distal et une largeur de corps interne de corps distal, dans lequel le corps interne (1048) de corps distal dans l'état détendu comprend une région conique distale de corps interne de corps distal dans laquelle la hauteur de corps interne de corps distal et la largeur de corps interne de corps distal diminuent à mesure que les extrémités distales de brins linéaires tissés (1058) s'approchent de la jonction distale (1060) de corps distal, dans lequel le corps externe (1042) de corps distal comprend une hauteur de corps externe de corps distal et une largeur de corps externe de corps distal, et en outre dans lequel le corps externe (1042) de corps distal comprend une région conique dans laquelle la hauteur de corps externe de corps distal et la largeur de corps externe de corps distal diminuent à mesure que les extrémités distales des bandes métalliques (1046) de mémoire de panier situées au niveau de l'extrémité distale du panier s'approchent de la jonction distale (1060) de corps distal.

11. Système (1010) selon la revendication 1, dans lequel, dans l'état détendu, le corps interne (1048) de corps distal empêche le débit sanguin dans une plus grande mesure que le corps externe (1042) de corps distal lorsque le corps externe (1042) de corps distal et le corps interne (1048) de corps distal sont placés dans un vaisseau sanguin, ou
dans lequel, avant le retrait d'une obstruction, le corps interne (1048) de corps distal est configuré pour réduire automatiquement le débit sanguin lorsque le corps interne (1048) de corps distal est placé dans un vaisseau sanguin.

12. Système (1010) selon la revendication 1, dans lequel, dans l'état détendu, le corps externe (1042) de corps distal comprend une première paire de couronnes distales (1096) non fixées à une autre cellule (1044) du panier (1040) et pointant généralement dans la direction distale, les couronnes distales (1096) dans la première paire de couronnes distales étant situées à environ la même distance de la jonction proximale (1038) de corps distal et situées entre 150 degrés et 180 degrés les unes par rapport aux autres, et en outre dans lequel le panier (1040) comprend en outre une seconde paire de couronnes distales (1096) non fixées à une autre cellule (1044) du panier (1040) et pointant généralement dans la direction distale, la seconde paire de couronnes distales (1096) étant située distalement par rapport à la première paire de couronnes distales (1096), chacune des couronnes distales (1096) dans la seconde paire de couronnes distales (1096) étant située entre 60 degrés et 90 degrés par rapport à une couronne distale (1096) dans la première paire de couronnes distales (1096), les couronnes distales (1096) dans la seconde paire de couronnes distales (1096) étant situées à environ la même distance de la jonction proximale (1038) de corps distal, chacune des couronnes distales (1096) formant une partie d'une cellule (1044),
dans lequel chaque couronne distale (1096) dans la première et la seconde paire de couronnes distales (1096) forme une partie d'une cellule agrandie différente, chaque cellule agrandie présentant un centre,
dans lequel les centres des cellules agrandies de la première paire de couronnes distales (1096) sont compris entre 150 degrés et 180 degrés les uns par rapport aux autres et entre 60 degrés et 90 degrés par rapport aux centres des cellules agrandies de la seconde paire de couronnes distales (1096),
dans lequel les cellules agrandies sont configurées pour permettre à un thrombus de passer à travers celles-ci et pour aller l'intérieur du panier, et
facultativement dans lequel, dans l'état détendu, la jonction proximale (1106) de corps interne de corps distal est située distalement par rapport à la première et à la seconde paire de couronnes distales (1096), ou
dans lequel, dans l'état détendu, la longueur de corps interne de corps distal n'est pas supérieure à environ 33 % de la longueur de corps externe de corps distal.

13. Système (1010) selon la revendication 1, dans lequel le système (1010) comprend en outre un fil conducteur s'étendant distalement à partir de la jonction distale (1060) de corps distal.

14. Système (1010) selon la revendication 1, dans lequel l'extrémité proximale (1112) de corps interne de corps distal est sensiblement fermée.

15. Système (1010) selon la revendication 1,
dans lequel, lors du déplacement de l'état détendu à l'état replié, la longueur du corps interne de corps distal est configurée pour s'allonger d'un pourcentage supérieur à la longueur du corps externe de corps distal,
dans lequel, lors du passage de l'état détendu à l'état replié, le corps interne (1048) de corps distal est configuré pour s'allonger proximalement dans l'intérieur (1052) de corps externe de corps distal en direction de la jonction proximale (1038) de corps distal,
dans lequel, dans l'état détendu, l'extrémité proximale (1112) de corps interne de corps distal est située à une première distance distale par rapport à la jonction proximale de corps distal,
dans lequel, dans l'état replié, l'extrémité proximale (1112) de corps interne de corps distal est située à une seconde distance distale par rapport à la jonction proximale de corps distal, la seconde distance étant inférieure à la première distance.
